# EUROPEAN PATENT SPECIFICATION

(11) **EP 3 942 052 B1**
(45) Date of publication and mention of the grant of the patent: **28.10.2026**
(21) Application number: 20712917.2
(22) Date of filing: 18.03.2020
(51) Int. Cl.: C12N 15/82, C07K 14/80, C12N 9/16

(54) **METHODS OF ENHANCING BIOMASS IN A PLANT THROUGH STIMULATION OF RUBP REGENERATION AND ELECTRON TRANSPORT**
METHODEN ZUR VERBESSERUNG DER BIOMASSE IN EINER PFLANZE DURCH STIMULIERUNG DER REGENERATION VON RUBP UND DES ELEKTRONENTRANSPORTS
MÉTHODES D'AMÉLIORATION DE LA BIOMASSE DANS UNE PLANTE PAR LA STIMULATION DE LA RÉGÉNÉRATION DE RUBP ET DU TRANSPORT DES ÉLECTRONS

(30) Priority: 21.03.2019 US 201962821786 P
(43) Date of publication of application: 26.01.2022
(73) Proprietor: University of Essex Enterprises Limited, Colchester Essex CO4 3SQ (GB)
(72) Inventor: LAWSON, Tracy, Colchester CO4 3SQ (GB); RAINES, Christine A., Colchester CO4 3SQ (GB); LÓPEZ-CALCAGNO, Patricia E., Colchester CO4 3SQ (GB)
(74) Representative: J A Kemp LLP
(86) International application number: PCT/EP2020/057475
(87) International publication number: WO 2020/187995

(56) References cited:
- WO-A1-2017/144901
- ANDREW J. SIMKIN ET AL: "Overexpression of the RieskeFeS Protein Increases Electron Transport Rates and Biomass Yield", PLANT PHYSIOLOGY, vol. 175, no. 1, 28 July 2017 (2017-07-28), Rockville, Md, USA, pages 134 - 145, XP055692987, ISSN: 0032-0889, DOI: 10.1104/pp.17.00622
- CHIDA HIROTAKA ET AL: "Expression of the algal cytochrome c(6) gene in Arabidopsis enhances photosynthesis and growth", PLANT AND CELL PHYSIOLOGY, OXFORD UNIVERSITY PRESS, UK, vol. 48, no. 7, 1 July 2007 (2007-07-01), pages 948 - 957, XP002516798, ISSN: 0032-0781, DOI: 10.1093/PCP/PCM064
- YADAV SWETA K ET AL: "Introgression of UfCyt c6, a thylakoid lumen protein from a green seaweed Ulva fasciata Delile enhanced photosynthesis and growth in tobacco", MOLECULAR BIOLOGY REPORTS, SPRINGER NETHERLANDS, NL, vol. 45, no. 6, 29 August 2018 (2018-08-29), pages 1745 - 1758, XP036644873, ISSN: 0301-4851, [retrieved on 20180829], DOI: 10.1007/S11033-018-4318-1
- A. J. SIMKIN ET AL: "Multigene manipulation of photosynthetic carbon assimilation increases CO2 fixation and biomass yield in tobacco", JOURNAL OF EXPERIMENTAL BOTANY, vol. 66, no. 13, 1 July 2015 (2015-07-01), GB, pages 4075 - 4090, XP055305505, ISSN: 0022-0957, DOI: 10.1093/jxb/erv204
- CHRISTOPH PETERHANSEL ET AL: "Metabolic Engineering Towards the Enhancement of Photosynthesis +", PHOTOCHEMISTRY AND PHOTOBIOLOGY, vol. 84, no. 6, 1 November 2008 (2008-11-01), pages 1317 - 1323, XP055081908, ISSN: 0031-8655, DOI: 10.1111/j.1751-1097.2008.00427.x

## Description

### TECHNICAL FIELD

The present disclosure relates to genetically altered plants. In particular, the present disclosure relates to genetically altered plants with enhanced biomass including genetic alterations that stimulate RuBP regeneration including through overexpression of Calvin Benson cycle (CB) proteins such as FBPase/SBPase or SBPase, and including genetic alterations that stimulate electron transport, including through overexpression of photosynthetic electron transport proteins such as cytochrome c₆ and Rieske FeS.

### BACKGROUND

The yield potential of crop species is limited by multiple external factors, including agricultural management and environmental conditions. Even under optimal management and conditions, however, the energy conversion efficiency of crop species can still limit yield. Energy conversion efficiency is the ratio of biomass energy produced divided by light energy intercepted by the crop canopy over a given period, and is determined by plant internal processes such as photosynthesis and respiration. Modeling has shown that the energy conversion efficiency of major crop species lags behind other yield potential improvement components, and represents a major roadblock in improving the yield potential of crop species (Zhu, et al., Annu. Rev. Plant. Biol. (2010) 61:235-261).

The Calvin Benson cycle (CB) is a promising target for improving photosynthesis, as it is involved in assimilating carbon, *i.e.,* producing biomass energy. Early studies showed that even small reductions in individual CB enzymes are sufficient to reduce carbon assimilation and plant growth. While some enzymes have a larger effect than others, research has shown that overexpressing different individual CB enzymes results in increased photosynthetic carbon assimilation and improved plant growth. Therefore, there is no single limiting step in photosynthetic carbon assimilation. This means that although manipulating CB enzyme activity might be used to increase productivity, developing an effective engineering strategy for major crop species has proven to date to not be as simple as altering one component.

Photosynthetic electron transport is another possible target for improving photosynthesis, as it is involved in harnessing the light energy intercepted by the crop canopy. Individual components of the photosynthetic electron transport chain have been shown to be able to increase electron transport rates. For example, overexpression of the plant Rieske FeS protein resulted in increased electron transport rates and increased plant biomass (Simkin, et al., Plant Physiol. (2017) 175:134-145). While individual components have provided promising results, studies have shown that overall, the efficiency of photosynthetic electron transport in higher plants is limited by the photosynthetic electron transport proteins of higher plants, such as plastocyanin (Chida, et al., Plant Cell Physiol. (2007) 48:948-957; Finazzi, et al., Proc. Natl. Acad. Sci. U S A. (2005) 102:7031-7036).

There exists a clear need for improved energy conversion efficiency in order to achieve optimal yield potential of crop species. In order to develop plants with improved energy conversion efficiency, multi-component engineering incorporating different aspects of photosynthesis is required.

### BRIEF SUMMARY

In order to meet these needs, the present disclosure provides means of enhancing plant biomass by stimulating RuBP regeneration and electron transport. In particular, the present disclosure relates to genetically altered plants with enhanced biomass through overexpression of CB proteins (*e.g*., FBPase/SBPase or SBPase), and overexpression of photosynthetic electron transport proteins (*e.g*., cytochrome c₆ and Rieske FeS).

An aspect of the disclosure includes a genetically altered plant, plant part, or plant cell, wherein the plant, part thereof or cell includes one or more RuBP regeneration enhancing genetic alterations that increase activity of a CB protein and one or more photosynthetic electron transport enhancing genetic alterations as compared to the unaltered plant, plant part, or plant cell grown under the same conditions, wherein the one or more RuBP regeneration enhancing genetic alterations comprise overexpression of a CB protein, and wherein the CB protein comprises a sedoheptulose-1,7-bisphosphatase (SBPase), a fructose-1,6-bisphophate aldolase (FBPA), a chloroplastic fructose-1,6-bisphosphatase (FBPase), or a bifunctional fructose-1,6-bisphosphatases/sedoheptulose-1,7-bisphosphatase (FBP/SBPase), wherein the one or more photosynthetic electron transport enhancing genetic alterations being overexpression of one or more photosynthetic electron transport proteins, wherein the one or more photosynthetic electron transport proteins being selected from the group of a cytochrome c₆ protein, a Rieske FeS protein, or a cytochrome c₆ protein and a Rieske FeS protein, and wherein the plant is not exclusively obtained by means of an essentially biological process.

A further embodiment of this aspect includes the one or more photosynthetic electron transport proteins being a cytochrome c₆ protein. Still another embodiment of this aspect includes the cytochrome c₆ protein being an algal cytochrome c₆ protein. In an additional embodiment of this aspect, the algal cytochrome c₆ protein includes an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 102. An additional embodiment of this aspect includes the one or more photosynthetic electron transport proteins being a Rieske FeS protein. In a further embodiment of this aspect, the Rieske FeS protein includes an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 101. An additional embodiment of this aspect includes the one or more photosynthetic electron transport proteins being a cytochrome c₆ protein and a Rieske FeS protein. In a further embodiment of this aspect, the cytochrome c₆ protein includes an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 102; and the Rieske FeS protein includes an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 101.

In yet another embodiment of this present aspect, which may be combined with any of the preceding embodiments that has cytochrome c₆, the cytochrome c₆ protein is localized to a thylakoid lumen of at least one chloroplast within a cell of the genetically altered plant. A further embodiment of this aspect includes the cytochrome c₆ protein including a transit peptide that localizes the cytochrome c₆ protein to the thylakoid lumen. An additional embodiment of this aspect includes the cytochrome c₆ transit peptide being selected from the group of a chlorophyll a/b binding protein 6 transit peptide, a light-harvesting complex I chlorophyll a/b binding protein 1 transit peptide, or a plastocyanin signal peptide. In still another embodiment of this present aspect, which may be combined with any of the preceding embodiments that has Rieske FeS, the Rieske FeS protein includes a transit peptide that localizes the Rieske FeS protein to the thylakoid membrane. An additional embodiment of this aspect includes the Rieske FeS transit peptide being selected from the group of a cytochrome f transit peptide, a cytochrome b6 transit peptide, a PetD transit peptide, a PetG transit peptide, a PetL transit peptide, a PetN transit peptide, a PetM transit peptide, and a plastoquinone transit peptide. Still another embodiment of this aspect that can be combined with any of the preceding embodiments that has cytochrome c₆ further includes a cytochrome c₆ protein encoding nucleic acid sequence operably linked to a plant promoter. A further embodiment of this aspect includes the promoter being selected from a constitutive promoter, an inducible promoter, a tissue or cell type specific promoter, or an inducible, tissue or cell type specific promoter. Yet another embodiment of this aspect that can be combined with any of the preceding embodiments that has Rieske FeS further includes a Rieske FeS protein encoding nucleic acid sequence operably linked to a plant promoter. An additional embodiment of this aspect includes the promoter being selected from a constitutive promoter, an inducible promoter, a tissue or cell type specific promoter, or an inducible, tissue or cell type specific promoter.

In still another embodiment of this aspect that can be combined with any of the preceding embodiments, the one or more RuBP regeneration enhancing genetic alterations include overexpression of a CB protein. A further embodiment of this aspect includes the CB protein being a SBPase. In yet another embodiment of this aspect, the SBPase includes an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 96. A further embodiment of this aspect includes the SBPase being localized to a chloroplast stroma of at least one chloroplast within a cell of the genetically altered plant. In yet another embodiment of this aspect, the SBPase includes a transit peptide that localizes the SBPase to the chloroplast stroma. Still another embodiment of this aspect that can be combined with any of the preceding embodiments that has SBPase further includes a SBPase encoding nucleic acid sequence operably linked to a plant promoter. An additional embodiment of this aspect includes the promoter being selected from a constitutive promoter, an inducible promoter, a tissue or cell type specific promoter, or an inducible, tissue or cell type specific promoter. A further embodiment of this aspect includes the CB protein being a FBPA. In yet another embodiment of this aspect, the FBPA includes an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 97. A further embodiment of this aspect includes the FBPA being localized to a chloroplast stroma of at least one chloroplast within a cell of the genetically altered plant. In yet another embodiment of this aspect, the FBPA includes a transit peptide that localizes the FBPA to the chloroplast stroma. Still another embodiment of this aspect that can be combined with any of the preceding embodiments that has FBPA further includes a FBPA encoding nucleic acid sequence operably linked to a plant promoter. An additional embodiment of this aspect includes the promoter being selected from a constitutive promoter, an inducible promoter, a tissue or cell type specific promoter, or an inducible, tissue or cell type specific promoter. A further embodiment of this aspect includes the CB protein being a FBPase. In yet another embodiment of this aspect, the FBPase includes an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 98. A further embodiment of this aspect includes the FBPase being localized to a chloroplast stroma of at least one chloroplast within a cell of the genetically altered plant. In yet another embodiment of this aspect, the FBPase includes a transit peptide that localizes the FBPase to the chloroplast stroma. Still another embodiment of this aspect that can be combined with any of the preceding embodiments that has FBPase further includes a FBPase encoding nucleic acid sequence operably linked to a plant promoter. An additional embodiment of this aspect includes the promoter being selected from a constitutive promoter, an inducible promoter, a tissue or cell type specific promoter, or an inducible, tissue or cell type specific promoter. A further embodiment of this aspect includes the CB protein being a FBP/SBPase. An additional embodiment of this aspect includes the FBP/SBPase being a cyanobacterial FBP/SBPase. In yet another embodiment of this aspect, the cyanobacterial FBP/SBPase includes an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 99. Still another embodiment of this aspect that can be combined with any of the preceding embodiments that has FBP/SBPase includes the FBP/SBPase being localized to a chloroplast stroma of at least one chloroplast within a cell of the genetically altered plant. In a further embodiment of this aspect, the FBP/SBPase includes a transit peptide that localizes the FBP/SBPase to the chloroplast stroma. Yet another embodiment of this aspect that can be combined with any of the preceding embodiments that has FBP/SBPase further includes a FBP/SBPase encoding nucleic acid sequence operably linked to a plant promoter. An additional embodiment of this aspect includes the promoter being selected from a constitutive promoter, an inducible promoter, a tissue or cell type specific promoter, or an inducible, tissue or cell type specific promoter.

Still another embodiment of this aspect, not part of the claimed invention, that can be combined with any of the preceding embodiments that has a Rieske FeS protein encoding nucleic acid sequence includes the nucleic acid encoding the Rieske FeS protein being endogenous. An additional embodiment of this aspect, not part of the claimed invention, includes the promoter operably linked to the nucleic acid encoding the Rieske FeS protein being genetically engineered to overexpress, inducibly express, express in a specific tissue or cell type, inducibly overexpress, or inducibly express in a specific tissue or cell type the Rieske FeS protein. Still another embodiment of this aspect, not part of the claimed invention, that can be combined with any of the preceding embodiments that has a Rieske FeS protein encoding nucleic acid sequence includes the nucleic acid encoding the CB protein being heterologous.

In yet another embodiment of this aspect, which may be combined with any of the preceding embodiments, the plant has increased biomass as compared to an unaltered wild type (WT) plant. An additional embodiment of this aspect includes the plant having improved water use efficiency as compared to an unaltered WT plant when grown in conditions with light intensities above 1000 µmol m⁻² s⁻¹. A further embodiment of this aspect includes the plant being selected from the group of cowpea, soybean, cassava, rice, wheat, barley, tomato, potato, tobacco, canola, or other C3 crop plants. Still another embodiment of this aspect includes the plant being selected from the group of cowpea, soybean, cassava, rice, wheat, barley, and tobacco.

An additional aspect of the disclosure includes methods of producing the genetically altered plant of any of the preceding embodiments including (a) introducing the one or more RuBP regeneration enhancing genetic alterations that increase activity of a CB protein, the one or more photosynthetic electron transport enhancing genetic alterations, or both the one or more RuBP regeneration enhancing genetic alterations that increase activity of a CB protein and the one or more photosynthetic electron transport enhancing genetic alterations into a plant cell, tissue, or other explant; (b) regenerating the plant cell, tissue, or other explant into a genetically altered plantlet; and (c) growing the genetically altered plantlet into a genetically altered plant with the one or more RuBP regeneration enhancing genetic alterations that increase activity of a CB protein, the one or more photosynthetic electron transport enhancing genetic alterations, or both the one or more RuBP regeneration enhancing genetic alterations that increase activity of a CB protein and the one or more photosynthetic electron transport enhancing genetic alterations.

### BRIEF DESCRIPTION OF THE DRAWINGS

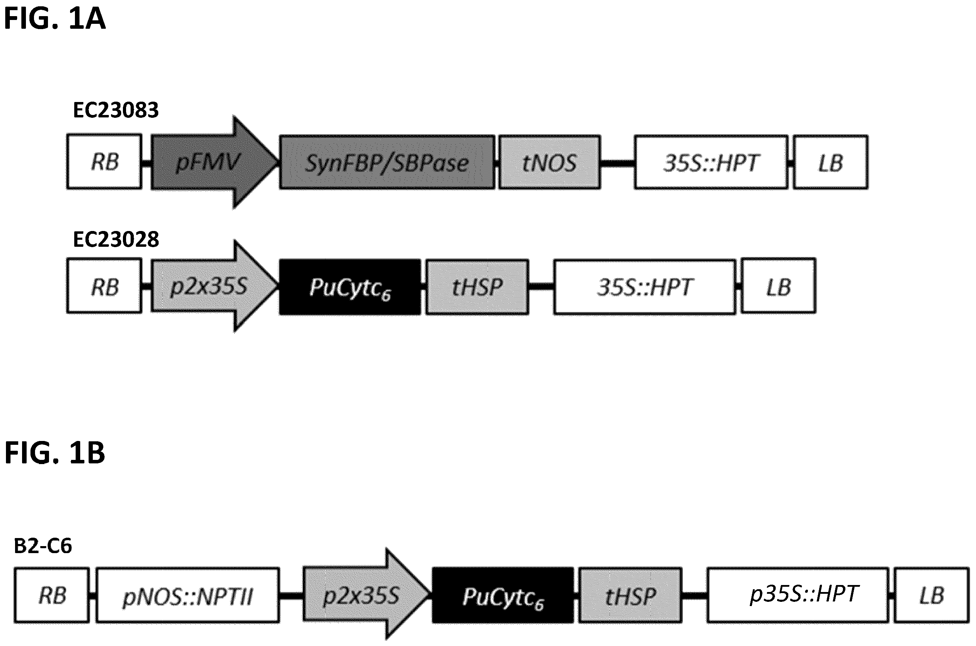

**FIGS. 1A-1B** show schematic representations of the constructs used to generate transgenic *N. tabacum* lines. **FIG. 1A** shows the construct (on the top, EC23083) used for expression of FBP/SBPase (SynFBP/SBPase) and the construct (on the bottom, EC23028) used for expression of *Porphyra umbilicalis* cytochrome *c*₆ (PuCytc₆) in *N. tabacum* cv. Petit Havana. **FIG. 1B** shows the construct (B2-C6) used for expression of cytochrome c*₆* (PuCytc₆) in *N. tabacum* cv. Samsun. RB = T-DNA right border; pFMV = figwart mosaic virus promoter; tNOS = nopaline synthase terminator; 35S = cauliflower mosaic virus 35S promoter; HPT = *A. thaliana* heat shock protein 18.2 (HSP) terminator; LB = T-DNA left border; p2x35S = 2x cauliflower mosaic virus 35S promoter; tHSP = *A. thaliana* heat shock protein 18.2 (HSP) terminator; pNos = nopaline synthase promoter; NPT II = neomycin phosphotransferase gene.

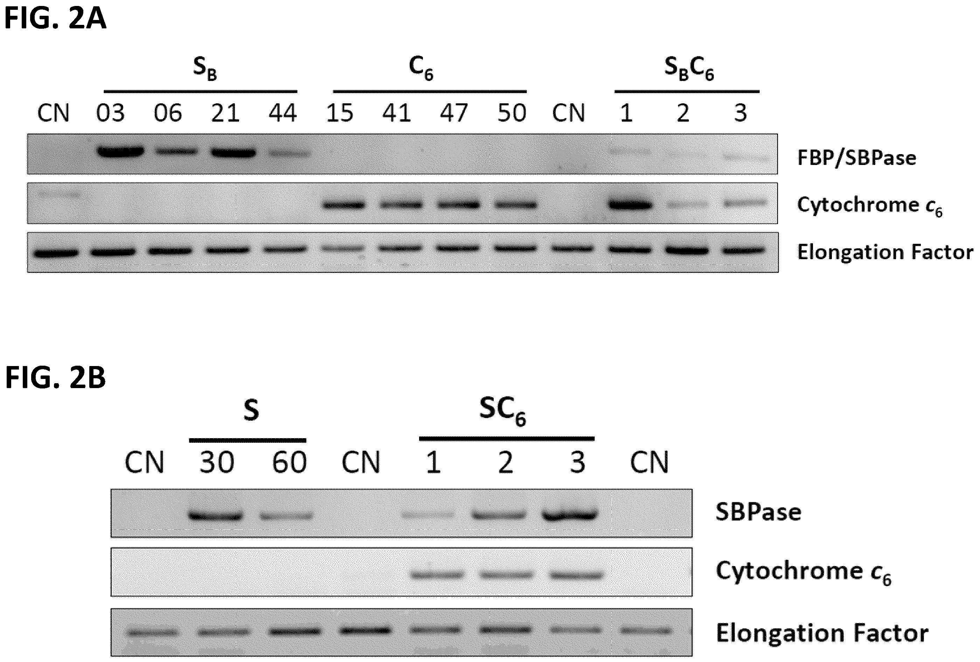

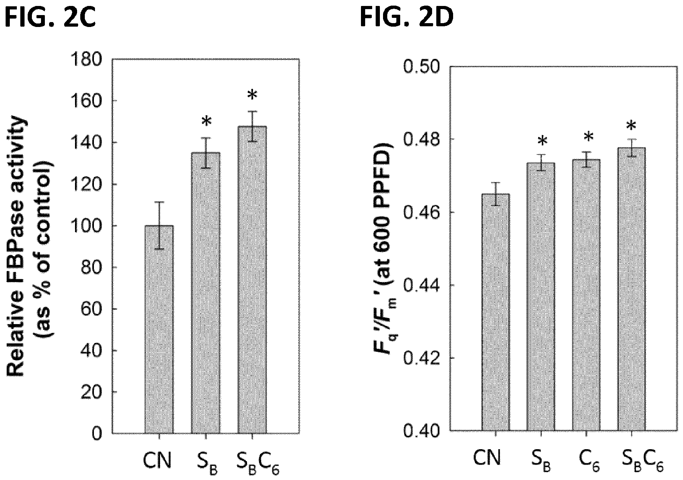

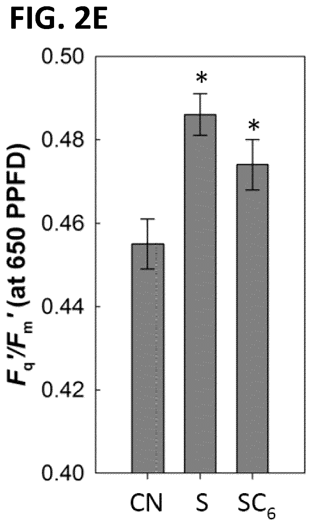

**FIGS. 2A-2E** show screening of transgenic plants overexpressing FBP/SBPase, SBPase, and cytochrome *c*₆. **FIG. 2A** shows transcript levels in S_{B} lines *(N. tabacum* cv. Petit Havana lines expressing FBP/SBPase; S_{B} lines 03, 06, 21, and 44), C₆ lines *(N. tabacum* cv. Petit Havana lines expressing cytochrome *c*₆; C₆ lines 15, 41, 47, and 50), S_{B}C₆ lines (*N*. *tabacum* cv. Petit Havana lines expressing FBP/SBPase and cytochrome *c*₆; S_{B}C₆ lines 1, 2, and 3), and control lines (CN; both WT and azygous plants). **FIG. 2B** shows transcript levels in S lines *(N. tabacum* cv. Samsun lines expressing SBPase; S lines 30 and 60), SC₆ lines (*N. tabacum* cv. Samsun lines expressing SBPase and cytochrome *c*₆; SC₆ lines 1, 2, and 3), and control lines (CN; both WT and azygous plants). **FIG. 2C** shows FBPase activity in S_{B} lines and S_{B}C₆ lines relative to control (CN; both WT and azygous plants). **FIGS. 2D-2E** show chlorophyll fluorescence imaging of plants grown in controlled environmental conditions used to determine F_{q}'/Fₘ' (maximum PSII operating efficiency) at 600-650 µmol m⁻² s⁻¹ (PPFD). **FIG. 2D** shows the maximum PSII operating efficiency of control (CN; both WT and azygous plants), S_{B}, C₆, and S_{B}C₆ lines (6 plants per line; 3-4 lines per manipulation) at 600 PPFD. **FIG. 2E** shows the maximum PSII operating efficiency of control (CN; WT plants), S and SC₆ lines (CN = 11 plants; S and SC₆ lines = 6-7 plants per manipulation) at 650 PPFD. In **FIGS. 2C-2E****,** asterisks indicate lines which are statistically different to control groups (*P < 0.05).

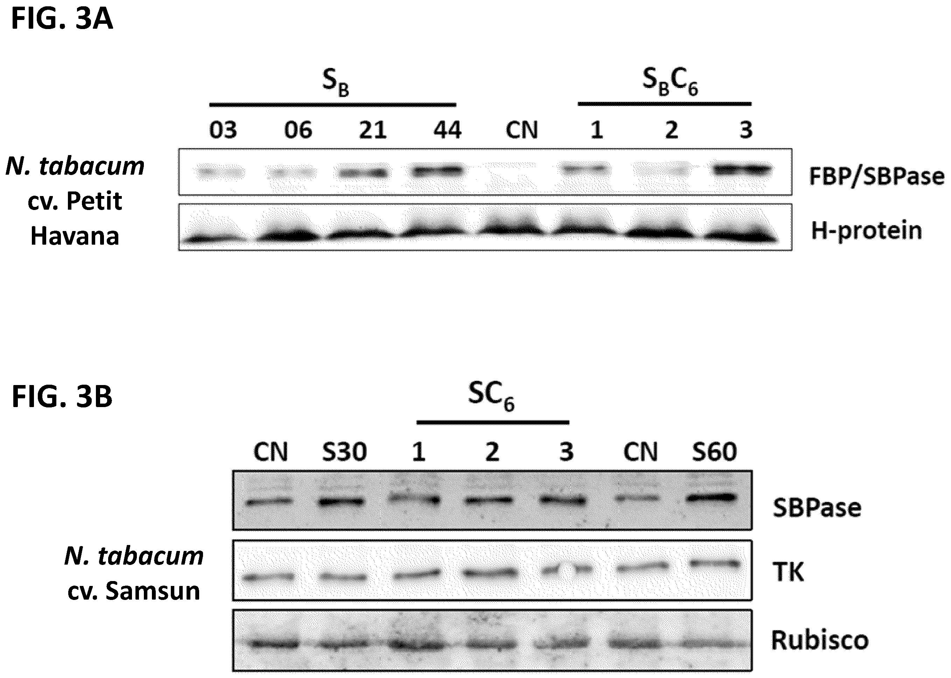

**FIGS. 3A-3B** show biochemical analysis of the transgenic *N. tabacum* cv. Petit Havana and *N. tabacum* cv. Samsun plants. **FIG. 3A** shows immunoblot analysis of protein extracts representative of multiple experiments from mature leaves of *N. tabacum* cv. Petit Havana lines expressing FBP/SBPase (S_{B} lines 03, 06, 21, and 44) and FBP/SBPase + cytochrome *c*₆ (S_{B}C₆ lines 1, 2, and 3) compared to extracts from wild type (WT) control plants (CN), and blotted against FBP/SBPase antibody. The expression of H-protein from the glycine cleavage system was used as a loading control. **FIG. 3B** shows immunoblot analysis of protein extracts representative of multiple experiments from mature leaves of *N. tabacum* cv. Samsun lines expressing SBPase (S lines S30 and S60) and SBPase + cytochrome *c*₆ (SC₆ lines 1, 2, and 3) compared to extracts from WT control plants (CN), and blotted against SBPase antibody. In **FIGS. 3A-3B****,** expression of H-protein from the glycine cleavage system (H-protein), transketolase (TK), and Rubisco were used as loading controls. Immunoblot analysis was repeated for multiple sets of plants, and results shown are representative of typical blots.

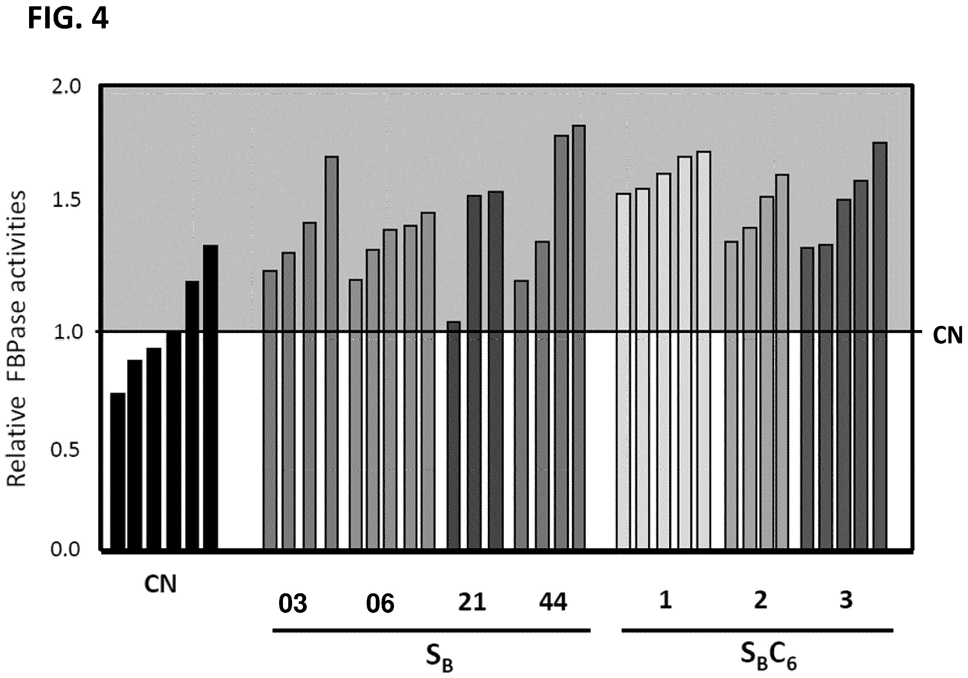

**FIG. 4** shows the complete data set of the FBPase enzyme assays in the analyzed *N. tabacum* cv. Petit Havana plants shown in **FIG. 2C****.** Bars represent FBPase activities in the transgenic lines tested relative to FBPase activities in controls (both WT and azygous plants). Each bar is an individual plant from S_{B} lines expressing FBP/SBPase (S_{B}03, S_{B}06, S_{B}21, S_{B}44; shown in middle and labeled "S_{B}"), S_{B}C₆ lines expressing FBP/SBPase + cytochrome *c*₆ (S_{B}C1, S_{B}C2, S_{B}C3; shown on right and labeled "S_{B}C₆"), and control lines (CN; both WT and azygous plants; shown in black on left). The average control activity is shown as a black horizontal bar at 1.0 relative FBPase activity and labeled "CN".

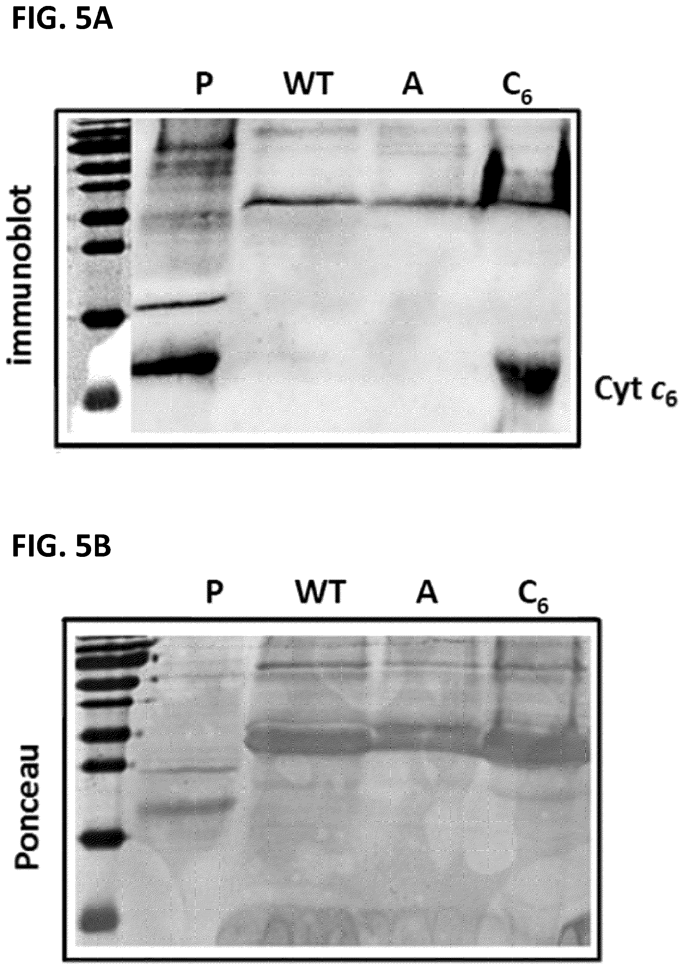

**FIGS. 5A-5B** show biochemical analysis of the transgenic *N. tabacum* cv. Petit Havana plants expressing cytochrome *c*₆. **FIG. 5A** shows an immunoblot analysis of protein extracts from pools of developing leaves of C₆ lines (C15, C41, and C47), WT control lines, and null segregant (A) control lines, as well as a *Porphyra umbilicalis* crude protein extract (P). **FIG. 5B** shows a Ponceau stain of the immunoblot membrane in **FIG. 5A****,** demonstrating similar loading levels of plant leaf extracts in **FIG. 5A****.**

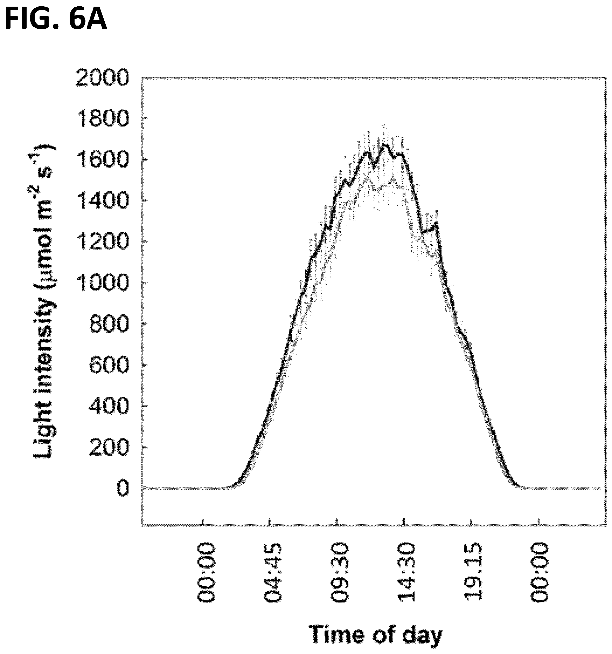

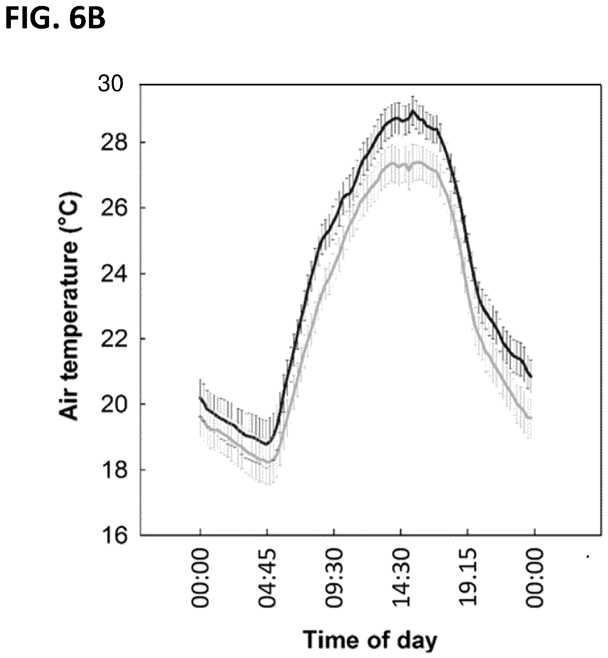

**FIGS. 6A-6B** show average environmental conditions during 2017 field experiments *(i.e.,* experiments assessing field-grown plants). **FIG. 6A** shows average daily light intensity (µmol m⁻² s⁻¹) from 2017 field experiments. **FIG. 6B** shows air temperature (°C) from 2017 field experiments. For **FIGS. 6A-6B****,** black = 2017 experiment 1 and grey = 2017 experiment 2.

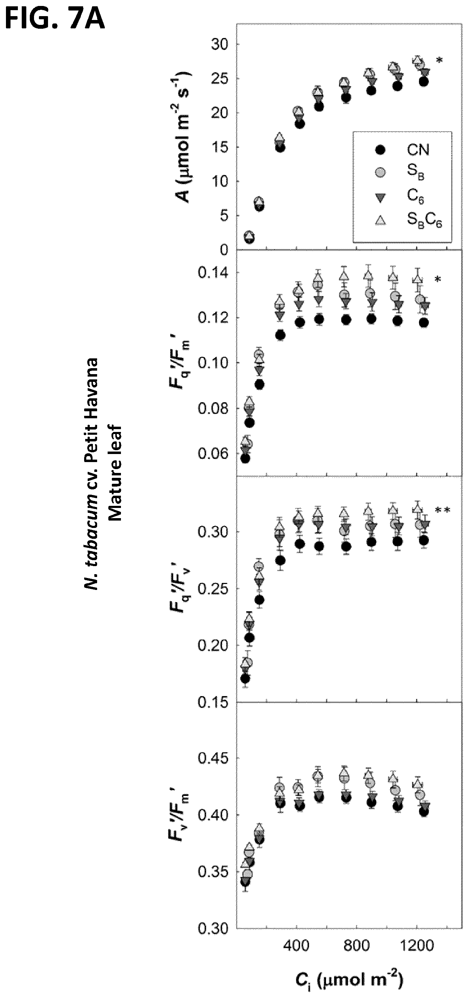

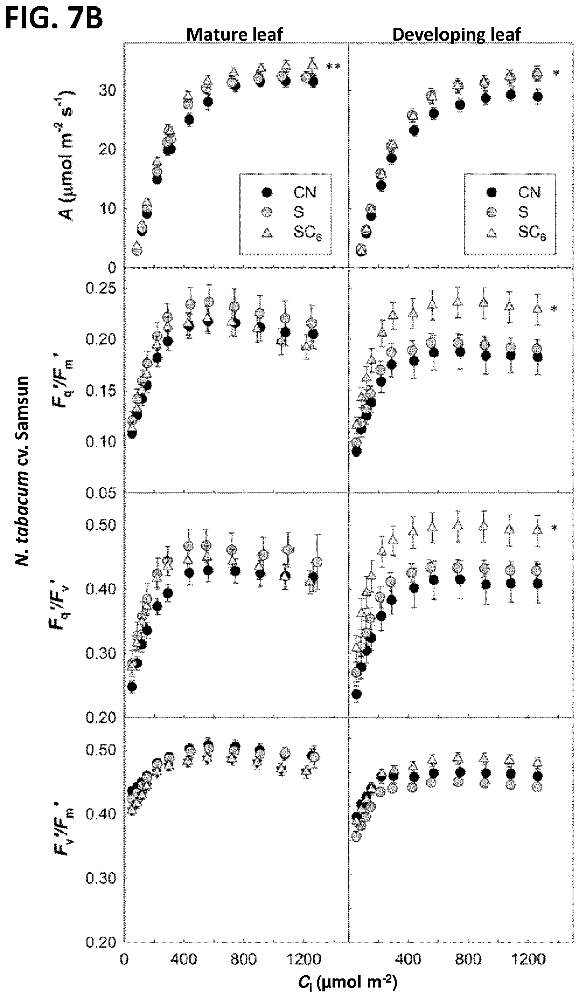

**FIGS. 7A-7B** show photosynthetic responses of transgenic plants grown under controlled conditions *(i.e.,* in the glasshouse (GH)). **FIGS. 7A-7B** show photosynthetic carbon fixation rates (A (µmol m⁻² s⁻¹)), actual operating efficiency of PSII in the light (F_{q}'/Fₘ'), electron sinks pulling away from PSII (F_{q}' /Fᵥ'), and PSII maximum efficiency (Fᵥ'/Fₘ'). Parameters were determined as a function of increasing CO₂ concentrations (*C*ᵢ (µmol m⁻²)) at saturating light levels (natural light levels in the glasshouse oscillated between 400 µmol m⁻² s⁻¹ and 1000 µmol m⁻² s⁻¹; supplemental light was provided as necessary to maintain a minimum irradiance level of 400 µmol m⁻² s⁻¹). **FIG. 7A** shows photosynthetic responses of mature leaves of *N. tabacum* cv. Petit Havana lines expressing FBP/SBPase (S_{B}), cytochrome *c*₆ (C₆), FBP/SBPase + cytochrome *c*₆ (S_{B}C₆), and control (CN; both WT and azygous plants). **FIG. 7B** shows photosynthetic responses of mature leaves (left column) and developing leaves *(i.e.,* 11-13 cm in length; right column) of *N. tabacum* cv. Samsun lines expressing SBPase (S), SBPase + cytochrome *c*₆ (SC₆), and control (CN; both WT and azygous plants). In **FIGS. 7A-7B****,** 3-4 individual plants from 3-4 independent transgenic lines were evaluated. Asterisks indicate significance between transgenics and control group determined using a linear mixed-effects model and type III ANOVA, *P < 0.05, **P < 0.01, ***P < 0.001.

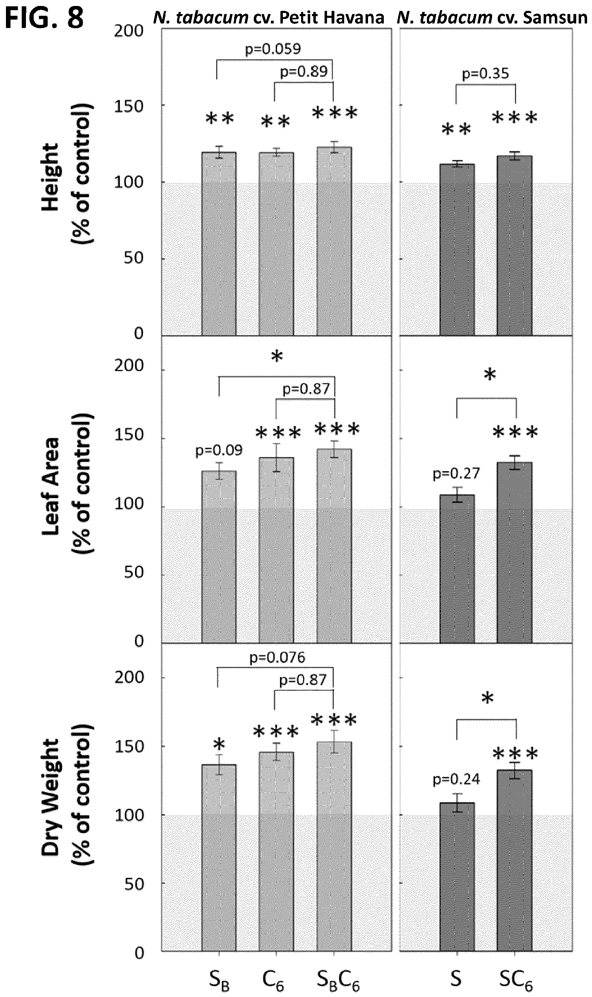

**FIG. 8** shows that increased expression of SBPase or expression of FBP/SBPase + cytochrome *c*₆ increases biomass in plants grown under controlled conditions *(i.e.,* in the glasshouse (GH)). The left column of graphs shows the mean ± SE of plant height, leaf area, and above-ground biomass dry weight displayed as a percentage of control values for forty-day-old *N. tabacum* cv. Petit Havana lines expressing FBP/SBPase (S_{B}), cytochrome *c*₆ (C₆), and FBP/SBPase + cytochrome c6 (S_{B}C₆). The right column of graphs shows the mean ± SE of plant height, leaf area, and above-ground biomass dry weight displayed as a percentage of control values for fifty-six-day-old *N. tabacum* cv. Samsun lines expressing SBPase (S) and SBPase + cytochrome *c*₆ (SC₆). 5-6 individual plants from 2-4 independent transgenic lines were evaluated. The values obtained for the control groups, which contained both WT and azygous plants, are shown as grey shading set to 100% and overlaid on the graphs. Asterisks indicate significance between transgenics and control group or between genotypes determined using ANOVA with Tukey's post hoc test, *P < 0.05, **P < 0.01, ***P < 0.001.

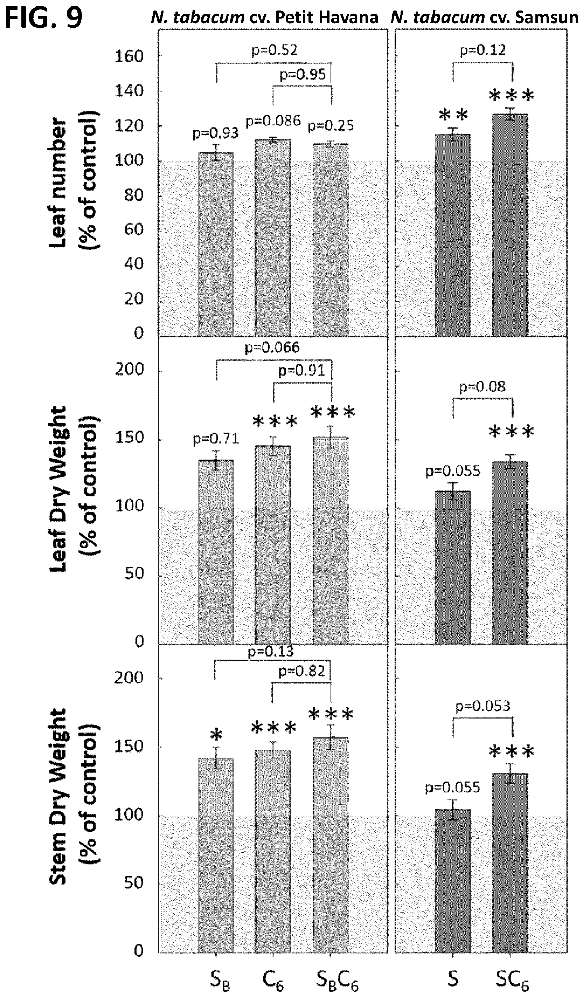

**FIG. 9** shows that increased expression of SBPase, or expression of FBP/SBPase + cytochrome *c*₆, causes an increase in the biomass of GH grown plants. The left column of graphs shows the mean ± SE of leaf number, leaf dry weight, and stem dry weight displayed as a percentage of control values for forty-day-old *N. tabacum* cv. Petit Havana lines expressing FBP/SBPase (S_{B}), cytochrome *c*₆ (C₆), and FBP/SBPase + cytochrome *c*₆ (S_{B}C₆). The right column of graphs shows the mean ± SE of leaf number, leaf dry weight, and stem dry weight displayed as a percentage of control values for fifty-six-day-old *N. tabacum* cv. Samsun lines expressing SBPase (S) and SBPase + cytochrome *c*₆ (SC₆). 5-6 individual plants from 2-4 independent transgenic lines were evaluated. The values obtained for the control groups, which contained both WT and azygous plants, are shown as grey shading set to 100% and overlaid on the graphs. Asterisks indicate significance between transgenics and control group or between genotypes determined using ANOVA with Tukey's post hoc test, *P < 0.05, **P < 0.01, ***P < 0.001.

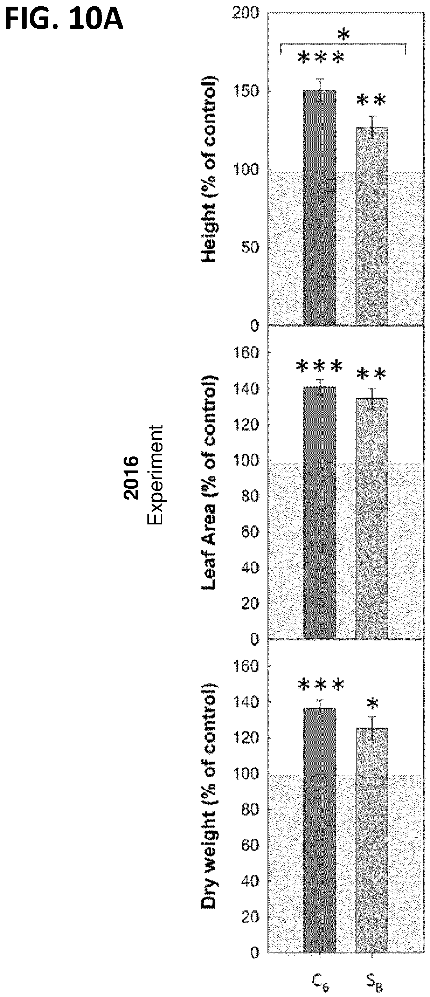

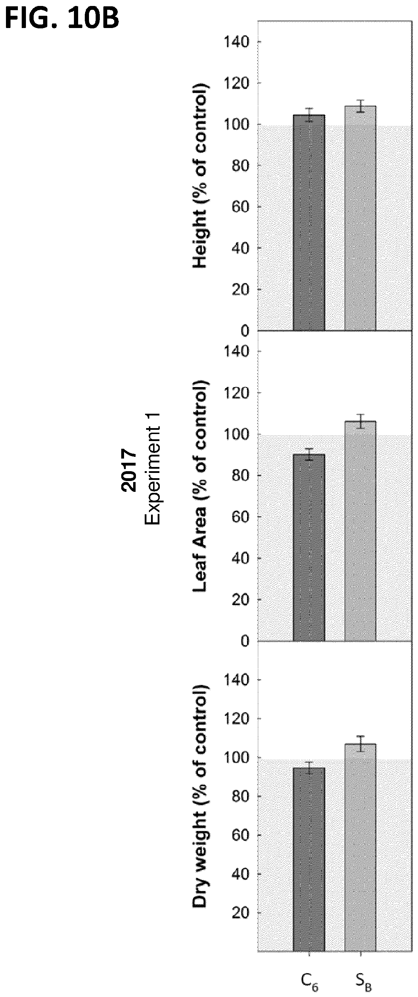

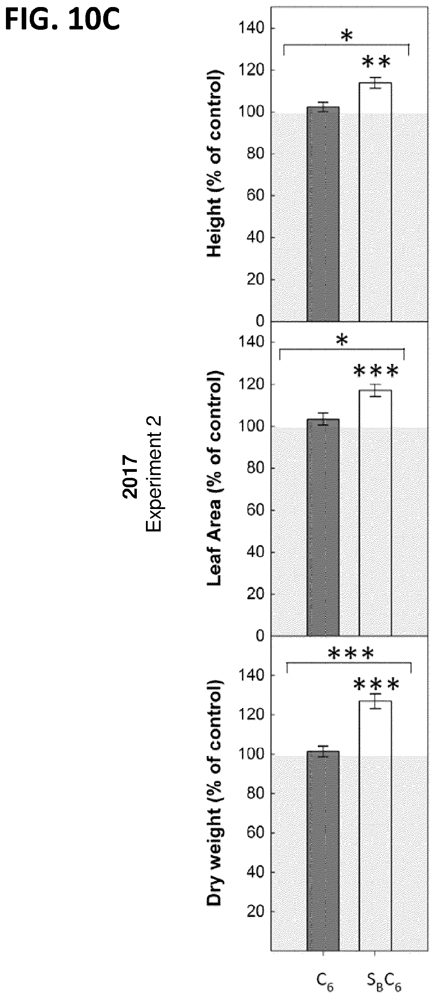

**FIGS. 10A-10C** show that simultaneous expression of FBP/SBPase + cytochrome *c*₆ increases biomass in field grown plants. **FIG. 10A** shows the mean ± SE of plant height, leaf area, and above-ground biomass dry weight displayed as a percentage of control values for forty-day-old *(i.e.,* young) 2016 field-grown *N. tabacum* cv. Petit Havana plants expressing cytochrome *c*₆ (C₆) or FBP/SBPase (S_{B}). **FIG. 10B** shows the mean ± SE of plant height, leaf area, and above-ground biomass dry weight displayed as a percentage of control values for fifty-seven-day-old field-grown *N. tabacum* cv. Petit Havana plants expressing FBP/SBPase (S_{B} lines; light grey bars) or cytochrome *c*₆ (C₆ lines; dark grey bars). **FIG. 10C** shows the mean ± SE of plant height, leaf area, and above-ground biomass dry weight displayed as a percentage of control values for sixty-one-day-old (*i.e..,* flowering) field-grown *N. tabacum* cv. Petit Havana plants expressing cytochrome *c*₆ (C₆ lines; dark grey bars) or FBP/SBPase + cytochrome *c*₆ (S_{B}C₆ lines; white bars). 6 individual plants from 2-3 independent transgenic lines **(****FIG. 10A****)** or 24 individual plants from 2-3 independent transgenic lines **(****FIGS. 10B-10C****)** were evaluated. The values obtained for the control groups, which contained both WT and azygous plants, are shown as grey shading set to 100% and overlaid on the graphs. Asterisks indicate significance between transgenics and control group, or between genotypes using ANOVA with Tukey's post hoc test, *P < 0.05, **P < 0.01, ***P < 0.001.

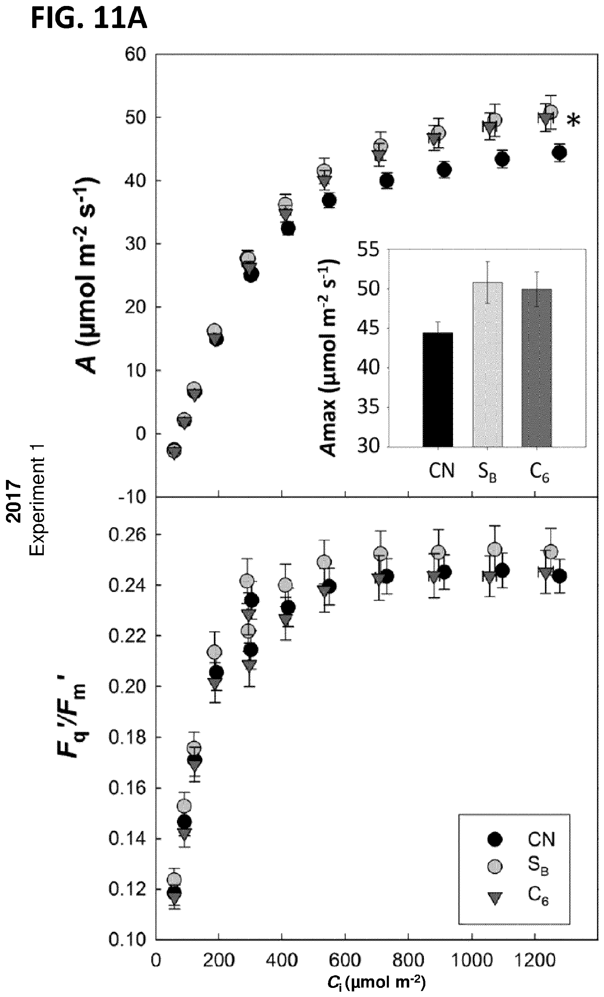

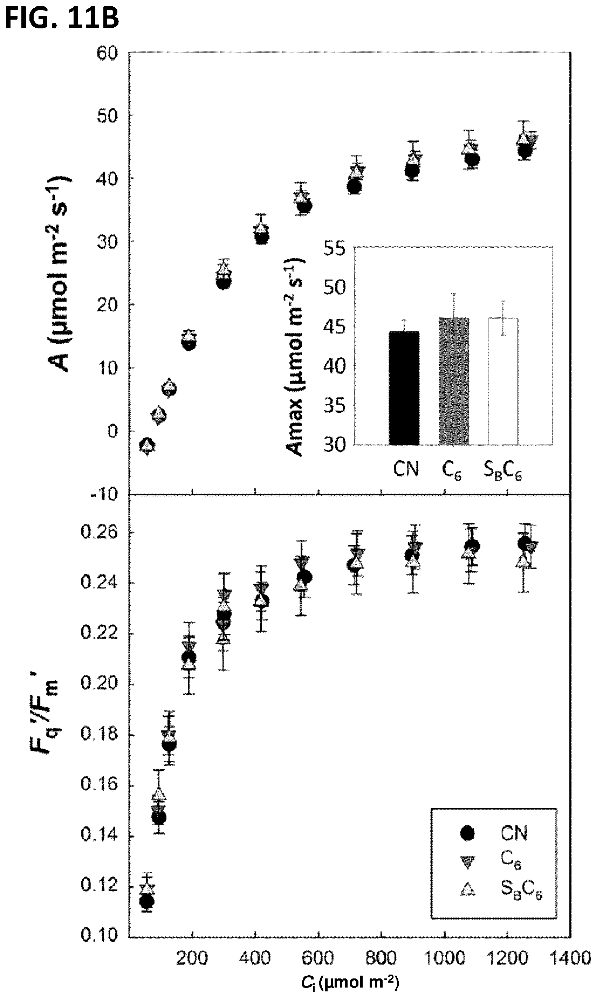

**FIGS. 11A-11B** show photosynthetic capacity of field-grown transgenic plants. **FIG. 11A** shows photosynthetic carbon fixation rates (*A* (µmol m⁻² s⁻¹)) and operating efficiency of PSII (F_{q}'/Fₘ') as a function of increasing CO₂ concentrations (Cᵢ (µmol m⁻²)) at saturating light levels in mature leaves from field-grown *N. tabacum* cv. Petit Havana lines expressing FBP/SBPase (S_{B}), cytochrome *c*₆ (C₆), and control plants (CN; both WT and azygous plants). The inset bar graph shows the maximum carbon fixation rate (*A*ₘₐₓ) for mature leaves from field grown S_{B} and C₆ *N. tabacum* cv. Petit Havana and CN lines. **FIG. 11B** shows photosynthetic carbon fixation rates (*A* (µmol m⁻² s⁻¹)) and operating efficiency of PSII (F_{q}'/Fₘ') as a function of increasing CO₂ concentrations (Cᵢ (µmol m⁻²)) at saturating light levels in mature leaves from field-grown *N. tabacum* cv. Petit Havana lines expressing cytochrome *c*₆ (C₆), FBP/SBPase + cytochrome *c*₆ (S_{B}C₆), and control plants (CN; both WT and azygous). The inset bar graph shows the maximum carbon fixation rate (*A*ₘₐₓ) for mature leaves from field grown C₆ and S_{B}C₆ *N. tabacum* cv. Petit Havana and CN lines. In **FIGS. 11A-11B****,** the mean ± SE of 4-5 individual plants from 2-3 independent transgenic lines is presented. Asterisk indicates significance between transgenics and control group as determined by a linear mixed-effects model and type III ANOVA, *P < 0.05.

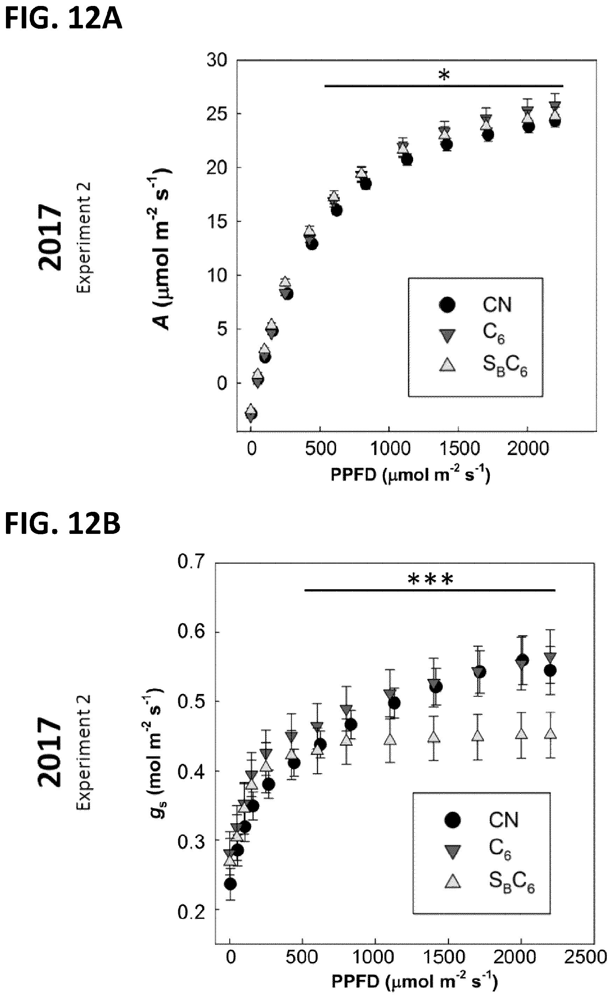

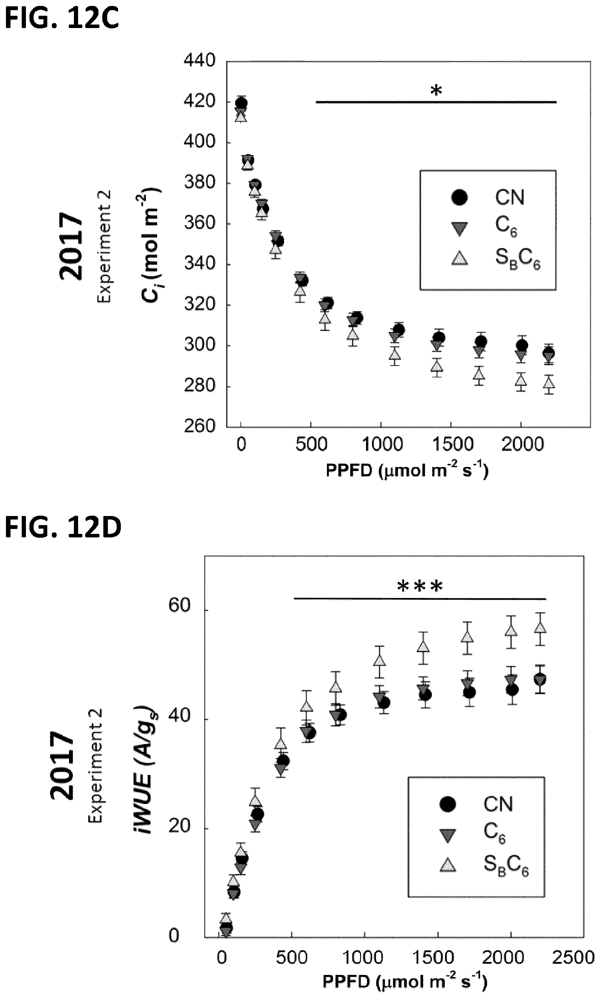

**FIGS. 12A-12D** show that simultaneous expression of FBP/SBPase + cytochrome *c*₆ increases water use efficiency under field conditions. **FIG. 12A** shows the mean ± SE net CO₂ assimilation rate (*A* (µmol m⁻² s⁻¹)), **FIG. 12B** shows the mean ± SE stomatal conductance (gₛ (mol m⁻² s⁻¹)), **FIG. 12C** shows the mean ± SE intercellular CO₂ concentration (Cᵢ (µmol m⁻²)), and **FIG. 12D** shows the mean ± SE intrinsic water-use efficiency *(iWUE* (*A*/gₛ))*.* The parameters shown in **FIGS. 12A-12D** are provided as a function of light (PPFD (µmol m⁻² s⁻¹)) in field-grown *N. tabacum* cv. Petit Havana lines expressing cytochrome c₆ (C₆), FBP/SBPase + cytochrome *c*₆ (S_{B}C₆), and control plants (CN; both WT and azygous). 4-5 individual plants from 2-3 independent transgenic lines were evaluated. Asterisks indicate significance between transgenic lines and control group determined using a linear mixed-effects model and type III ANOVA, *P < 0.05, **P < 0.01, and ***P < 0.001.

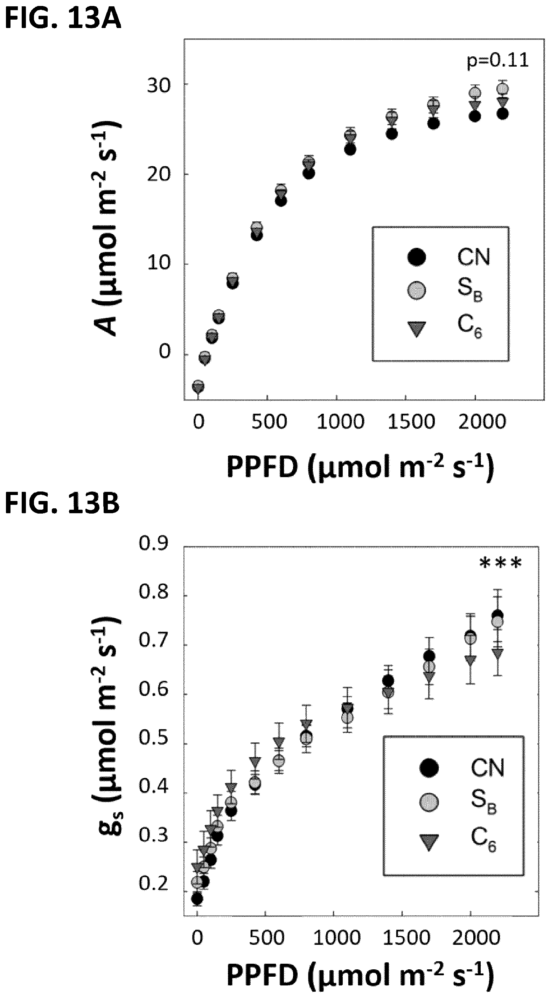

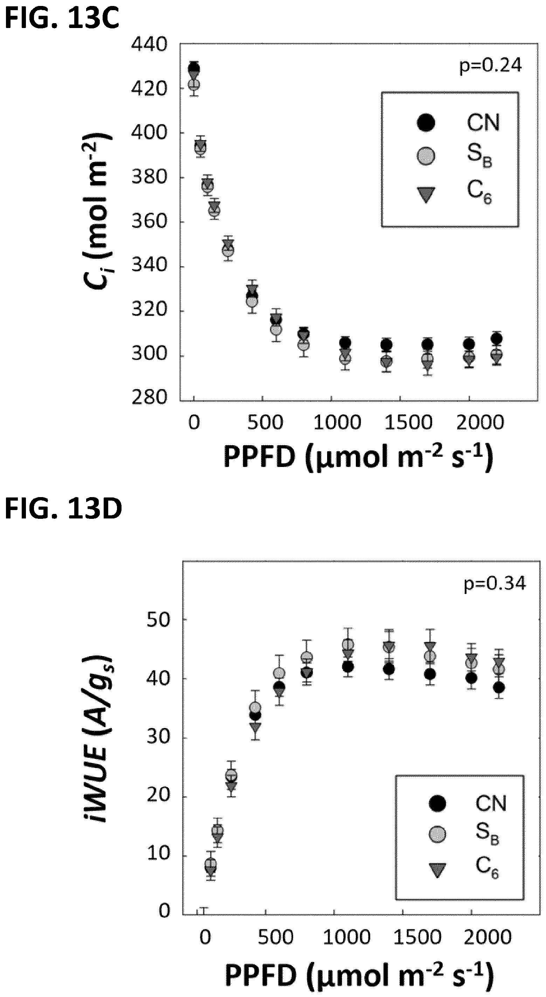

**FIGS. 13A-13D** show the response of gas exchange parameters to absorbed light intensity in *N. tabacum* cv. Petit Havana plants expressing FBP/SBPase or cytochrome *c*₆ in the 2017 field experiment 1. **FIG. 13A** shows net CO₂ assimilation rate (A (µmol m⁻² s⁻¹)), **FIG. 13B** shows stomatal conductance (gₛ (mol m⁻² s⁻¹)), **FIG. 13C** shows intercellular CO₂ concentration (Cᵢ (µmol m⁻²)), and **FIG. 13D** shows intrinsic water-use efficiency (*iWUE* (*A*/gₛ)). The parameters shown in **FIGS. 13A-13D** are provided as a function of light (PPFD (µmol m⁻² s⁻¹)) in field-grown *N. tabacum* cv. Petit Havana lines expressing FBP/SBPase (S_{B}), cytochrome *c*₆ (C₆), and control plants (CN; both WT and azygous plants). 4-5 individual plants from 2-3 independent transgenic lines were evaluated and the means ± SE are presented. Asterisks indicate significance between groups determined using a linear mixed-effects model and type III ANOVA, *P < 0.05, **P < 0.01, ***P < 0.001.

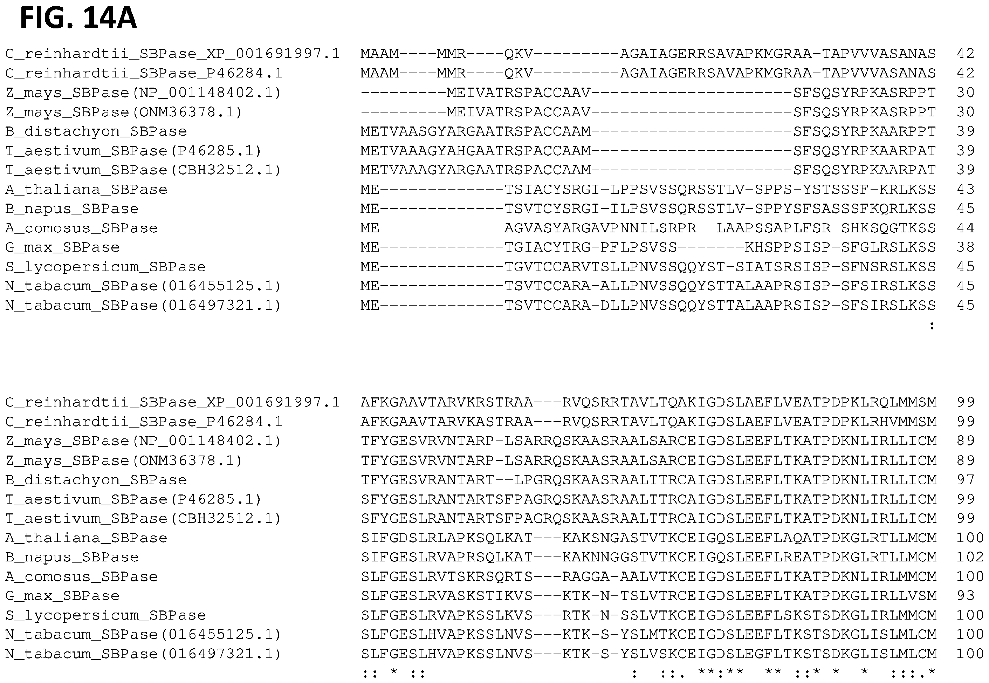

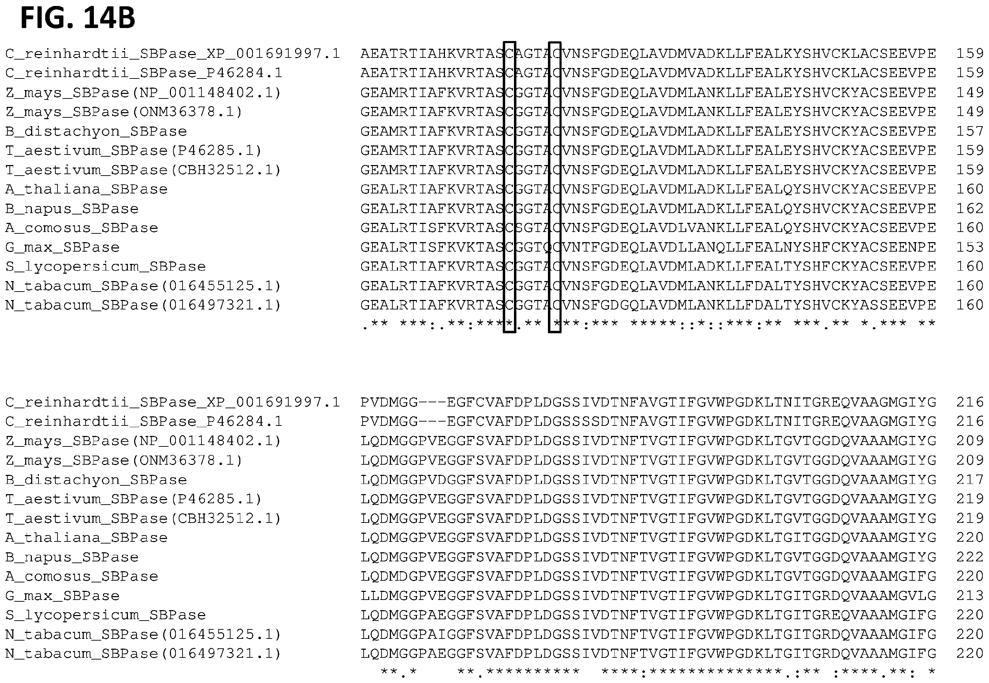

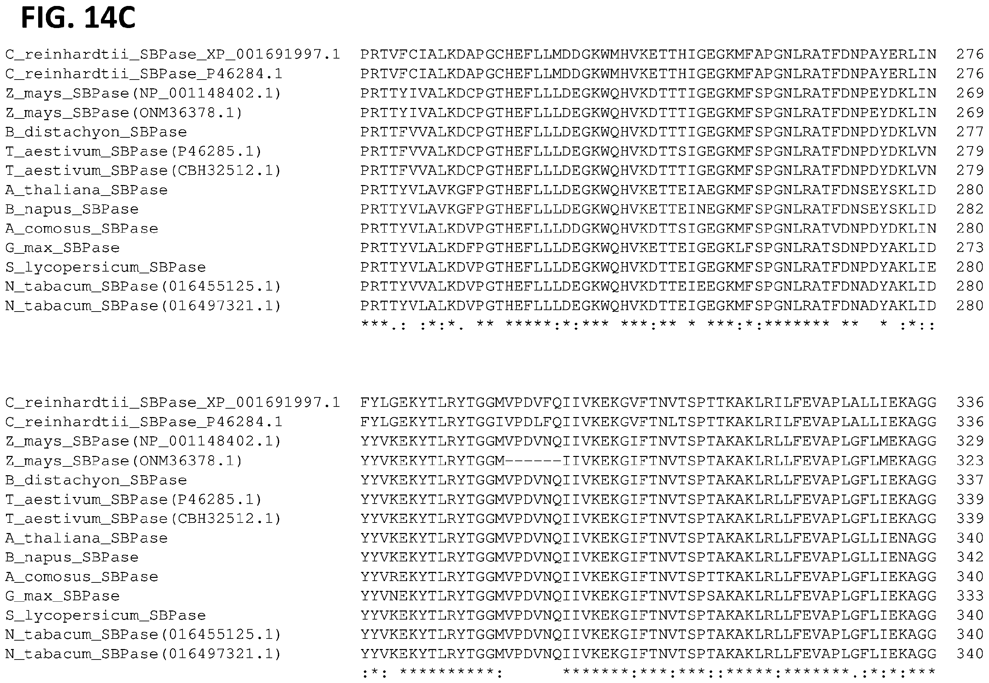

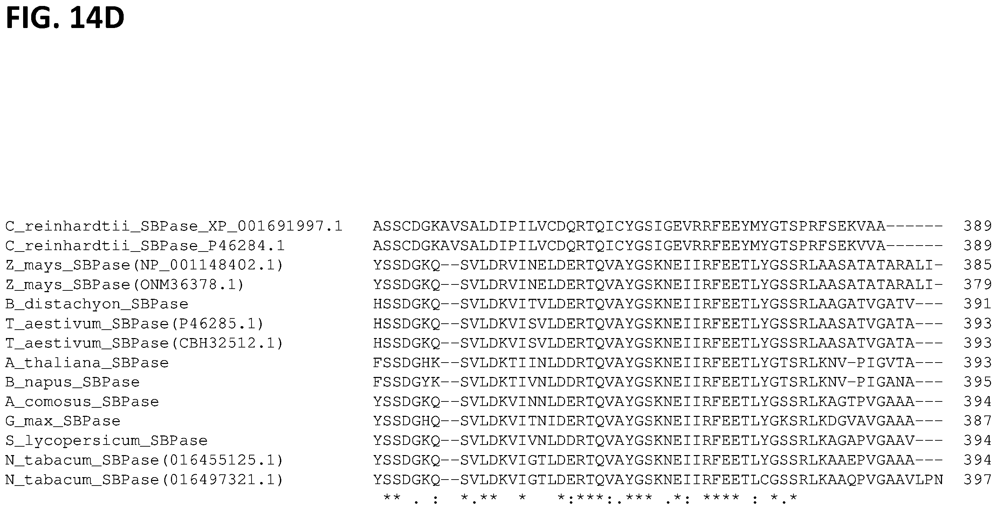

**FIGS. 14A-14D** show the alignment of SBPase polypeptide sequences from *Chlamydomonas reinhardtii* (C_reinhardtii_SBPase_XP_001691997.1 (SEQ ID NO: 13); C reinhardtii_SBPase_P46284.1 (SEQ ID NO: 14)), *Zea mays* (Z_mays_SBPase_NP_001148402.1 (SEQ ID NO: 10); Z_mays_SBPase_ONM36378.1 SEQ ID NO: 11)), *Brachypodium distachyon* (SEQ ID NO: 9), *Triticum aestivum* (T_aestivum_SBPase_P46285.1 (SEQ ID NO: 7); T_aestivum_SBPase_CBH32512.1 (SEQ ID NO: 8)), *Arabidopsis thaliana* (SEQ ID NO: 1), *Brassica napus* (SEQ ID NO: 2), *Ananas comosus* (SEQ ID NO: 6), *Glycine max* (SEQ ID NO: 12), *Solanum lycopersicum* (SEQ ID NO: 3), and *Nicotiana tabacum* (N_tabacum_SBPase_016455125.1 (SEQ ID NO: 4); N_tabacum_SBPase_016497321.1 (SEQ ID NO: 5)). **FIG. 14A** shows the alignment of the N terminal portion of the SBPase polypeptide. **FIG. 14B** shows the alignment of the first part of the central portion of the SBPase polypeptide (boxes indicate cysteine residues to be mutated for producing plants with non-TRx (redox) activated SBPase). **FIG. 14C** shows the alignment of the second part of the central portion of the SBPase polypeptide. **FIG. 14D** shows the C terminal portion of the SBPase polypeptide.

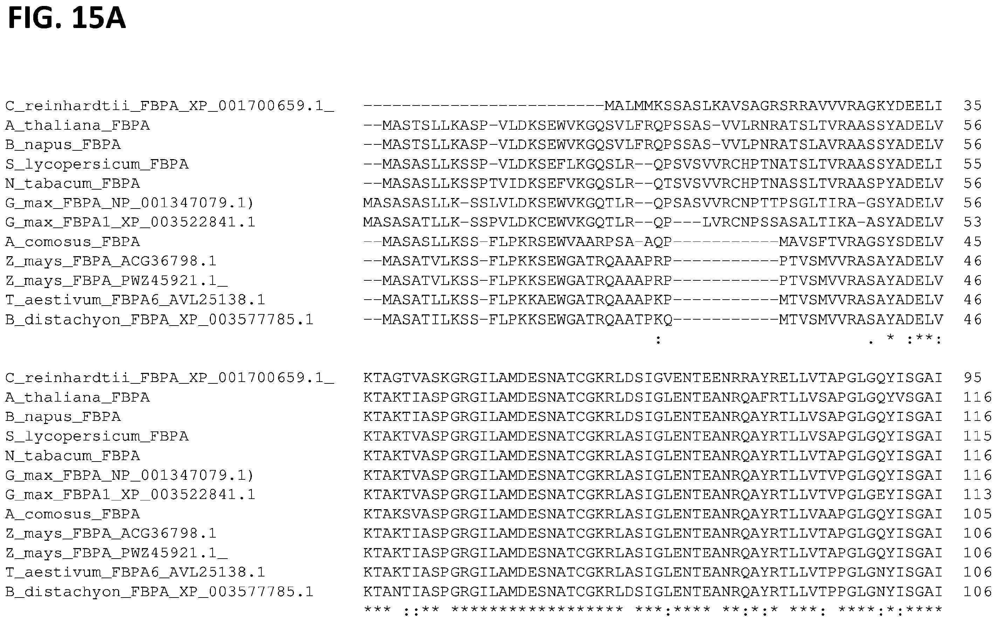

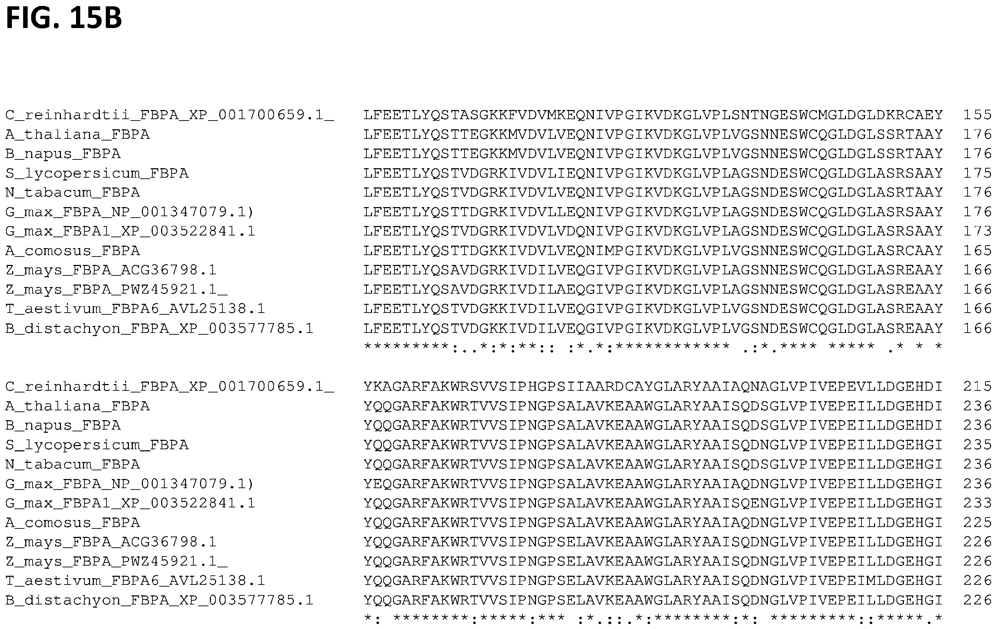

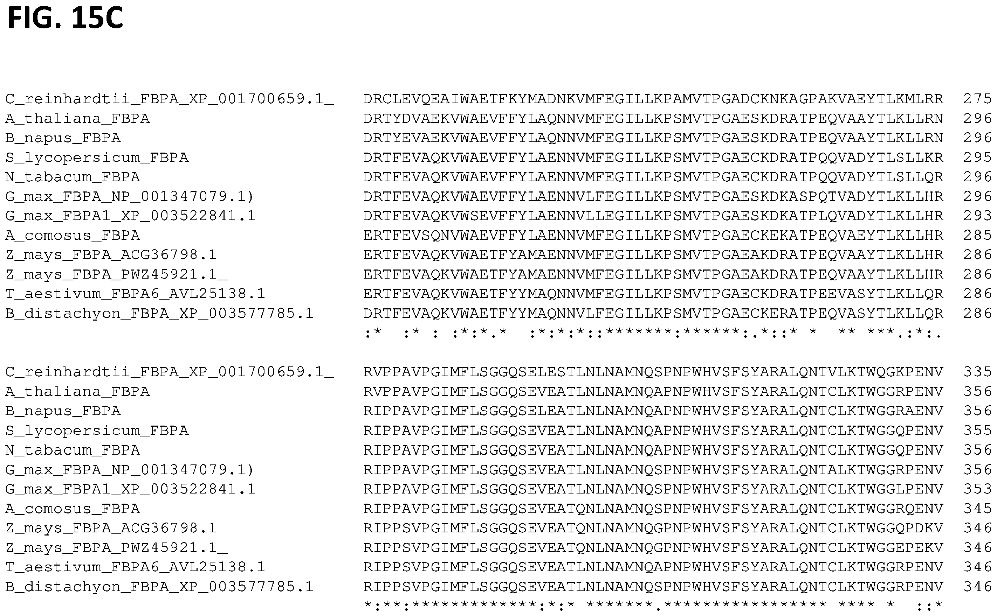

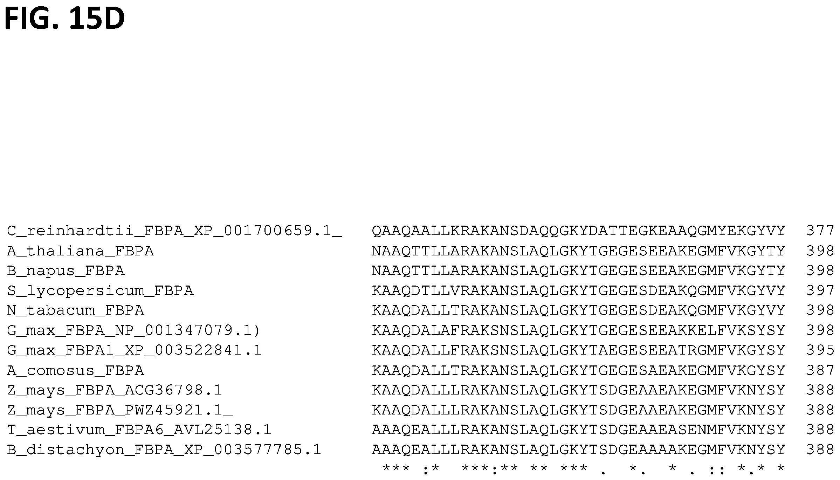

**FIGS. 15A-15D** show the alignment of FBPA polypeptide sequences from *Chlamydomonas reinhardtii* (SEQ ID NO: 26), *Arabidopsis thaliana* (SEQ ID NO: 17), *Brassica napus* (SEQ ID NO: 18), *Solanum lycopersicum* (SEQ ID NO: 15), *Nicotiana tabacum* (SEQ ID NO: 16), *Glycine max* (G_max_FBPA_NP_001347079.1 (SEQ ID NO: 22); G_max_FBPA1_XP_003522841.1 (SEQ ID NO: 23)), *Ananas comosus* (SEQ ID NO: *24), Zea mays (Z_mays_FBPA_ACG36798.1* (SEQ ID NO: 19); Z_mays_FBPA_PWZ45921.1 (SEQ ID NO: 20)), *Triticum aestivum* (SEQ ID NO: 21), and *Brachypodium distachyon* (SEQ ID NO: 25). **FIG. 15A** shows the alignment of the N terminal portion of the FBPA polypeptide. **FIG. 15B** shows the alignment of the first part of the central portion of the FBPA polypeptide. **FIG. 15C** shows the alignment of the second part of the central portion of the FBPA polypeptide. **FIG. 15D** shows the alignment of the C terminal portion of the FBPA polypeptide.

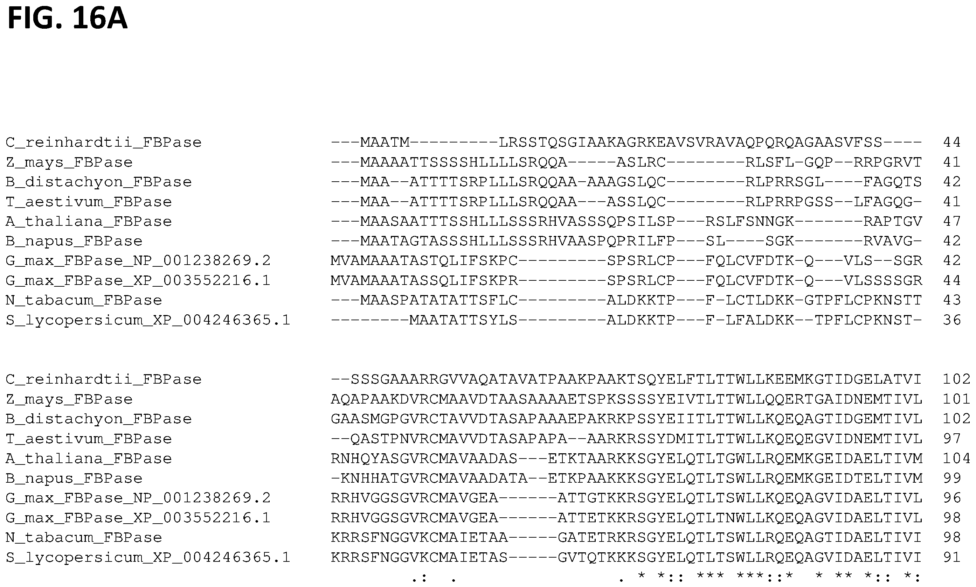

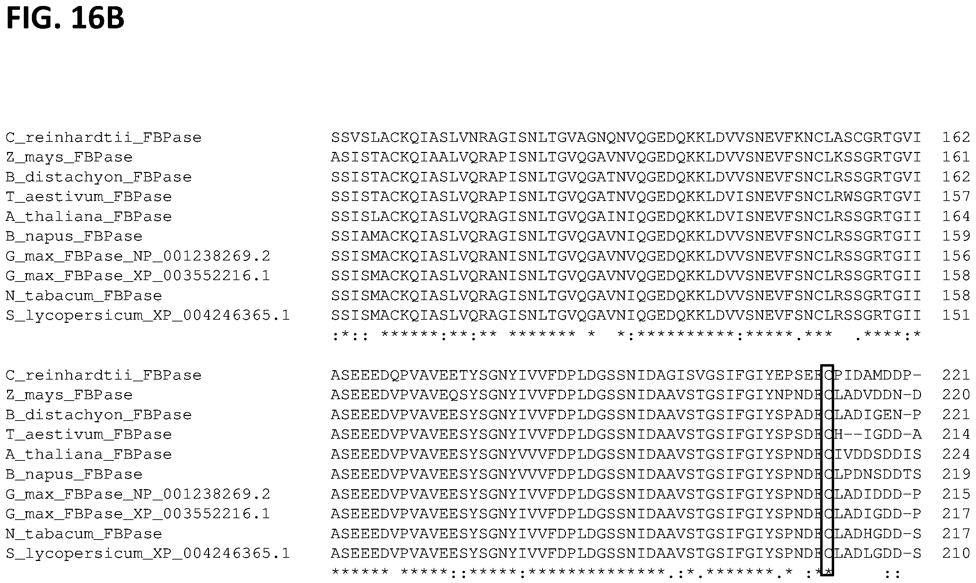

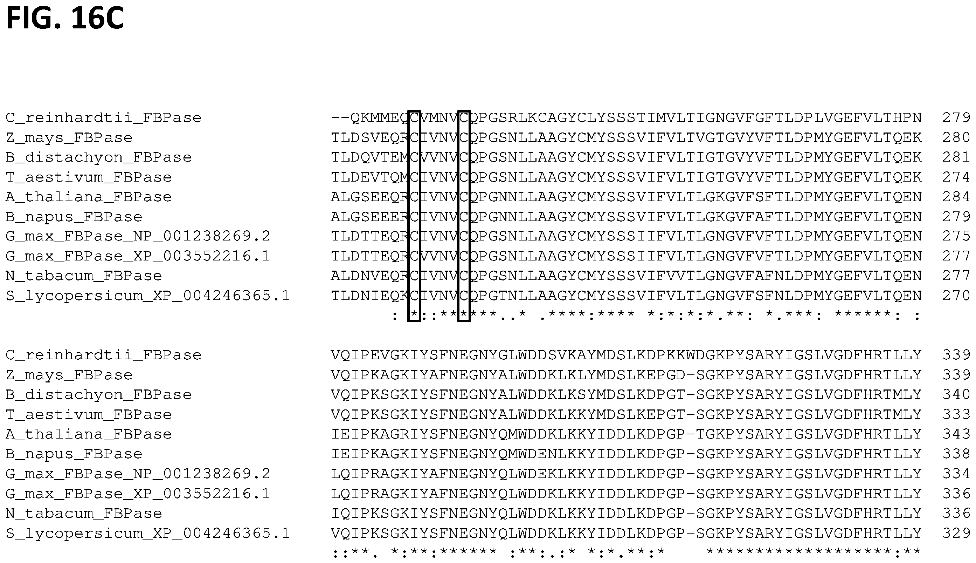

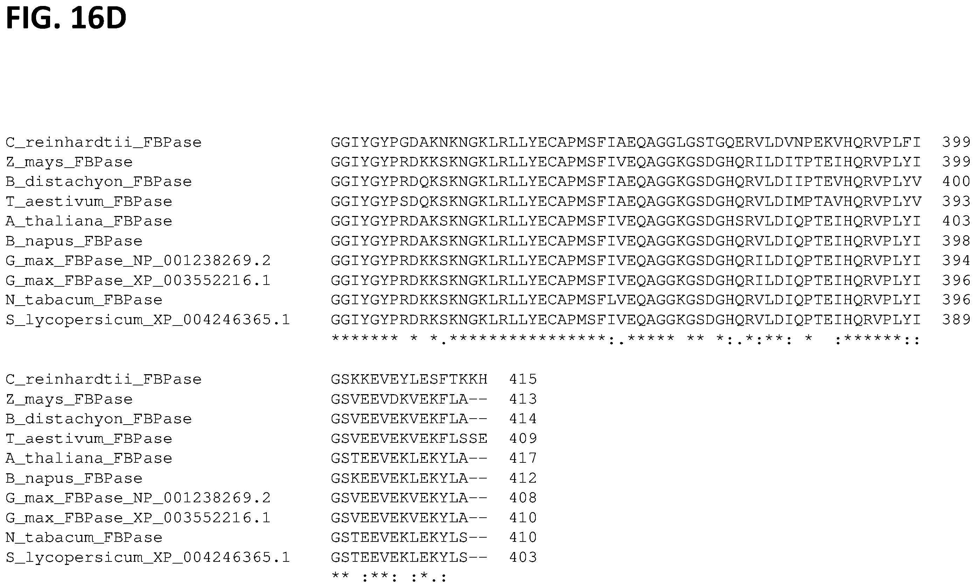

**FIGS. 16A-16D** show the alignment of FBPase polypeptide sequences from *Chlamydomonas reinhardtii* (SEQ ID NO: 37), *Zea mays* (SEQ ID NO: 35), *Brachypodium distachyon* (SEQ ID NO: 33), *Triticum aestivum* (SEQ ID NO: 36), *Arabidopsis thaliana* (SEQ ID NO: 27), *Brassica napus* (SEQ ID NO: 34), *Glycine max* (G_max_FBPase_NP_001238269.2 (SEQ ID NO: 28); G_max_FBPase_XP_003552216.1 (SEQ ID NO: 29)), *Nicotiana tabacum* (SEQ ID NO: 30), and *Solanum lycopersicum* (SEQ ID NO: 32). **FIG. 16A** shows the alignment of the N terminal portion of the FBPase polypeptide. **FIG. 16B** shows the alignment of the first part of the central portion of the FBPase polypeptide. **FIG. 16C** shows the alignment of the second part of the central portion of the FBPase polypeptide. **FIG. 16D** shows the alignment of the C terminal portion of the FBPase polypeptide. In **FIGS. 16B-16C****,** boxes indicate cysteine residues to be mutated for producing plants with non-TRx (redox) activated FBPase.

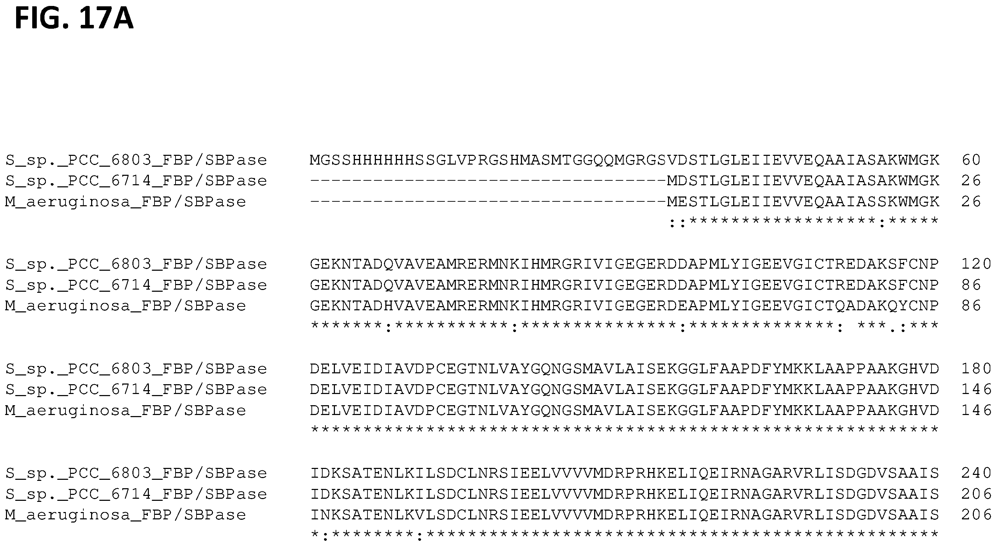

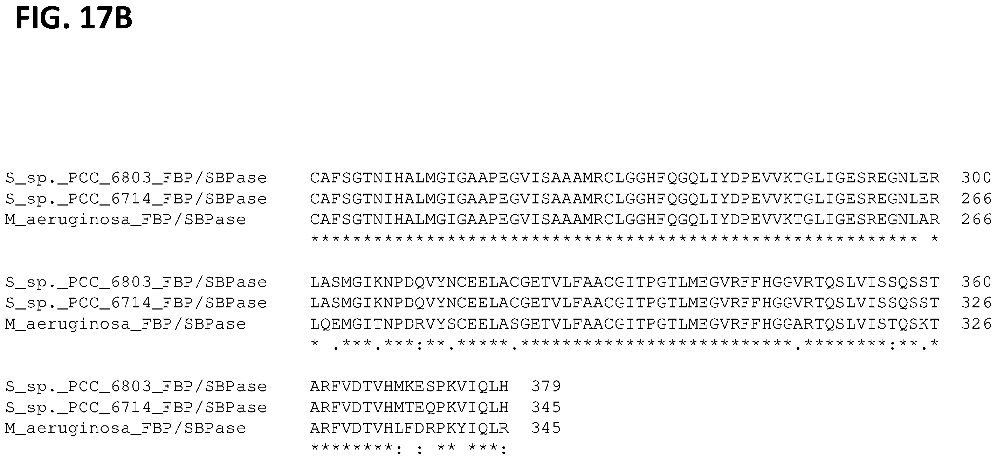

**FIGS. 17A-17B** show the alignment of FBP/SBPase polypeptide sequences from *Synechocystis* sp. PCC 6803 (SEQ ID NO: 38), *Synechocystis* sp. PCC 6714 (SEQ ID NO: 39) and *Microcystis aeruginosa* (SEQ ID NO: 40). **FIG. 17A** shows the alignment of the N terminal portion of the FBP/SBPase polypeptide. **FIG. 17B** shows the alignment of the C terminal portion of the FBP/SBPase polypeptide.

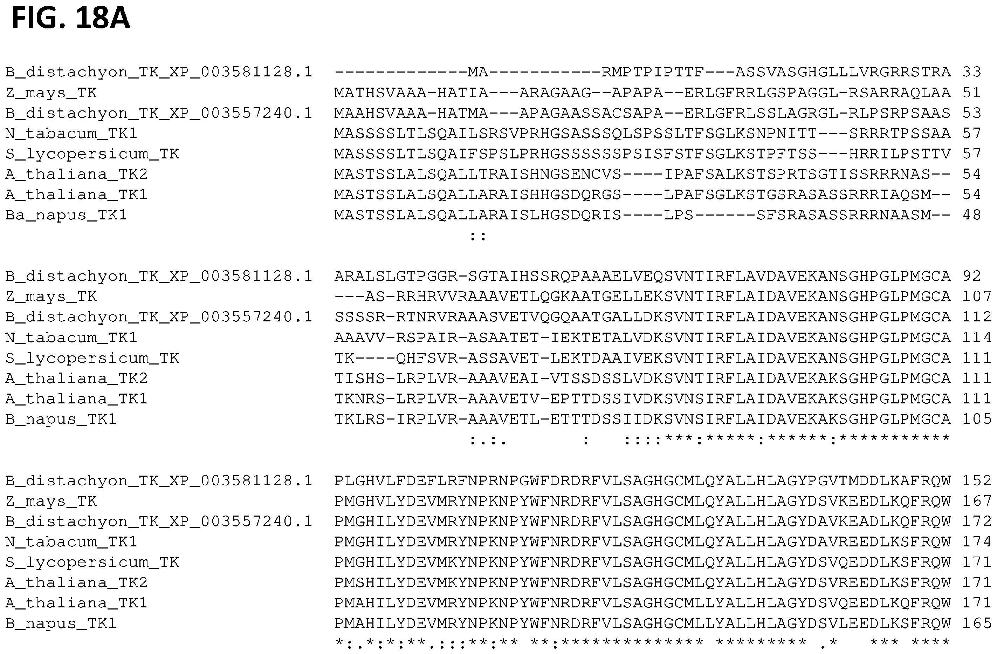

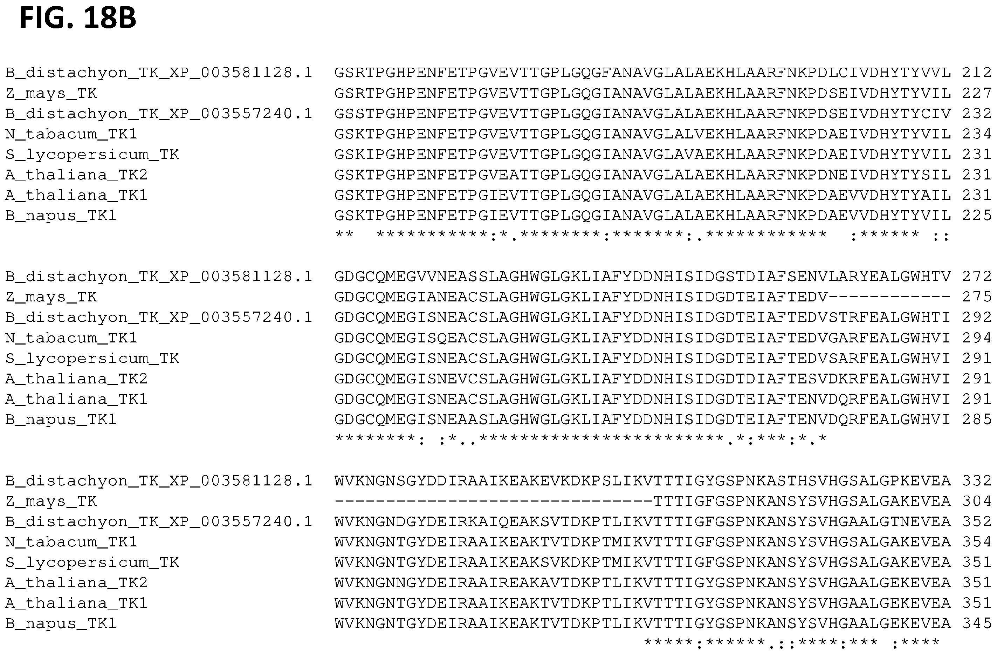

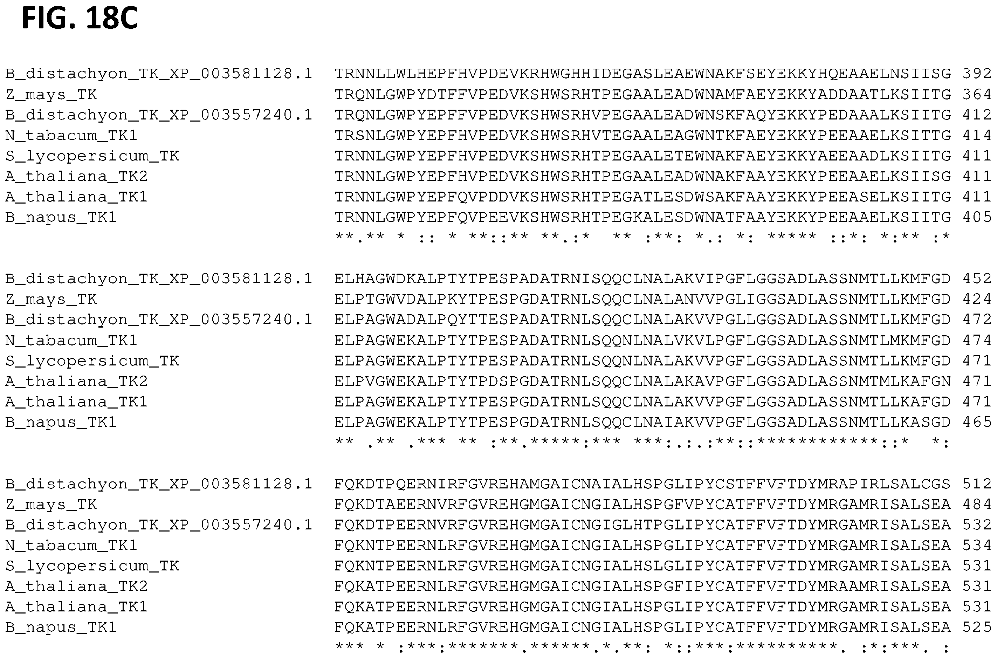

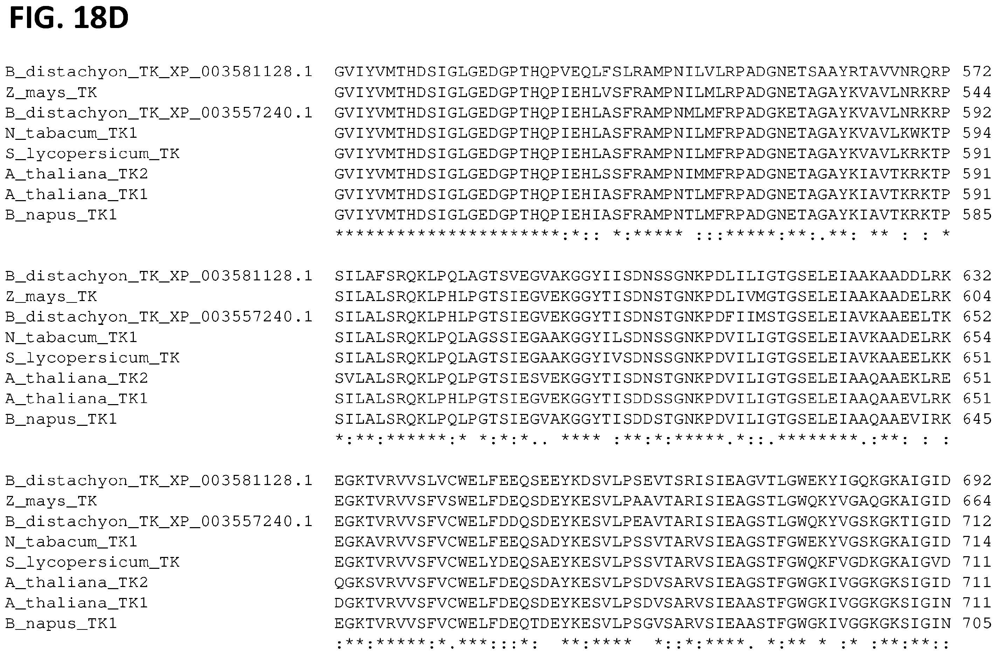

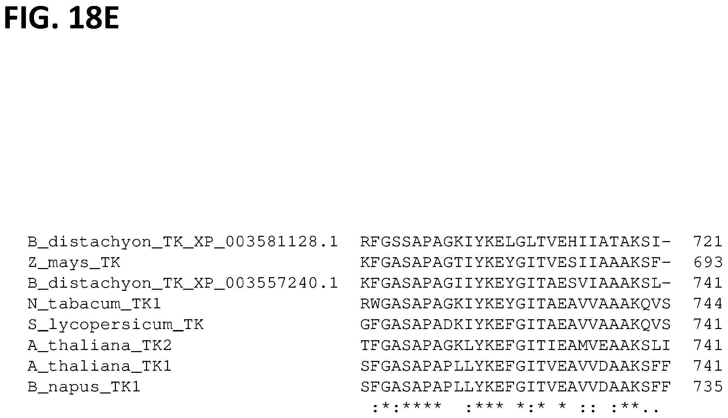

**FIGS. 18A-18E** show the alignment of transketolase polypeptide sequences from *Brachypodium distachyon* (B_distachyon_TK_XP_003557240.1 (SEQ ID NO: 46); B_distachyon_TK_XP_003581128.1 (SEQ ID NO: 47)), *Zea mays* (SEQ ID NO: 45), *Nicotiana tabacum* (SEQ ID NO: 43), *Solanum lycopersicum* (SEQ ID NO: 44), *Arabidopsis thaliana* (A_thaliana_TK1 (SEQ ID NO: 41); A_thaliana_TK2 (SEQ ID NO: 48)), and *Brassica napus* (SEQ ID NO: 42). **FIG. 18A** shows the alignment of the N terminal portion of the transketolase polypeptide. **FIG. 18B** shows the alignment of the first part of the central portion of the transketolase polypeptide. **FIG. 18C** shows the alignment of the second part of the central portion of the transketolase polypeptide. **FIG. 18D** shows the alignment of the third part of the central portion of the transketolase polypeptide. **FIG. 18E** shows the alignment of the C terminal portion of the transketolase polypeptide.

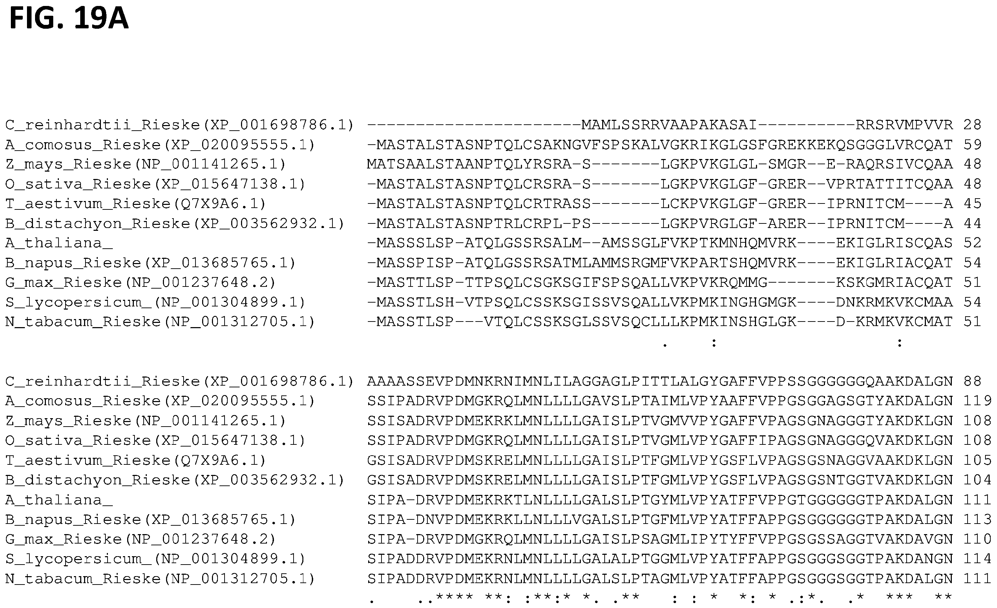

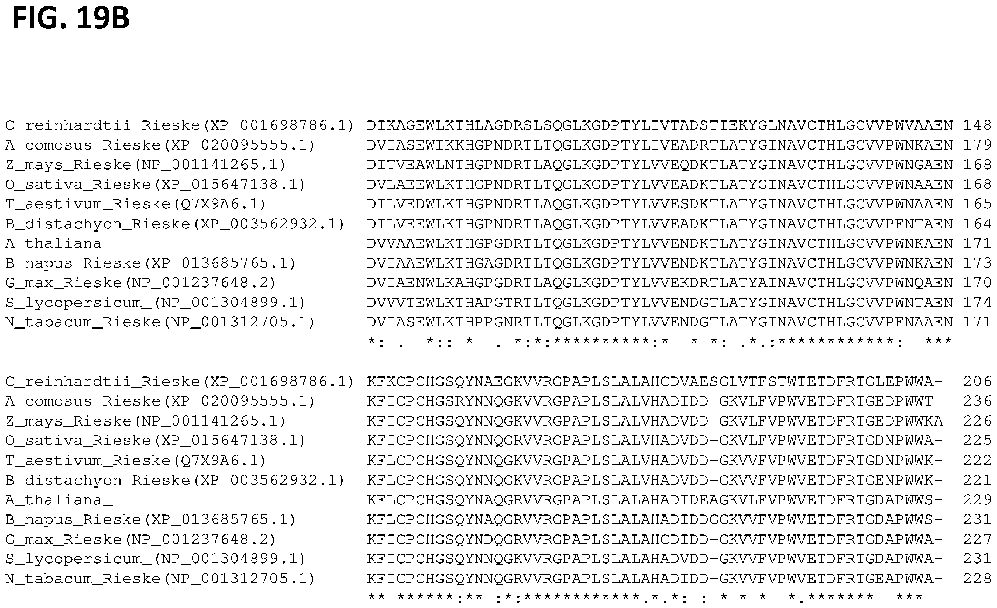

**FIGS. 19A-19B** show the alignment of Rieske FeS polypeptide sequences from *Chlamydomonas reinhardtii* (SEQ ID NO: 80), *Ananas comosus* (SEQ ID NO: 74), *Zea mays* (SEQ ID NO: 78), *Oryza sativa* (SEQ ID NO: 76), *Triticum aestivum* (SEQ ID NO: 75), *Brachypodium distachyon* (SEQ ID NO: 77), *Arabidopsis thaliana* (SEQ ID NO: 70), *Brassica napus* (SEQ ID NO: 71), *Glycine max* (SEQ ID NO: 79), *Solanum lycopersicum* (SEQ ID NO: 72), and *Nicotiana tabacum* (SEQ ID NO: 73). **FIG. 19A** shows the alignment of the N terminal portion of the Rieske FeS polypeptide. **FIG. 19B** shows the alignment of the C terminal portion of the transketolase polypeptide.

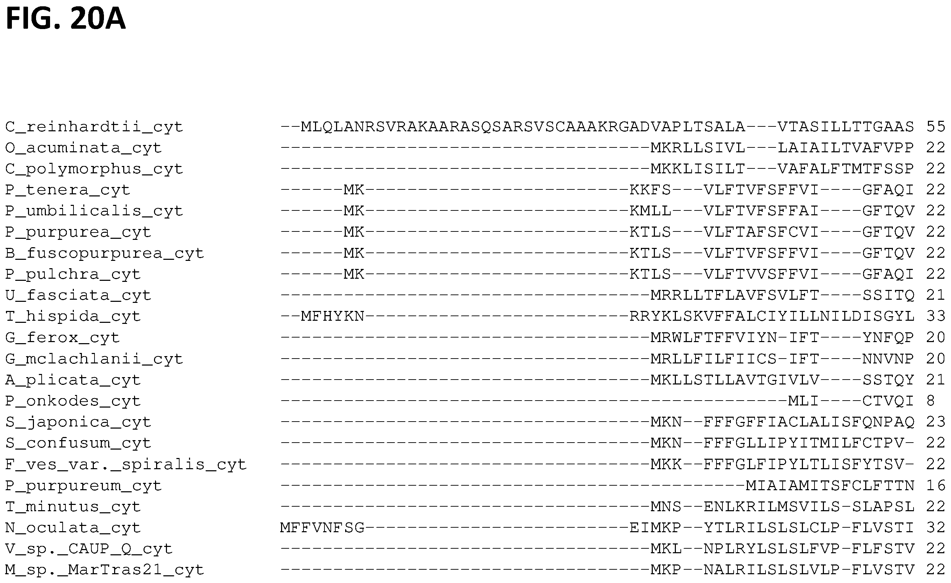

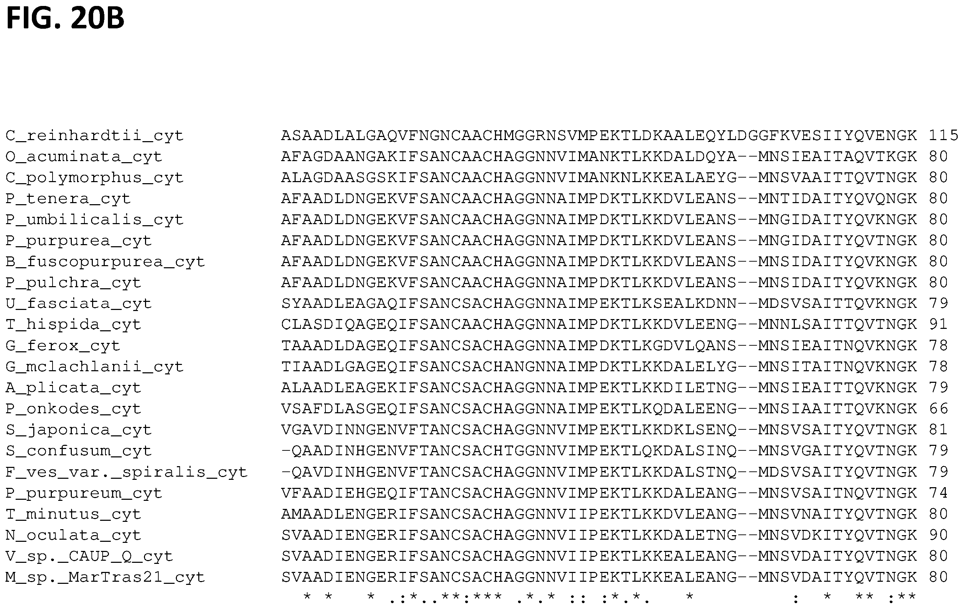

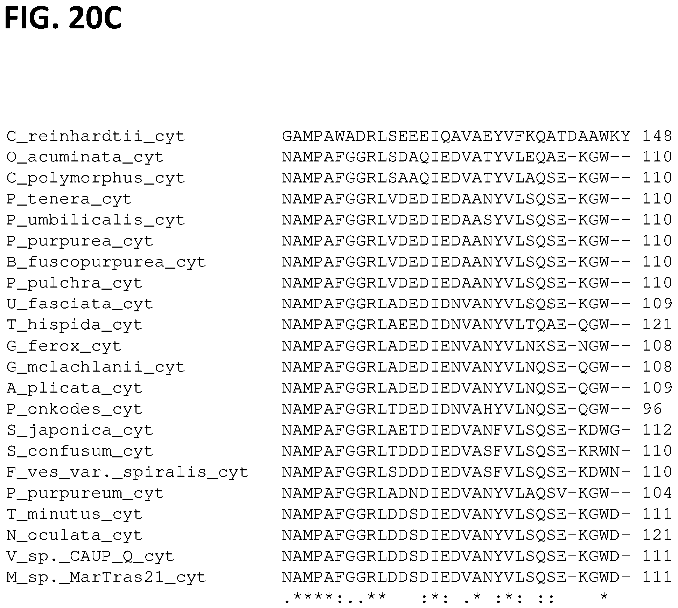

**FIGS. 20A-20C** show the alignment of cytochrome c₆ polypeptide sequences from *Chlamydomonas reinhardtii* (SEQ ID NO: 49), *Oscillatoria acuminata* (SEQ ID NO: *68), Chamaesiphon polymorphus* (SEQ ID NO: 69), *Pyropia tenera* (SEQ ID NO: 53), *Porphyra umbilicalis* (SEQ ID NO: 95), *Porphyra purpurea* (SEQ ID NO: 51), *Bangia fuscopurpurea* (SEQ ID NO: 50), *Pyropia pulchra* (SEQ ID NO: 52), *Ulva fasciata* (SEQ ID NO: 64), *Thorea hispida* (SEQ ID NO: 55), *Gracilaria ferox* (SEQ ID NO: 58), *Gracilariopsis mclachlanii* (SEQ ID NO: 62), *Ahnfeltia plicata* (SEQ ID NO: 56), *Porolithon onkodes* (SEQ ID NO: 57), *Saccharina japonica* (SEQ ID NO: 67), *Sargassum confusum* (SEQ ID NO: 59), *Fucus vesiculosus* var. *spiralis* (SEQ ID NO: 65), *Porphyridium purpureum* (SEQ ID NO: 54), *Trachydiscus minutus* (SEQ ID NO: 60), *Nannochloropsis oculata* (SEQ ID NO: 66), *Vischeria* sp. CAUP Q (SEQ ID NO: 61), and *Monodopsis* sp. MarTras21 (SEQ ID NO: 63). **FIG. 20A** shows the alignment of the N terminal portion of the cytochrome c₆ polypeptide. **FIG. 20B** shows the alignment of the central portion of the cytochrome c₆ polypeptide. **FIG. 20C** shows the alignment of the C terminal portion of the cytochrome c₆ polypeptide.

### DETAILED DESCRIPTION

The following description sets forth exemplary methods, parameters, and the like. It should be recognized, however, that such description is not intended as a limitation on the scope of the present disclosure but is instead provided as a description of exemplary embodiments.

### Genetically altered plants and seeds

An aspect of the disclosure includes a genetically altered plant, plant part, or plant cell, wherein the plant, part thereof or cell includes one or more RuBP regeneration enhancing genetic alterations that increase activity of a CB protein and one or more photosynthetic electron transport enhancing genetic alterations. An additional embodiment of this aspect includes the one or more photosynthetic electron transport enhancing genetic alterations being overexpression of one or more photosynthetic electron transport proteins. Yet another embodiment of this aspect includes the one or more photosynthetic electron transport proteins being selected from the group of a cytochrome c₆ protein, a Rieske FeS protein, or a cytochrome c₆ protein and a Rieske FeS protein. A further embodiment of this aspect includes the one or more photosynthetic electron transport proteins being a cytochrome c₆ protein. Still another embodiment of this aspect includes the cytochrome c₆ protein being an algal cytochrome c₆ protein. In an additional embodiment of this aspect, the algal cytochrome c₆ protein includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 95, or SEQ ID NO: 102. In a further embodiment of this aspect, the algal cytochrome c₆ protein includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 95. In yet another embodiment of this aspect, the algal cytochrome c₆ protein includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 102. An additional embodiment of this aspect includes the one or more photosynthetic electron transport proteins being a Rieske FeS protein. In a further embodiment of this aspect, the Rieske FeS protein includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, or SEQ ID NO: 101. In still another embodiment of this aspect, the Rieske FeS protein includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 101. An additional embodiment of this aspect includes the one or more photosynthetic electron transport proteins being a cytochrome c₆ protein and a Rieske FeS protein. In a further embodiment of this aspect, the cytochrome c₆ protein includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 95, or SEQ IF NO: 102; and the Rieske FeS protein includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, or SEQ ID NO: 101.

In yet another embodiment of this present aspect, which may be combined with any of the preceding embodiments that has cytochrome c₆, the cytochrome c₆ protein is localized to a thylakoid lumen of at least one chloroplast within a cell of the genetically altered plant. A further embodiment of this aspect includes the cytochrome c₆ protein including a transit peptide that localizes the cytochrome c₆ protein to the thylakoid lumen. An additional embodiment of this aspect includes the cytochrome c₆ transit peptide being selected from the group of a chlorophyll a/b binding protein 6 transit peptide, a light-harvesting complex I chlorophyll a/b binding protein 1 transit peptide, or a plastocyanin signal peptide. In still another embodiment of this present aspect, which may be combined with any of the preceding embodiments that has Rieske FeS, the Rieske FeS protein includes a transit peptide that localizes the Rieske FeS protein to the thylakoid membrane. Another embodiment of this present aspect includes the Rieske FeS transit peptide being a cytochrome b6f complex protein transit peptide. An additional embodiment of this aspect includes the Rieske FeS transit peptide being selected from the group of a cytochrome f transit peptide, a cytochrome b6 transit peptide, a PetD transit peptide, a PetG transit peptide, a PetL transit peptide, a PetN transit peptide, a PetM transit peptide, and a plastoquinone transit peptide. Still another embodiment of this aspect that can be combined with any of the preceding embodiments that has cytochrome c₆ further includes a cytochrome c₆ protein encoding nucleic acid sequence operably linked to a plant promoter. A further embodiment of this aspect includes the promoter being selected from a constitutive promoter, an inducible promoter, a tissue or cell type specific promoter, or an inducible, tissue or cell type specific promoter. Yet another embodiment of this aspect that can be combined with any of the preceding embodiments that has Rieske FeS further includes a Rieske FeS protein encoding nucleic acid sequence operably linked to a plant promoter. An additional embodiment of this aspect includes the promoter being selected from a constitutive promoter, an inducible promoter, a tissue or cell type specific promoter, or an inducible, tissue or cell type specific promoter.

In still another embodiment of this aspect that can be combined with any of the preceding embodiments, the one or more RuBP regeneration enhancing genetic alterations include overexpression of a CB protein. An additional embodiment of this aspect includes the CB protein being selected from the group of a sedoheptulose-1,7-bisphosphatase (SBPase), a fructose-1,6-bisphophate aldolase (FBPA), a chloroplastic fructose-1,6-bisphosphatase (FBPase), a bifunctional fructose-1,6-bisphosphatases/sedoheptulose-1,7-bisphosphatase (FBP/SBPase), or a transketolase (TK). A further embodiment of this aspect includes the CB protein being a SBPase. In yet another embodiment of this aspect, the SBPase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 96. In still another embodiment of this aspect, the SBPase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 96. A further embodiment of this aspect includes the SBPase being localized to a chloroplast stroma of at least one chloroplast within a cell of the genetically altered plant. In yet another embodiment of this aspect, the SBPase includes a transit peptide that localizes the SBPase to the chloroplast stroma. Still another embodiment of this aspect that can be combined with any of the preceding embodiments that has SBPase further includes a SBPase encoding nucleic acid sequence operably linked to a plant promoter. An additional embodiment of this aspect includes the promoter being selected from a constitutive promoter, an inducible promoter, a tissue or cell type specific promoter, or an inducible, tissue or cell type specific promoter. A further embodiment of this aspect includes the CB protein being a FBPA. In yet another embodiment of this aspect, the FBPA includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, or SEQ ID NO: 97. In still another embodiment of this aspect, the FBPA includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 97. A further embodiment of this aspect includes the FBPA being localized to a chloroplast stroma of at least one chloroplast within a cell of the genetically altered plant. In yet another embodiment of this aspect, the FBPA includes a transit peptide that localizes the FBPA to the chloroplast stroma. Still another embodiment of this aspect that can be combined with any of the preceding embodiments that has FBPA further includes a FBPA encoding nucleic acid sequence operably linked to a plant promoter. An additional embodiment of this aspect includes the promoter being selected from a constitutive promoter, an inducible promoter, a tissue or cell type specific promoter, or an inducible, tissue or cell type specific promoter. A further embodiment of this aspect includes the CB protein being a FBPase. In yet another embodiment of this aspect, the FBPase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, or SEQ ID NO: 98. In still another embodiment of this aspect, the FBPase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 98. A further embodiment of this aspect includes the FBPase being localized to a chloroplast stroma of at least one chloroplast within a cell of the genetically altered plant. In yet another embodiment of this aspect, the FBPase includes a transit peptide that localizes the FBPase to the chloroplast stroma. Still another embodiment of this aspect that can be combined with any of the preceding embodiments that has FBPase further includes a FBPase encoding nucleic acid sequence operably linked to a plant promoter. An additional embodiment of this aspect includes the promoter being selected from a constitutive promoter, an inducible promoter, a tissue or cell type specific promoter, or an inducible, tissue or cell type specific promoter. A further embodiment of this aspect includes the CB protein being a FBP/SBPase. An additional embodiment of this aspect includes the FBP/SBPase being a cyanobacterial FBP/SBPase. In yet another embodiment of this aspect, the cyanobacterial FBP/SBPase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 38, SEQ ID NO: 39, SEQ ID NO: 40, or SEQ ID NO: 99. In still another embodiment of this aspect, the cyanobacterial FBP/SBPase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 99. Still another embodiment of this aspect that can be combined with any of the preceding embodiments that has FBP/SBPase includes the FBP/SBPase being localized to a chloroplast stroma of at least one chloroplast within a cell of the genetically altered plant. In a further embodiment of this aspect, the FBP/SBPase includes a transit peptide that localizes the FBP/SBPase to the chloroplast stroma. Yet another embodiment of this aspect includes the transit peptide being a chloroplast stromal protein transit peptide in plant. An additional embodiment of this aspect include the transit peptide being selected from the group of a geraniol synthase transit peptide, a SBPase transit peptide, a FBPA transit peptide, a FBPase transit peptide, a transketolase transit peptide, a PGK transit peptide, a GAPDH transit peptide, an AGPase transit peptide, a RPI transit peptide, a RPE transit peptide, a PRK transit peptide, or a Rubisco transit peptide. Yet another embodiment of this aspect that can be combined with any of the preceding embodiments that has FBP/SBPase further includes a FBP/SBPase encoding nucleic acid sequence operably linked to a plant promoter. S An additional embodiment of this aspect includes the promoter being selected from a constitutive promoter, an inducible promoter, a tissue or cell type specific promoter, or an inducible, tissue or cell type specific promoter. A further embodiment of this aspect (not part of the claimed invention) includes the CB protein being a transketolase. In yet another embodiment of this aspect (not part of the claimed invention), the transketolase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, or SEQ ID NO: 100. In still another embodiment of this aspect (not part of the claimed invention), the transketolase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 100. A further embodiment of this aspect (not part of the claimed invention) includes the transketolase being localized to a chloroplast stroma of at least one chloroplast within a cell of the genetically altered plant. Yet another embodiment of this aspect (not part of the claimed invention) that can be combined with any of the preceding embodiments that has transketolase further includes a transketolase encoding nucleic acid sequence operably linked to a plant promoter. An additional embodiment of this aspect (not part of the claimed invention) includes the promoter being selected from a constitutive promoter, an inducible promoter, a tissue or cell type specific promoter, or an inducible, tissue or cell type specific promoter.

A further embodiment of this aspect that can be combined with any of the preceding embodiments that has a CB protein that is not FBP/SBPase includes the nucleic acid encoding the CB protein being endogenous. An additional embodiment of this aspect includes the promoter operably linked to the nucleic acid encoding the CB protein being genetically engineered to overexpress, inducibly express, express in a specific tissue or cell type, inducibly overexpress, or inducibly express in a specific tissue or cell type the CB protein. Still another embodiment of this aspect that can be combined with any of the preceding embodiments that has a CB protein includes the nucleic acid encoding the CB protein being heterologous.

In yet another embodiment of this aspect, which may be combined with any of the preceding embodiments, the plant has increased biomass as compared to an unaltered wild type (WT) plant. An additional embodiment of this aspect includes the plant having improved water use efficiency as compared to an unaltered WT plant when grown in conditions with light intensities above 1000 µmol m⁻² s⁻¹. A further embodiment of this aspect includes the plant being selected from the group of cowpea (*e.g*., black-eyed pea, catjang, yardlong bean, *Vigna unguiculata*), soybean (*e.g., Glycine max, Glycine soja*), cassava *(e.g.,* manioc, yucca, *Manihot esculenta*), rice *(e.g.,* indica rice, japonica rice, aromatic rice, glutinous rice, *Oryza sativa, Oryza glaberrima*), wheat *(e.g.,* common wheat, spelt, durum, einkorn, emmer, kamut, *Triticum aestivum, Triticum spelta, Triticum durum, Triticum urartu, Triticum monococcum, Triticum turanicum, Triticum* spp.), barley (*e.g., Hordeum vulgare),* tomato (*e.g., Solanum lycopersicum*), potato *(e.g.,* russet potatoes, yellow potatoes, red potatoes, *Solanum tuberosum*), tobacco (*e.g., Nicotiana tabacum*), canola (*e.g., Brassica rapa, Brassica napus, Brassica juncea*), or other C3 crop plants. Still another embodiment of this aspect includes the plant being selected from the group of cowpea (*e.g*., black-eyed pea, catjang, yardlong bean, *Vigna unguiculata*), soybean (*e.g., Glycine max, Glycine soja*), cassava *(e.g.,* manioc, yucca, *Manihot esculenta*), rice *(e.g.,* indica rice, japonica rice, aromatic rice, glutinous rice, *Oryza sativa, Oryza glaberrima*), wheat *(e.g.,* common wheat, spelt, durum, einkorn, emmer, kamut, *Triticum aestivum, Triticum spelta, Triticum durum, Triticum urartu, Triticum monococcum, Triticum turanicum, Triticum* spp.), barley (*e.g., Hordeum vulgare*), and tobacco (*e.g., Nicotiana tabacum*).

Yet another embodiment of this aspect that can be combined with any of the preceding embodiments with respect to plant part includes the plant part being a leaf, a stem, a root, a tuber, a flower, a seed, a kernel, a grain, a fruit, a cell, or a portion thereof and the genetically altered plant part including the one or more genetic alterations. A further embodiment of this aspect includes the plant part being a fruit, a tuber, a kernel, or a grain. Still another embodiment of this aspect that can be combined with any of the preceding embodiments with respect to pollen grain or ovules includes a genetically altered pollen grain or a genetically altered ovule of the plant of any one of the preceding embodiments, wherein the genetically altered pollen grain or the genetically altered ovule includes the one or more genetic alterations. A further embodiment of this aspect that can be combined with any of the preceding embodiments includes a genetically altered protoplast produced from the genetically altered plant of any of the preceding embodiments, wherein the genetically altered protoplast includes the one or more genetic alterations. An additional embodiment of this aspect that can be combined with any of the preceding embodiments includes a genetically altered tissue culture produced from protoplasts or cells from the genetically altered plant of any one of the preceding embodiments, wherein the cells or protoplasts are produced from a plant part selected from the group of leaf, leaf mesophyll cell, anther, pistil, stem, petiole, root, root tip, tuber, fruit, seed, kernel, grain, flower, cotyledon, hypocotyl, embryo, or meristematic cell, wherein the genetically altered tissue culture includes the one or more genetic alterations. An additional embodiment of this aspect includes a genetically altered plant regenerated from the genetically altered tissue culture that includes the one or more genetic alterations. Yet another embodiment of this aspect that can be combined with any of the preceding embodiments includes a genetically altered plant seed produced from the genetically altered plant of any one of the preceding embodiments.

### Methods of producing and cultivating genetically altered plants

An additional aspect of the disclosure includes methods of producing the genetically altered plant of any of the preceding embodiments including (a) introducing the one or more RuBP regeneration enhancing genetic alterations that increase activity of a CB protein, the one or more photosynthetic electron transport enhancing genetic alterations, or both the one or more RuBP regeneration enhancing genetic alterations that increase activity of a CB protein and the one or more photosynthetic electron transport enhancing genetic alterations into a plant cell, tissue, or other explant; (b) regenerating the plant cell, tissue, or other explant into a genetically altered plantlet; and (c) growing the genetically altered plantlet into a genetically altered plant with the one or more RuBP regeneration enhancing genetic alterations that increase activity of a CB protein, the one or more photosynthetic electron transport enhancing genetic alterations, or both the one or more RuBP regeneration enhancing genetic alterations that increase activity of a CB protein and the one or more photosynthetic electron transport enhancing genetic alterations. An additional embodiment of this aspect further includes identifying successful introduction of the one or more genetic alterations by screening or selecting the plant cell, tissue, or other explant prior to step (b); screening or selecting plantlets between step (b) and (c); or screening or selecting plants after step (c). In yet another embodiment of this aspect, which may be combined with any of the preceding embodiments, transformation is done using a transformation method selected from the group of particle bombardment *(i.e.,* biolistics, gene gun), Agrobacterium-mediated transformation, Rhizobium-mediated transformation, or protoplast transfection or transformation.

Still another embodiment of this aspect that can be combined with any of the preceding embodiments includes genetic alterations being introduced with a vector. In an additional embodiment of this aspect, the vector includes a promoter operably linked to a nucleotide encoding one or more photosynthetic electron transport proteins, a nucleotide encoding one or more CB proteins, or a nucleotide encoding one or more photosynthetic electron transport protein and one or more CB proteins. Yet another embodiment of this aspect includes the promoter being selected from the group of a constitutive promoter, an inducible promoter, a tissue or cell type specific promoter, or an inducible, tissue or cell type specific promoter. In a further embodiment of this aspect, which may be combined with any of the preceding embodiments, the photosynthetic electron transport protein is selected from the group of a cytochrome c₆ protein, a Rieske FeS protein, or a cytochrome c₆ protein and a Rieske FeS protein. In yet another embodiment of this aspect, the cytochrome c₆ protein includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 95, or SEQ ID NO: 102. **FIGS. 20A-20C** show an alignment of exemplary cytochrome c₆ protein polypeptide sequences. In still another embodiment of this aspect, the Rieske FeS protein includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, or SEQ ID NO: 101. **FIGS. 19A-19B** show an alignment of exemplary Rieske FeS polypeptide sequences. In a further embodiment of this aspect, the vector includes one or more gene editing components that target a nuclear genome sequence operably linked to the nucleic acid encoding the CB protein. In yet another embodiment of this present aspect, the one or more gene editing components are selected from the group of a ribonucleoprotein complex that targets the nuclear genome sequence; a vector including a TALEN protein encoding sequence, wherein the TALEN protein targets the nuclear genome sequence; a vector including a ZFN protein encoding sequence, wherein the ZFN protein targets the nuclear genome sequence; an oligonucleotide donor (ODN), wherein the ODN targets the nuclear genome sequence; or a vector including a CRISPR/Cas enzyme encoding sequence and a targeting sequence, wherein the targeting sequence targets the nuclear genome sequence.

In a further embodiment of this aspect that can be combined with any of the preceding embodiments that has a vector including a nucleotide encoding one or more CB proteins, the CB protein is selected from the group of a sedoheptulose-1,7-bisphosphatase (SBPase), a fructose-1,6-bisphophate aldolase (FBPA), a chloroplastic fructose-1,6-bisphosphatase (FBPase), a bifunctional fructose-1,6-bisphosphatases/sedoheptulose-1,7-bisphosphatase (FBP/SBPase), or a transketolase (TK). In an additional embodiment of this aspect, the CB protein is a SBPase, and the SBPase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 96. **FIGS. 14A-****14D** show an alignment of exemplary SBPase polypeptide sequences. In another embodiment of this aspect, the CB protein is a FBPA, and the FBPA includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, or SEQ ID NO: 97. **FIGS. 15A-15D** show an alignment of exemplary FBPA polypeptide sequences. In still another embodiment of this aspect, the CB protein is a FBPase, and the FBPase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 98. **FIGS. 16A-16D** show an alignment of exemplary FBPase polypeptide sequences. In a further embodiment of this aspect, the CB protein is a FBP/SBPase, and the FBP/SBPase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 38, SEQ ID NO: 39, SEQ ID NO: 40, or SEQ ID NO: 99. **FIGS. 17A-17B** show an alignment of exemplary FBP/SBPase polypeptide sequences. In yet another embodiment of this aspect, the CB protein is a transketolase, and the transketolase includes an amino acid sequence with at least 70% sequence identity, at least 71% sequence identity, at least 72% sequence identity, at least 73% sequence identity, at least 74% sequence identity, at least 75% sequence identity, at least 76% sequence identity, at least 77% sequence identity, at least 78% sequence identity, at least 79% sequence identity, at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, or SEQ ID NO: 100. **FIGS. 18A-18E** show an alignment of exemplary transketolase sequences.

A further aspect of the disclosure includes methods of cultivating the genetically altered plant of any of the preceding embodiments that has a genetically altered plant, including the steps of: planting a genetically altered seedling, a genetically altered plantlet, a genetically altered cutting, a genetically altered tuber, a genetically altered root, or a genetically altered seed in soil to produce the genetically altered plant or grafting the genetically altered seedling, the genetically altered plantlet, or the genetically altered cutting to a root stock or a second plant grown in soil to produce the genetically altered plant; cultivating the plant to produce harvestable seed, harvestable leaves, harvestable roots, harvestable cuttings, harvestable wood, harvestable fruit, harvestable kernels, harvestable tubers, and/or harvestable grain; and harvesting the harvestable seed, harvestable leaves, harvestable roots, harvestable cuttings, harvestable wood, harvestable fruit, harvestable kernels, harvestable tubers, and/or harvestable grain.

### Molecular biological methods to produce genetically altered plants, plant parts, and plant cells

One aspect of the present invention provides genetically altered plants, plant parts, or plant cells with modified expression of one or more CB proteins and modified expression of one or more photosynthetic electron transport proteins as compared to the unaltered plants, plant parts, or plant cells. For example, the present disclosure provides genetically altered plants, plant parts, or plant cells with the addition of one or more CB proteins and the addition of one or more photosynthetic electron transport proteins operably linked to a constitutive promoter, an inducible promoter, a tissue or cell type specific promoter, or an inducible, tissue or cell type specific promoter, where the nucleic acid encoding the one or more CB proteins and/or the one or more photosynthetic electron transport proteins has been introduced by genetic alteration of the plant, the promoter has been introduced by genetic alteration of the plant, or both the nucleic acid encoding the one or more CB proteins and/or the one or more photosynthetic electron transport proteins and the promoter have been introduced by genetic alteration of the plant.

Transformation and generation of genetically altered monocotyledonous and dicotyledonous plant cells is well known in the art. See, *e.g*., Weising, et al., Ann. Rev. Genet. 22:421-477 (1988); U.S. Patent 5,679,558; Agrobacterium Protocols, ed: Gartland, Humana Press Inc. (1995); Wang, et al. Acta Hort. 461:401-408 (1998), and Broothaerts, et al. Nature 433:629-633 (2005). The choice of method varies with the type of plant to be transformed, the particular application and/or the desired result. The appropriate transformation technique is readily chosen by the skilled practitioner.

Any methodology known in the art to delete, insert or otherwise modify the cellular DNA (*e.g*., genomic DNA and organelle DNA) can be used in practicing the inventions disclosed herein. As an example, the CRISPR/Cas-9 system and related systems *(e.g.,* TALEN, ZFN, ODN, *etc.)* may be used to insert a heterologous gene to a targeted site in the genomic DNA or substantially edit an endogenous gene to express the heterologous gene or to modify the promoter to increase or otherwise alter expression of an endogenous gene through, for example, removal of repressor binding sites or introduction of enhancer binding sites. For example, a disarmed Ti plasmid, containing a genetic construct for deletion or insertion of a target gene, in *Agrobacterium tumefaciens* can be used to transform a plant cell, and thereafter, a transformed plant can be regenerated from the transformed plant cell using procedures described in the art, for example, in EP 0116718, EP 0270822, PCT publication WO 84/02913 and published European Patent application ("EP") 0242246. Ti-plasmid vectors each contain the gene between the border sequences, or at least located to the left of the right border sequence, of the T-DNA of the Ti-plasmid. Of course, other types of vectors can be used to transform the plant cell, using procedures such as direct gene transfer (as described, for example in EP 0233247), pollen mediated transformation (as described, for example in EP 0270356, PCT publication WO 85/01856, and US Patent 4,684,611), plant RNA virus-mediated transformation (as described, for example in EP 0 067 553 and US Patent 4,407,956), liposome-mediated transformation (as described, for example in US Patent 4,536,475), and other methods such as the methods for transforming certain lines of corn *(e.g.,* US patent 6,140,553; Fromm et al., Bio/Technology (1990) 8, 833-839); Gordon-Kamm et al., The Plant Cell, (1990) 2, 603-618), rice (Shimamoto et al., Nature, (1989) 338, 274-276; Datta et al., Bio/Technology, (1990) 8, 736-740), and the method for transforming monocots generally (PCT publication WO 92/09696). For cotton transformation, the method described in PCT patent publication WO 00/71733 can be used. For soybean transformation, reference is made to methods known in the art, *e.g.,* Hinchee et al. (Bio/Technology, (1988) 6, 915) and Christou et al. (Trends Biotech, (1990) 8, 145) or the method of WO 00/42207.

Genetically altered plants of the present invention can be used in a conventional plant breeding scheme to produce more genetically altered plants with the same characteristics, or to introduce the genetic alteration(s) in other varieties of the same or related plant species. Seeds, which are obtained from the altered plants, preferably contain the genetic alteration(s) as a stable insert in chromosomal DNA or as modifications to an endogenous gene or promoter. Plants including the genetic alteration(s) in accordance with the invention include plants including, or derived from, root stocks of plants including the genetic alteration(s) of the invention, e.g., fruit trees or ornamental plants. Hence, any nontransgenic grafted plant parts inserted on a transformed plant or plant part are included in the invention.

Genetic alterations of the disclosure, including in an expression vector or expression cassette, which result in the expression of an introduced gene or altered expression of an endogenous gene will typically utilize a plant-expressible promoter. A 'plant-expressible promoter' as used herein refers to a promoter that ensures expression of the genetic alteration(s) of the invention in a plant cell. Examples of constitutive promoters that are often used in plant cells are the cauliflower mosaic (CaMV) 35S promoter (KAY et al. Science, 236, 4805, 1987), the minimal CaMV 35S promoter (Benfey & Chua, Science, (1990) 250, 959-966), various other derivatives of the CaMV 35S promoter, the figwort mosaic virus (FMV) promoter (Richins, et al., Nucleic Acids Res. (1987) 15:8451-8466)the maize ubiquitin promoter (CHRISTENSEN & QUAIL, Transgenic Res, 5, 213-8, 1996), the trefoil promoter (Ljubql, MAEKAWA et al. Mol Plant Microbe Interact. 21, 375-82, 2008), the vein mosaic cassava virus promoter (International Application WO 97/48819), and the Arabidopsis UBQ10 promoter, Norris et al. Plant Mol. Biol. 21, 895-906, 1993).

Additional examples of promoters directing constitutive expression in plants are known in the art and include: the strong constitutive 35S promoters (the "35S promoters") of the cauliflower mosaic virus (CaMV), *e.g.,* of isolates CM 1841 (Gardner et al., Nucleic Acids Res, (1981) 9, 2871-2887), CabbB S (Franck et al., Cell (1980) 21, 285-294) and CabbB JI (Hull and Howell, Virology, (1987) 86, 482-493); promoters from the ubiquitin family *(e.g.,* the maize ubiquitin promoter of Christensen et al., Plant Mol Biol, (1992) 18, 675-689), the gos2 promoter (de Pater et al., The Plant J (1992) 2, 834-844), the emu promoter (Last et al., Theor Appl Genet, (1990) 81, 581-588), actin promoters such as the promoter described by An et al. (The Plant J, (1996) 10, 107), the rice actin promoter described by Zhang et al. (The Plant Cell, (1991) 3, 1155-1165); promoters of the figwort mosaic virus (FMV) (Richins, et al., Nucleic Acids Res. (1987) 15:8451-8466), promoters of the Cassava vein mosaic virus (WO 97/48819; Verdaguer et al., Plant Mol Biol, (1998) 37, 1055-1067) , the pPLEX series of promoters from Subterranean Clover Stunt Virus (WO 96/06932, particularly the S4 or S7 promoter), an alcohol dehydrogenase promoter, e.g., pAdh1S (GenBank accession numbers X04049, X00581), and the TR1' promoter and the TR2' promoter (the "TR1' promoter" and "TR2' promoter", respectively) which drive the expression of the 1' and 2' genes, respectively, of the T DNA (Velten et al., EMBO J, (1984) 3, 2723-2730).

Alternatively, a plant-expressible promoter can be a tissue-specific promoter, *i.e.,* a promoter directing a higher level of expression in some cells or tissues of the plant, *e.g.,* in green tissues (such as the promoter of the chlorophyll a/b binding protein (Cab)). The plant Cab promoter (Mitra et al., Planta, (2009) 5: 1015-1022) has been described to be a strong bidirectional promoter for expression in green tissue (*e.g*., leaves and stems) and is useful in one embodiment of the current invention. These plant-expressible promoters can be combined with enhancer elements, they can be combined with minimal promoter elements, or can include repeated elements to ensure the expression profile desired.

Additional non-limiting examples of tissue-specific promoters include the maize allothioneine promoter (DE FRAMOND et al, FEBS 290, 103-106, 1991; Application EP 452269), the chitinase promoter (SAMAC et al. Plant Physiol 93, 907-914, 1990), the maize ZRP2 promoter (U.S. Pat. No. 5,633,363), the tomato LeExtl promoter (Bucher et al. Plant Physiol. 128, 911-923, 2002), the glutamine synthetase soybean root promoter (HIREL et al. Plant Mol. Biol. 20, 207-218, 1992), the RCC3 promoter (PCT Application WO 2009/016104), the rice antiquitine promoter (PCT Application WO 2007/076115), the LRR receptor kinase promoter (PCT application WO 02/46439), and the Arabidopsis pCO2 promoter (HEIDSTRA et al, Genes Dev. 18, 1964-1969, 2004). Further non-limiting examples of tissue-specific promoters include the RbcS2B promoter, RbcS1B promoter, RbcS3B promoter, LHB1B1 promoter, LHB1B2 promoter, cab 1 promoter, and other promoters described in Engler et al., ACS Synthetic Biology, DOI: 10.1021/sb4001504, 2014. These plant promoters can be combined with enhancer elements, they can be combined with minimal promoter elements, or can include repeated elements to ensure the expression profile desired.

In some embodiments, further genetic alterations to increase expression in plant cells can be utilized. For example, an intron at the 5' end or 3' end of an introduced gene, or in the coding sequence of the introduced gene, *e.g.,* the hsp70 intron. Other such genetic elements can include, but are not limited to, promoter enhancer elements, duplicated or triplicated promoter regions, 5' leader sequences different from another transgene or different from an endogenous (plant host) gene leader sequence, 3' trailer sequences different from another transgene used in the same plant or different from an endogenous (plant host) trailer sequence.

An introduced gene of the present disclosure can be inserted in host cell DNA so that the inserted gene part is upstream *(i.e.,* 5') of suitable 3' end transcription regulation signals *(i.e.,* transcript formation and polyadenylation signals). This is preferably accomplished by inserting the gene in the plant cell genome (nuclear or chloroplast). Preferred polyadenylation and transcript formation signals include those of the nopaline synthase gene (Depicker et al., J. Molec Appl Gen, (1982) 1, 561-573), the octopine synthase gene (Gielen et al., EMBO J, (1984) 3:835-845), the SCSV or the Malic enzyme terminators (Schunmann et al., Plant Funct Biol, (2003) 30:453-460), and the T DNA gene 7 (Velten and Schell, Nucleic Acids Res, (1985) 13, 6981-6998), which act as 3' untranslated DNA sequences in transformed plant cells. In some embodiments, one or more of the introduced genes are stably integrated into the nuclear genome. Stable integration is present when the nucleic acid sequence remains integrated into the nuclear genome and continues to be expressed *(i.e.,* detectable mRNA transcript or protein is produced) throughout subsequent plant generations. Stable integration into the nuclear genome can be accomplished by any known method in the art *(e.g.,* microparticle bombardment, *Agrobacterium*-mediated transformation, CRISPR/Cas9, electroporation of protoplasts, microinjection, *etc.).*

The term recombinant or modified nucleic acids refers to polynucleotides which are made by the combination of two otherwise separated segments of sequence accomplished by the artificial manipulation of isolated segments of polynucleotides by genetic engineering techniques or by chemical synthesis. In so doing one may join together polynucleotide segments of desired functions to generate a desired combination of functions.

As used herein, the term *"overexpression"* refers to increased expression *(e.g.,* of mRNA, polypeptides, *etc.)* relative to expression in a wild type organism *(e.g.,* plant) as a result of genetic modification and can refer to expression of heterologous genes at a sufficient level to achieve the desired result such as increased yield. In some embodiments, the increase in expression is a slight increase of about 10% more than expression in wild type. In some embodiments, the increase in expression is an increase of 50% or more *(e.g.,* 60%, 70%, 80%, 100%, *etc.)* relative to expression in wild type. In some embodiments, an endogenous gene is upregulated. In some embodiments, an exogenous gene is upregulated by virtue of being expressed. Upregulation of a gene in plants can be achieved through any known method in the art, including but not limited to, the use of constitutive promoters with inducible response elements added, inducible promoters, high expression promoters (*e.g*., PsaD promoter) with inducible response elements added, enhancers, transcriptional and/or translational regulatory sequences, codon optimization, modified transcription factors, and/or mutant or modified genes that control expression of the gene to be upregulated in response to a stimulus such as cytokinin signaling.

Where a recombinant nucleic acid is intended for expression, cloning, or replication of a particular sequence, DNA constructs prepared for introduction into a host cell will typically include a replication system (*e.g*., vector) recognized by the host, including the intended DNA fragment encoding a desired polypeptide, and can also include transcription and translational initiation regulatory sequences operably linked to the polypeptide-encoding segment. Additionally, such constructs can include cellular localization signals (*e.g*., plasma membrane localization signals). In preferred embodiments, such DNA constructs are introduced into a host cell's genomic DNA, chloroplast DNA or mitochondrial DNA.

In some embodiments, a non-integrated expression system can be used to induce expression of one or more introduced genes. Expression systems (expression vectors) can include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Signal peptides can also be included where appropriate from secreted polypeptides of the same or related species, which allow the protein to cross and/or lodge in cell membranes, cell wall, or be secreted from the cell.

Selectable markers useful in practicing the methodologies of the invention disclosed herein can be positive selectable markers. Typically, positive selection refers to the case in which a genetically altered cell can survive in the presence of a toxic substance only if the recombinant polynucleotide of interest is present within the cell. Negative selectable markers and screenable markers are also well known in the art and are contemplated by the present invention. One of skill in the art will recognize that any relevant markers available can be utilized in practicing the inventions disclosed herein.

Screening and molecular analysis of recombinant strains of the present invention can be performed utilizing nucleic acid hybridization techniques. Hybridization procedures are useful for identifying polynucleotides, such as those modified using the techniques described herein, with sufficient homology to the subject regulatory sequences to be useful as taught herein. The particular hybridization techniques are not essential to the subject invention. As improvements are made in hybridization techniques, they can be readily applied by one of skill in the art. Hybridization probes can be labeled with any appropriate label known to those of skill in the art. Hybridization conditions and washing conditions, for example temperature and salt concentration, can be altered to change the stringency of the detection threshold. See, e.g., Sambrook et al. (1989) vide infra or Ausubel et al. (1995) Current Protocols in Molecular Biology, John Wiley & Sons, NY, N.Y., for further guidance on hybridization conditions.

Additionally, screening and molecular analysis of genetically altered strains, as well as creation of desired isolated nucleic acids can be performed using Polymerase Chain Reaction (PCR). PCR is a repetitive, enzymatic, primed synthesis of a nucleic acid sequence. This procedure is well known and commonly used by those skilled in this art (see Mullis, U.S. Pat. Nos. 4,683,195, 4,683,202, and 4,800,159; Saiki et al. (1985) Science 230:1350-1354). PCR is based on the enzymatic amplification of a DNA fragment of interest that is flanked by two oligonucleotide primers that hybridize to opposite strands of the target sequence. The primers are oriented with the 3' ends pointing towards each other. Repeated cycles of heat denaturation of the template, annealing of the primers to their complementary sequences, and extension of the annealed primers with a DNA polymerase result in the amplification of the segment defined by the 5' ends of the PCR primers. Because the extension product of each primer can serve as a template for the other primer, each cycle essentially doubles the amount of DNA template produced in the previous cycle. This results in the exponential accumulation of the specific target fragment, up to several million-fold in a few hours. By using a thermostable DNA polymerase such as the Taq polymerase, which is isolated from the thermophilic bacterium *Thermus aquaticus,* the amplification process can be completely automated. Other enzymes which can be used are known to those skilled in the art.

Nucleic acids and proteins of the present invention can also encompass homologues of the specifically disclosed sequences. Homology (*e.g*., sequence identity) can be 50%-100%. In some instances, such homology is greater than 80%, greater than 85%, greater than 90%, or greater than 95%. The degree of homology or identity needed for any intended use of the sequence(s) is readily identified by one of skill in the art. As used herein percent sequence identity of two nucleic acids is determined using an algorithm known in the art, such as that disclosed by Karlin and Altschul (1990) Proc. Natl. Acad. Sci. USA 87:2264-2268, modified as in Karlin and Altschul (1993) Proc. Natl. Acad. Sci. USA 90:5873-5877. Such an algorithm is incorporated into the BLASTN, BLASTP, and BLASTX, programs of Altschul et al. (1990) J. Mol. Biol. 215:402-410. BLAST nucleotide searches are performed with the BLASTN program, score=100, wordlength=12, to obtain nucleotide sequences with the desired percent sequence identity. To obtain gapped alignments for comparison purposes, Gapped BLAST is used as described in Altschul et al. (1997) Nucl. Acids. Res. 25:3389-3402. When utilizing BLAST and Gapped BLAST programs, the default parameters of the respective programs (BLASTN and BLASTX) are used. See www.ncbi.nih.gov. One of skill in the art can readily determine in a sequence of interest where a position corresponding to amino acid or nucleic acid in a reference sequence occurs by aligning the sequence of interest with the reference sequence using the suitable BLAST program with the default settings (e.g., for BLASTP: Gap opening penalty: 11, Gap extension penalty: 1, Expectation value: 10, Word size: 3, Max scores: 25, Max alignments: 15, and Matrix: blosum62; and for BLASTN: Gap opening penalty: 5, Gap extension penalty:2, Nucleic match: 1, Nucleic mismatch -3, Expectation value: 10, Word size: 11, Max scores: 25, and Max alignments: 15).

Preferred host cells are plant cells. Recombinant host cells, in the present context, are those which have been genetically modified to contain an isolated nucleic molecule, contain one or more deleted or otherwise non-functional genes normally present and functional in the host cell, or contain one or more genes to produce at least one recombinant protein. The nucleic acid(s) encoding the protein(s) of the present invention can be introduced by any means known to the art which is appropriate for the particular type of cell, including without limitation, transformation, lipofection, electroporation or any other methodology known by those skilled in the art.

Having generally described this invention, the same will be better understood by reference to certain specific examples, which are included herein to further illustrate the invention and are not intended to limit the scope of the invention as defined by the claims.

### EXAMPLES

The present disclosure is described in further detail in the following examples which are not in any way intended to limit the scope of the disclosure as claimed. The attached figures are meant to be considered as integral parts of the specification and description of the disclosure. The following example is offered to illustrate, but not to limit the claimed disclosure.

### Example 1: Generation of constructs and transgenic N. tabacum plants

The following example describes the generation of constructs and transgenic *N*. *tabacum* (tobacco) plants in order to test the combination of manipulation of genes involved in RuBP regeneration with manipulation of genes involved in electron transport. Two different tobacco cultivars with very different growth habits were used: *Nicotiana tabacum* cv. Petit Havana and *Nicotiana tabacum* cv. Samsun.

### Materials and Methods

*Generation of constructs:* Constructs were generated using Golden Gate cloning (Engler, et al., Plos One (2009) 4; Engler, et al., Plos One (2008) 3:e3647) or Gateway cloning technology (Nakagawa, et al., J. Biosci. Bioeng. (2007) 104:34-41). Transgenes were expressed under the control of CaMV35S and FMV constitutive promoters.

For *Nicotiana tabacum* cv. Petit Havana transgenic lines, the codon optimized cyanobacterial bifunctional fructose-1,6-bisphosphatases/sedoheptulose-1,7-bisphosphatase (FBP/SBPase; *slr2094 Synechocystis* sp. PCC 7942 (Miyagawa, et al., Nat. Biotechnol. (2001) 19:965-969)) linked to the geraniol synthase transit peptide (Simkin, et al., Phytochemistry (2013) 85:36-43), and the codon optimized *P. umbilicalis* cytochrome *c*₆ (AFC39870) with the chlorophyll a/b binding protein 6 transit peptide from *Arabidopsis thaliana* (AT3G54890) were used to generate Golden Gate (Engler, et al., Plos One (2008) 3:e3647) overexpression constructs (EC23083 and EC23028), driven by the FMV (Richins, et al., Nucleic Acids Res. (1987) 15:8451-8466) and CaMV 35S promoters, respectively **(****FIG. 1A****).**

For *N. tabacum* cv. Samsun transgenic lines, the full-length *P. umbilicalis* cytochrome *c*₆ gene linked to the transit peptide from the light-harvesting complex I chlorophyll a/b binding protein 6 (AT3G54890), driven by the CaMV 35S promoter, was used to generate over-expression construct B2-C6, in the vector pGWB2 (Nakagawa, et al., J. Biosci. Bioeng. (2007) 104:34-41) **(****FIG. 1B****).**

*Production of tobacco transformants:* Sixty lines of *N. tabacum* cv. Petit Havana, and twelve to fourteen lines of *N. tabacum* cv. Samsun were generated per construct. The recombinant plasmids EC23083 and EC23028 were introduced into WT *N. tabacum* cv. Petit Havana using *Agrobacterium tumefaciens* strain LBA4404 via leaf-disc transformation (Horsch, et al., Abstr. Pap. Am. Chem. S. (1985) 190:67), and shoots were regenerated on MS medium containing, hygromycin (20 mg L⁻¹) and cefotaxime (400 mg L⁻¹). Hygromycin resistant primary transformants (T0 generation) with established root systems were transferred to soil and allowed to self-fertilize. T0 and T1 lines expressing the integrated transgenes were screened using semi-quantitative RT-PCR. *N. tabacum* cv. Petit Havana T2/T3 progeny expressing FBP/SBPase (S_{B} lines: 03, 06, 21, 44) or cytochrome *c*₆ (C₆ lines: C15, C41, C47, C50) were selected from primary transformants produced as described above. *N. tabacum* cv. Petit Havana plants expressing both S_{B} and C₆ were generated by crossing S_{B} lines (S_{B}06, S_{B}44, S_{B}21) with C₆ lines (C15, C47, C50) to generate four independent S_{B}C₆ lines: S_{B}C1 (S_{B}06 x C47), S_{B}C2 (S_{B}06 x C50), S_{B}C3 (S_{B}44 x C47) and S_{B}C₆ (S_{B}21 x C15). These four independent lines were then allowed to self-pollinate.

The recombinant plasmid B2-C6 was introduced into the SBPase-overexpressing *N. tabacum* cv. Samsun T4 line described in Lefebvre, et al., Plant Physiol. (2005) 138:451-460, using *Agrobacterium tumefaciens* strain AGL1 via leaf-disc transformation (Horsch, et al., Abstr. Pap. Am. Chem. S. (1985) 190:67). Primary transformants (T0 generation, 39 plants) were regenerated on MS medium containing kanamycin (100 mg L⁻¹), hygromycin (20 mg L⁻¹) and augmentin (500 mg L⁻¹). Plants expressing the integrated transgenes were screened using semi-quantitative RT-PCR. *N. tabacum* cv. Samsun lines expressing SBPase + cytochrome *c*₆ (SC₆ lines: 1, 2 and 3) were allowed to self-pollinate, and progeny used for subsequent experiments were checked for the presence and expression of the transgene by semi-quantitative RT-PCR.

Control plants used in this study were a combined group of WT and null segregants from the transgenic lines *(i.e.,* azygous lines), which were verified by PCR and semi-quantitative RT-PCR for non-integration of the transgene. A full list of transgenic lines and control lines used in the experiments described in the below examples is provided in **Table 1.**

**Table 1: Tobacco transgenic lines and control lines used in experiments**

| Tobacco cultivar | Transgene(s) | **Generation** | **Lines** |
|---|---|---|---|
| *N. tabacum* cv. Petit Havana | FBP/SBPase | T2/T3 progeny of initial transformants | S_{B} lines: S_{B}03, S_{B}06, S_{B}21, S_{B}44 |
| | cytochrome *c*₆ | T2/T3 progeny of initial transformants | C₆ lines: C15, C41, C47, C50 |
| | FBP/SBPase + cytochrome *c*₆ | Cross of S_{B} and C₆ lines | S_{B}C₆ lines: S_{B}C1 (S_{B}06 x C47), S_{B}C2 (S_{B}06 x C50), S_{B}C3 (S_{B}44 x C47), S_{B}C4 (S_{B}44 x C50) and S_{B}C₆ (S_{B}21 x C15) |
| | None (azygous control) | Null segregants from S_{B}C₆ lines | aS_{B}C2 and aS_{B}C4 |
| | None (WT control) | N/A | N/A |
| *N. tabacum* cv. Samsun | SBPase | T4 lines (described in Lefebvre, et al., Plant Physiol. (2005) 138:451-460) | S lines: S30, S60 |
| | SBPase + cytochrome *c*₆ | T2/T3 progeny of initial transformation of S lines (T4 lines described in Lefebvre, et al. (2005)) | SC₆ lines: 1, 2, and 3 |
| | None (azygous control) | Null segregants from SC₆ lines | aSC₆ lines |
| | None (WT control) | N/A | N/A |

*Selection of tobacco transformants:* Semi-quantitative RT-PCR (described in Example 2) was used to detect the presence of the FBP/SBPase transcript in lines S_{B} and S_{B}C₆, the presence of the cytochrome *c*₆ transcript in lines C₆, S_{B}C₆ and SC₆, and the presence of the SBPase transcript in lines S and SC₆ **(****FIGS. 2A-2B****).** Immunoblot analysis was used to show that the selected S_{B} and S_{B}C₆ lines accumulated FBP/SBPase protein, and the S and SC₆ lines overexpressed the SBPase protein **(****FIGS. 3A-3B****;** immunoblot analysis described in Example 4). In addition to immunoblot analysis, total extractable FBPase activity in the leaves of the *N. tabacum* cv. Petit Havana S_{B} and C₆ lines (T2/T4 generation) and S_{B}C₆ lines (F3 homozygous generation; F1 was the initial seed from the cross) was determined. This analysis showed that S_{B} and S_{B}C₆ lines had increased levels of FBPase activity ranging from 34% to 47% more activity than the controls **(****FIG. 2B****).** The full set of assays showing the variation in FBPase enzyme activities from multiple S_{B} and S_{B}C₆ plants can be seen in **FIG. 4****.** In addition, expression of cytochrome *c*₆ protein in *c*₆ lines was determined by immunoblot using antibodies raised against the *P. umbilicalis* cytochrome *c*₆ protein. As shown in **FIG. 5A****,** a unique band appeared in the *P. umbilicalis* crude protein extract (P) and in the combined protein mix of C₆ lines 15, 41, and 47 (C₆). No bands were observed in wild type (WT) or the azygous (A) control **(****FIGS. 5A-5B****).**

Chlorophyll fluorescence analysis of *N. tabacum* cv. Petit Havana lines S_{B}, C₆ and S_{B}C₆ at an irradiance of 600 µmol m⁻² s⁻¹, or *N. tabacum* cv. Samsun lines S or SC₆ at an irradiance of 650 µmol m⁻² s⁻¹ showed that in young plants, the operating efficiency of photosystem two (PSII) photochemistry (*F*_{q}'/*F*ₘ') was significantly higher in all transgenic lines compared to either WT or null segregant controls **(****FIGS. 2C-2D****).** However, the *F*_{q}'/*F*ₘ' values of the S_{B}C₆ and SC₆ lines were not significantly different from the *F*_{q}'/*F*ₘ' values obtained from plants individually expressing FBP/SBPase (S_{B}), cytochrome *c*₆ (C₆), or SBPase (S).

### Example 2: cDNA generation and semi-quantitative RT-PCR

*cDNA generation:* The leaves used for cDNA generation were the same leaves used for photosynthetic measurements (see Example 7). Total RNA was extracted from tobacco leaf disks (sampled from glasshouse-grown plants and quickly frozen in liquid nitrogen) using the NucleoSpin^{®} RNA Plant Kit (Macherey-Nagel, Fisher Scientific, UK). cDNA was synthesized using 1 µg total RNA in 20 µl using the oligo-dT primer according to the protocol in the RevertAid Reverse Transcriptase kit (Fermentas, Life Sciences, UK). cDNA was diluted 1 in 4 to a final concentration of 12.5ng µL⁻¹.

*RT-PCR:* For semi-quantitative RT-PCR, 2 µL of RT reaction mixture (100 ng of RNA) in a total volume of 25 µL was used with DreamTaq DNA Polymerase (Thermo Fisher Scientific, UK) according to manufacturer's recommendations. PCR products were fractionated on 1.0% agarose gels. Primers used for semi-quantitative RT-PCR are provided in **Table 2,** below.

**Table 2: Primers used for semi-quantitative RT-PCR.**

| **Cultivar** | **Gene** | **Forward Primer** | **Reverse Primer** | **Amplicon** |
|---|---|---|---|---|
| *N*. *tabacum* cv. Samsun | **Cytochrome** *c*₆ | 5'TGCTGCAGATCTAGATAAT GG'3 (SEQ ID NO: 81) | 5'CGATCGTTCAAACATTT GGCA'3 (SEQ ID NO: 87) | 354 bp |
| | SBPase | 5'ATGGAGACCAGCATCGCG TGCTACTC'3 (SEQ ID NO: 82) | 5'CGATCGTTCAAACATTT GGCA'3 (SEQ ID NO: 88) | 1269 bp |
| | EF | 5'TGAGATGCACCACGAAGC TC'3 (SEQ ID NO: 83) | 5’CCAACATTGTCACCAG GAAGTG‘3 (SEQ ID NO: 89) | 479 bp |
| *N*. *tabacum* cv. Petit Havana | Cytochrome *c*₆ | 5'TCGCTTATGAGCTGTGGCA T'3 (SEQ ID NO: 84) | 5'CAACTAGCCGACCACC GAAG'3 (SEQ ID NO: 90) | 652 bp |
| | FBP/SBPase | 5'TGCTTCTGCTAAGTGGATG GG'3 (SEQ ID NO: 85) | 5’ACATCTCATAGCAGCA GCAGA‘3 (SEQ ID NO: 91) | 427 bp |
| | EF | 5'TGAGATGCACCACGAAGC TC'3 (SEQ ID NO: 86) | 5'CCAACATTGTCACCAG GAAGTG'3 (SEQ ID NO: 92) | 479 bp |

### Example 3: Plant Growth

### Generation of transgenic plant lines

Wild-type tobacco plants and T1 progeny resulting from self-fertilization of transgenic plants were grown to seed in soil (Levington F2, Fisons, Ipswich, UK). As described in Example 1, for the experiments in *N. tabacum* cv. Samsun, the null segregants were selected from transformed lines. For the experiments in *N. tabacum* cv. Petit Havana, the null segregants were selected from the S_{B}C₆ lines. Seeds used for experimental study were germinated as described below, and the resulting plants were grown in controlled conditions.

### Controlled conditions

For experimental study, T2-T4 and F1-F3 progeny seeds were germinated on soil in controlled environment chambers at an irradiance of 130 µmol photons m⁻² s⁻¹, a temperature of 22°C, in a relative humidity of 60%, and in a 16-h photoperiod (16-h light: 8-h dark). Plants were transferred to individual 8 cm pots and grown for two weeks under the same conditions (irradiance of 130 µmol photons m⁻²s⁻¹, temperature of 22°C, relative humidity of 60%, and a 16-h photoperiod). Plants were then transferred to 4 L pots and cultivated in a controlled-environment glasshouse (16-h photoperiod; temperature of between 25°C-30°C during the day and 20°C at night). During periods of low natural light induced by cloud cover, natural light was supplemented with high-pressure sodium light bulbs to provide a minimum irradiance of 380-1000 µmol photons m⁻² s⁻¹ (high-light), from the pot level to the top of the plant, respectively. The positions of the plants were changed 3 times each week, and plants were watered regularly with a nutrient medium (Hoagland, et al., The College of Agriculture (1950) 1). Plants were positioned such that at maturity, a near-to-closed canopy was achieved and the temperature range was maintained to be similar to the ambient external environment.

### Field

Plants were grown as described in López-Calcagno, et al., Plant Biotechnol. J. (2018). The field site was situated at the University of Illinois Energy Farm (40.11°N, 88.21°W, Urbana, IL). Two different experimental designs were used in 2 different years.

**FIG. 6A** shows the replicated control design used in 2016. Plants were grown in rows spaced 30 cm apart, with the outer boundary being a border of wild-type plants. The entire experiment was surrounded by a border of two rows of wild-type plants. Plants were irrigated when required using rain towers. T2 seed was germinated and seedlings were moved to individual pots (350 mL) after 11 days. The seedlings were grown in the glasshouse for a further 15 days before being moved into the field. Plants were allowed to grow in the field for 14 days before harvest.

**FIG. 6B** shows the blocks within rows design used in 2017, when two experiments were carried out two weeks apart. In the design, one block contains one independent transgenic line of each of the five constructs and each row has all lines. The central 20 plants of each block are divided into five rows of four plants per genotype. The 2017 experiment 1 contained controls (WT and null segregants), FBP/SBPase expressing lines (S_{B}) and cytochrome *c*₆ expressing lines (C₆). The 2017 experiment 2 contained controls (WT and null segregants), cytochrome *c*₆ expressing lines (C₆), and FBP/SBPase + cytochrome *c*₆ expressing lines (S_{B}C₆). The 2017 experiment also contained lines that were separately evaluated: lines overexpressing the H-protein of the glycine cleavage system (G lines) and the null segregants from these lines (aG lines) (data was published in Lopez-Calcagno, et al., Plant Biotechnol. J. (2019) 17(1):141-151), and lines expressing the B and C proteins and overexpressing the H-protein (S_{B}CG lines) and the null segregants from these lines (a S_{B}CG lines) (data not published). Seed was germinated and after 12 days moved to hydroponic trays (Trans-plant Tray GP009 6912 cells; Speedling Inc., Ruskin, FL). Seedlings were grown in the glasshouse for 31-33 days before being moved to the field. The plants were allowed to grow in the field until flowering (an additional 24-30 days) before harvest.

The field was prepared in both years as described in Kromdijk, et al., Science (2016) 354:857-861. Light intensity (LI-quantum sensor; LI-COR) and air temperature (Model 109 temperature probe; Campbell Scientific Inc., Logan, UT) were measured nearby on the same field site, and 15 minute averages (FIGS. 6A-6B) were logged using a data logger (CR1000; Campbell Scientific).

### Example 4: Protein Extraction and immunoblot analysis

Leaf discs (0.8 cm in diameter) were taken from the same areas of the leaf used for photosynthetic measurements (see Example 7) and immediately plunged into liquid N₂ and stored at -80°C. The leaf discs were ground in dry ice. Protein extractions were performed as described in Lopez-Calcagno, et al., J. Exp. Bot. (2017) 68:2285-2298, or using the Nucleospin RNA/Protein kit (Macherey-Nagel; www.mn-net.com) during RNA preparations. Protein quantification was performed using a protein quantification Kit from Macherey-Nagel. Samples were loaded on an equal protein basis, separated using 12% (w/v) SDS-PAGE, transferred to a nitrocellulose membrane (GE Healthcare Life science, Germany), and probed using antibodies raised against SBPase and FBP/SBPase. Proteins were detected using horseradish peroxidase conjugated to the secondary antibody and ECL chemiluminescence detection reagent (Amersham, Buckinghamshire, UK). SBPase antibodies were previously characterized (Lefebvre, et al., Plant Physiol. (2005) 138:451-460; Dunford, et al., Protein Expr. Purif. (1998) 14:139-145). FBP/SBPase antibodies were raised against a peptide from a conserved region of the protein [C]-DRPRHKELIQEIRNAG-amide (SEQ ID NO: 93), and cytochrome *c*₆ antibodies were raised against peptide [C]-[Nle]-PDKTLKKDVLEANS-amide (SEQ ID NO: 94) (Cambridge Research Biochemicals, Cleveland, UK). In addition to the aforementioned antibodies, samples were probed using antibodies raised against transketolase (Henkes, et al., Plant Cell (2001) 13:535-551; Khozaei, et al., Plant Cell (2015) 27:432-447) and the Glycine decarboxylase H-protein for use as loading controls. Glycine decarboxylase H-protein antibodies were previously characterized in Timm, et al., Febs Lett. (2012) 586:3692-3697.

### Protein Extraction for cytochrome c₆

Whole leaves were harvested from 8 week old plants, washed in cold water and then wiped with a cloth soaked in 80% ethanol to remove the majority of leaf residue. The leaves were then washed twice more in cold water, the midrib was removed, and 50 g of the remaining tissue was placed in a sealed plastic bag and stored overnight in the dark at 4°C. Proteins were extracted as in Hiyama, Methods Mol. Biol. (2004) 274:11-17, with a few modifications. Leaf tissue was homogenized in 250 ml of chilled chloroplast preparation buffer (50 mM sodium phosphate buffer, pH 7, 10 mM NaCl) for 30 seconds. The solution was then filtered through 4 layers of muslin cloth and centrifuged at 10,000 x g for 5 minutes. The resulting pellet was then gently resuspended in 50 ml of chilled chloroplast preparation buffer and the chlorophyll concentration was measured and adjusted to approximately 2 mg ml⁻¹. The resulting mixture was then added to two volumes of preheated (45°C) solubilization medium (50 mM Tris-HCl, pH 8.8, and 3% triton X-100), incubated at 45°C for 30 minutes, and then chilled in an ice bath for a further 30 minutes before centrifugation at 12000 g for 30 minutes. The supernatant was stored at -80°C for use in the next stage. To purify cytochrome *c*₆ protein, a Biorad Econo-Pac High-Q 5ml type wash column was used at a flow rate of 1ml min⁻¹. First, the column was prepared by washing with 100 ml of starting buffer (10 mM Tris-HCl pH 8.8, 0.2% triton X-100, and 20% sucrose). Then, the protein mixture from the previous step was diluted with an equal volume of chilled starting buffer and passed through the column at a flow rate of 1 ml min⁻¹. Once all the protein was loaded onto the column, it was then washed with 1000 ml of starting buffer supplemented with 10 mM NaCl. The column was then washed with 300 ml of starting buffer supplemented with 50 ml NaCl, and finally the column was eluted with a linear gradient of starting buffer supplemented with NaCl concentrations from 50 mM to 200 mM over a period of 4 hours at a flow rate of 1 ml min⁻¹, with aliquots being collected at multiple times. Samples were mixed with 300µl of loading buffer (50% glycerol, 25% β- mercaptoethanol, 25% EDTA) and loaded on an equal protein basis, separated using 18% (w/v) SDS-PAGE, transferred to nitrocellulose membrane, and probed using antibodies raised against cytochrome *c*₆.

### Example 5: Determination of FBPase Activity by Phosphate Release

FBPase activity was determined by phosphate release as described previously for SBPase with minor modifications (Simkin, et al., J. Exp. Bot. (2015) 66:4075-4090). Leaf discs were obtained from the same leaves used for photosynthetic measurements (see Example 7), and discs were isolated and frozen in liquid nitrogen after photosynthesis measurements were completed. Leaf discs were ground to a fine powder in liquid nitrogen, immersed in extraction buffer (50 mM HEPES, pH8.2; 5 mM MgCl; 1 mM EDTA; 1 mM EGTA; 10% glycerol; 0.1% Triton X-100; 2 mM benzamidine; 2 mM aminocapronic acid; 0.5 mM phenylmethylsulfonylfluoride; 10 mM dithiothreitol), and centrifuged for 1 min at 14,000 x g, 4°C. The resulting supernatant (1 ml) was desalted through a NAP-10 column (Amersham) and stored in liquid nitrogen. The assay was carried out as descried in Simkin, et al., J. Exp. Bot. (2015) 66:4075-4090. In brief, 20 µl of extract were added to 80 µl of assay buffer (50 mM Tris, pH 8.2; 15 mM MgCl₂; 1.5 mM EDTA; 10 mM DTT; 7.5 mM fructose-1,6-bisphosphate) and incubated at 25°C for 30 min. The reaction was stopped by the addition of 50 µl of 1 M perchloric acid. 30 µl of samples or standards (PO³⁻₄ concentrations of 0.125 nmol to 4 nmol) were incubated for 30 min at room temperature following the addition of 300 µl of Biomol Green (Affiniti Research Products, Exeter, UK) and the light absorbance at 620 nm (A620) was measured using a microplate reader (VERSAmax, Molecular Devices, Sunnyvale, CA). FBPase activities were normalized to transketolase activity (Zhao, et al., Biomed. Res. Int. (2014) 2014:572915).

### Example 6: Chlorophyll fluorescence imaging screening in seedlings

Chlorophyll fluorescence imaging was performed on 2-3 week-old tobacco seedlings grown in a controlled environment chamber at 130 µmol mol⁻² s⁻¹ and ambient CO₂ concentration (400 µmol mol⁻¹). Chlorophyll fluorescence parameters were obtained using a chlorophyll fluorescence (CF) imaging system (Technologica, Colchester, UK (Barbagallo, et al., Plant Physiol. (2003)132:485-493; von Caemmerer, et al., J. Exp. Bot. (2004) 55:1157-1166)). The operating efficiency of photosystem two (PSII) photochemistry, *F*_{q}' /*F*ₘ', was calculated from measurements of steady state fluorescence in the light (F') and maximum fluorescence (*F*ₘ') following a saturating 800 ms pulse of 6300 µmol m⁻² s⁻¹ PPFD and using the following equation *F*_{q}'/*F*ₘ' = (*F*ₘ'*-F'*)/*F*ₘ'*.* Images of *F*_{q}' /*F*ₘ' were taken under stable PPFD of 600 µmol m⁻² s⁻¹ for *N. tabacum* cv. Petit Havana and under stable PPFD of 650 µmol m⁻² s⁻¹ for *N. tabacum* cv. Samsun (Baker, et al., Journal of Experimental Botany (2001) 52:615-621; Oxborough, et al., Philos. Trans. R. Soc. Lond. B. Biol. Sci. (2000) 355:1489-1498; Lawson, et al., J. Exp. Bot. (2008) 59:3609-3619).

### Example 7: Leaf Gas Exchange

Photosynthetic gas-exchange and chlorophyll fluorescence parameters were recorded using a portable infrared gas analyzer (LI-COR 6400; LI-COR, Lincoln, NE, USA) with a 6400-40 fluorometer head unit. Unless stated otherwise, all measurements were taken with LI-COR 6400 cuvettes. For plants grown in the glasshouse, conditions were maintained at a CO₂ concentration of 400 µmol mol⁻¹, leaf temperature of 25°C, and vapor pressure deficit (VPD) of 1 ± 0.2 kPa. The chamber conditions for plants grown under field conditions had a CO₂ concentration of 400 µmol mol⁻¹, the block temperature was set to 2°C above ambient temperature (ambient air temperature was measured before generation of each gas exchange response curve) and VPD was maintained as close to 1 kPa as possible.

### AlCᵢ response curves (Photosynthetic capacity)

The response of net photosynthesis (A) to intracellular CO₂ concentration (*C*ᵢ) was measured at a saturating light intensity of 2000 µmol mol⁻² s⁻¹. Illumination was provided by a red-blue light source attached to the leaf cuvette. Measurements of A were started at ambient CO₂ concentration (Cₐ) of 400 µmol mol⁻¹, before Cₐ was decreased step-wise to a lowest concentration of 50 µmol mol⁻¹ and then increased step-wise to an upper concentration of 2000 µmol mol⁻¹. To calculate the parameters of maximum saturated CO₂ assimilation rate (*A*ₘₐₓ), maximum carboxylation rate (*Vc*ₘₐₓ) and maximum electron transport flow (*J*ₘₐₓ), the C3 photosynthesis model (Farquhar, et al., Planta (1980) 149:78-90) was fitted to the *A*/*C*ᵢ data using a spreadsheet provided by Sharkey, et al., Plant Cell Environ. (2007) 30:1035-1040. Additionally, chlorophyll fluorescence parameters including PSII operating efficiency (*F*_{q}'/*F*ₘ') and the coefficient of photochemical quenching (*q*_{P}), which is mathematically identical to the PSII efficiency factor *F*_{q}'/*F*ᵥ', were recorded at each point.

### A/Q response curves

Photosynthesis as a function of light (*A*/*Q* response curves) was measured under the same cuvette conditions as the *A*/*C*ᵢ curves mentioned above. Leaves were initially stabilized at saturating irradiance of 2200 to µmol m⁻² s⁻¹, after which A and *g*ₛ were measured at the following light levels: 2000 µmol m⁻² s⁻¹, 1650 µmol m⁻² s⁻¹, 1300 µmol m⁻² s⁻¹, 1000 µmol m⁻² s^{l}, 750 µmol m⁻² s⁻¹, 500 µmol m⁻² s⁻¹, 400 µmol m⁻² s⁻¹, 300 µmol m⁻² s⁻¹, 200 µmol m⁻² s⁻¹, 150 µmol m⁻² s⁻¹, 100 µmol m⁻² s⁻¹, 50 µmol m⁻² s⁻¹ and 0 µmol m⁻² s⁻¹. Measurements were recorded after A reached a new steady state (1 min to 3 min) and before *g*ₛ changed to the new light levels. Values of *A* and *g*ₛ were used to estimate the intrinsic water-use efficiency (*iWUE =A*/*gs*).

### Example 8: Statistical Analysis

All statistical analyses were done using Sys-stat, University of Essex, UK, and R (see the website www.r-project.org). For harvest data, seedling chlorophyll imaging, and enzyme activities, analysis of variance (ANOVA) and Post hoc Tukey tests were done. For gas exchange curves, data were compared by linear mixed model analysis using lmer function and type III ANOVA (Vialet-Chabrand, et al., Plant Physiol. (2017) 173:2163-2179). Significant differences between manipulations were identified using contrasts analysis (lsmeans package).

### Example 9: Stimulation of electron transport and RuBP regeneration increases photosynthetic performance in two distinct tobacco varieties under glasshouse conditions

Transgenic lines selected based on the initial screens described above were grown in the glasshouse, with natural light supplemented to provide illumination of between 400 µmol m⁻² s^{- 1} to 1000 µmol m⁻² s⁻¹. The rate of net CO₂ assimilation (*A*) and *F*_{q}'/*F*ₘ' were determined as a function of internal CO₂ concentration (*C*ᵢ) in mature and developing leaves of *N. tabacum* cv. Samsun (S and SC₆) and in mature leaves of *N. tabacum* cv. Petit Havana (S_{B}, C₆ and S_{B}C₆) **(****FIGS. 7A-7B****).** The transgenic lines displayed greater CO₂ assimilation rates than that of the control plants (CN). *A* was 15% higher than the controls in the mature leaves of the SC₆, at a *C*ᵢ of approximately 300 µmol mol⁻¹ (while current ambient CO₂ concentrations are around 400 µmol mol-1, the measured Cᵢ concentration is lower than the ambient due to multiple factors, including stomatal limitation) **(****FIG. 7B****).** The developing leaves of the SC₆ plants also showed significant increases in PSII operating efficiency (*F*_{q}'/*F*ₘ'), and in the PSII efficiency factor (*F*_{q}'/*F*ᵥ'), which was determined by the ability of the photosynthetic apparatus to maintain Q_{A} in the oxidized state, and is therefore a measure of photochemical quenching when compared to control plants **(****FIG. 7B****).** Interestingly, in mature leaves of *N. tabacum* cv. Samsun transgenic plants, the differences in assimilation rates and in the operating efficiency of PSII photochemistry between the transgenic and the control plants were smaller than in the developing leaves. Only the mature leaves of S transgenic plants displayed higher average values for *F*_{q}'/*F*ₘ' and *F*_{q}'/*F*ᵥ' relative to the control plants at all measured CO₂ concentrations **(****FIG. 7B****).** In contrast, the mature leaves of SC₆ plants displayed *F*_{q}'/*F*ᵥ' values higher than the control only at Cᵢ levels between 300 µmol m⁻¹ and 900 µmol m⁻¹ **(****FIG. 7B****).**

Similar trends were shown for the *N. tabacum* cv. Petit Havana transgenic plants which displayed higher average values of *A, F*_{q}'/*F*ₘ', and *F*_{q}'/*F*ᵥ' compared to controls **(****FIG. 7A****).** In the mature leaves of the S_{B}C₆ plants *(N. tabacum* cv. Petit Havana) these significant increases were similar to the trends shown for the developing leaves of the SC₆ lines (N. *tabacum* cv. Samsun) **(****FIGS. 7A-7B****).**

The developing leaves of both the S and SC₆ plants *(N. tabacum* cv. Samsun) showed significant increases in *J*ₘₐₓ and *A*ₘₐₓ when compared to control plants **(Table 3).** The mature leaves of the SC₆ transgenic plants also displayed a significantly higher *Vc*ₘₐₓ, *J*ₘₐₓ, and *A*ₘₐₓ values relative to the control plants. In contrast, the leaves of the S_{B}C₆ plants (*N*. *tabacum* cv. Petit Havana) only had significant increases in *A*ₘₐₓ, although higher average values for *Vc*ₘₐₓ, and *J*ₘₐₓ were evident. These results showed that simultaneous stimulation of electron transport and RuBP regeneration by expression of cytochrome *c₆* in combination with FBP/SBPase or SBPase has a greater impact on photosynthesis than the single manipulations in all analyzed plants.

**Table 3. Maximum electron transport and RuBP regeneration rate (Jₘₐₓ), maximum carboxylation rate of Rubisco (Vcₘₐₓ) and maximum assimilation (Aₘₐₓ) of wild-type and transgenic lines¹.**

| | *AlC*ᵢ | | | | |
|---|---|---|---|---|---|
| | Leaf Stage | Line | *Vc*ₘₐₓ (µmol m⁻² s⁻¹) | *Jₘₐₓ* (µmol m² s⁻¹) | *A*ₘₐₓ (µmol m⁻² s⁻¹) |
| *N. tabacum* cv. Samsun | Developing | CN | 72.32 ± 5.5 | 157.51 ± 6.0 | 29.6 ± 1.1 |
| | | S | 87.7 ± 4.3 | **179.8 ± 4.9*** | **34.1 ± 0.7*** |
| | | SC₆ | 86.5 ± 3.5 | **181.2 ± 3.6*** | **33.7 ± 1.1*** |
| | Mature | CN | 77.2 ± 3.3 | 171.0 ± 6.0 | 31.6 ± 1.0 |
| | | S | 81.3 ± 6.1 | 183.5 ± 9.0 | 32.2 ± 0.7 |
| | | SC₆ | 90.3 ± 3.3 | 193.1 ± 5.4 | **34.9 ± 1.1*** |
| *N. tabacum* cv. Petit Havana | Mature | CN | 69.6 ± 2.0 | 121.5 ± 1.3 | 24.6 ± 0.5 |
| | | S_{B} | 69.0 ± 5.1 | 128.7 ± 3.8 | 27.0 ± 0.8 |
| | | C₆ | 79.3 ± 7.0 | 129.9 ± 5.1 | 25.6 ± 0.5 |
| | | S_{B}C₆ | 76.5 ± 4.2 | 132.0 ± 3.8 | **27.4 ± 0.8*** |

| | | | | | |
|---|---|---|---|---|---|
| ¹ Results were determined from the A/Cᵢ curves in **FIGS. 7A-7B** using the equations published in von Caemmerer, et al., Planta (1981) 153:376-387. Statistical differences are shown in boldface (*p<0.05), and n = 6-11 plants per manipulation. Mean and SE are shown. | | | | | |

### Example 10: Stimulation of electron transport and RuBP regeneration stimulates growth in two distinct tobacco varieties under glasshouse conditions

In parallel experiments, *N. tabacum* cv. Petit Havana plants expressing FBP/SBPase (S_{B}), cytochrome *c*₆ (C₆), or FBP/SBPase + cytochrome *c*₆ (S_{B}C₆) were grown in controlled conditions for four weeks before harvesting, and *N. tabacum* cv. Samsun plants expressing SBPase (S), or SBPase + cytochrome *c*₆ (SC₆) were grown in controlled conditions for six weeks before harvesting. Height, leaf number, total leaf area and above ground biomass were determined **(****FIGS. 8** **and** **9****).** All of the analyzed transgenic plants displayed larger heights relative to control plants. Plants expressing cytochrome *c*₆ (C₆ and S_{B}C₆ = *N. tabacum* cv. Petit Havana; and SC₆ = *N. tabacum* cv. Samsun) had a significant increase in leaf area and in stem and leaf biomass compared to their respective controls. In the S_{B} transgenic plants *(N. tabacum* cv. Petit Havana) only the biomass of the stem was greater than in the control plants. Notably, the S_{B}C₆ and SC₆ transgenics displayed significantly greater leaf area than the single S_{B} and S transgenic plants, respectively. The total increases in above ground biomass when compared to the control groups were 35% for S_{B}, 44% for C₆ and 9% for S. The double-manipulation transgenic lines (S_{B}C₆ and SC₆) showed consistently higher above ground mass averages relative to control groups; 52% higher for S_{B}C₆ and 32% higher for SC₆ **(****FIGS. 8** **and** **9****).**

### Example 11: Simultaneous expression of FBP/SBPase and cytochrome c₆ increases growth and water use efficiency under field conditions

To test whether the increases in biomass observed in the transgenic plants under controlled glasshouse conditions could be reproduced in a field environment, a subset of lines was selected for testing in the field. Since larger percent increases in biomass were displayed by the transgenic *N. tabacum* cv. Petit Havana lines, these plants were selected and tested in three field experiments in two different years (one in 2016, and two in 2017).

In 2016, a small-scale replicated control experiment of the lines expressing single gene constructs for FBP/SBPase (S_{B}) and cytochrome *c*₆ (C₆) was carried out to evaluate vegetative growth in the field. Plants were germinated and grown under controlled environment conditions for 26 days before being moved to the field. After 14 days in the field, plants were harvested at an early vegetative stage and plant height, total leaf area, and above ground biomass were measured **(****FIG. 10A****).** These data revealed that, relative to controls, the S_{B} plants showed an increase in height, leaf area and above ground biomass of 27%, 35% and 25%, respectively **(****FIG. 10A****).** C₆ plants also showed an increase relative to controls in height, leaf area and above ground biomass of 50%, 41%, and 36%, respectively **(****FIG. 10A****).** In 2017, two larger scale, randomized block design field experiments were carried out to evaluate the performance of S_{B}, C₆, and S_{B}C₆ plants relative to control plants. Plants were grown from seed in the glasshouse for 31-13 days, and then moved to the field and allowed to grow until the onset of flowering (an additional 24-30 days) before harvesting. In **FIGS. 10B-10C****,** it can be seen that the S_{B} and C₆ plants harvested after the onset of flowering did not display any significant increases in height, leaf area or biomass. Interestingly, plants expressing FBP/SBPase + cytochrome *c*₆ (S_{B}C₆) displayed a significant increase in a number of growth parameters, with 13%, 17% and 27% increases in height, leaf area, and above ground biomass, respectively, when compared to controls **(****FIG. 10C****).**

Additionally, in the 2017 field experiments, *A* as a function of *Cᵢ* at saturating light (*A*/*C*ᵢ) was determined. In the 2017 experiment 1, a significant increase in *A* was observed in S_{B} and C₆ plants without differences in PSII operating efficiency (*F*_{q}'/*F*ₘ') **(****FIG. 11A****).** However, in the 2017 experiment 2, no differences in *A* or in *F*_{q}'/*F*ₘ' values were evident in the C₆ and S_{B}C₆ plants when compared to the control plants **(****FIG. 11B****).** Analysis of *A* as a function of light (PPFD) showed either small or not significant differences in *A* between genotypes **(****FIG. 12A** and **FIG. 13A****).** Interestingly, stomatal conductance (*gₛ*) in the S_{B}C₆ plants was significantly lower than in C₆ or control plants at light intensities above 1000 µmol m⁻² s⁻¹ **(****FIG. 12B****).** This resulted in a significant increase in intrinsic water use efficiency (*iWUE*) for S_{B}C₆ plants **(****FIG. 12D****).** No significant differences in *iWUE* were observed for S_{B} or C₆ transgenic plants **(****FIG. 12D** and **FIG. 13D****).**

The above examples describe the generation and analysis of transgenic plants with simultaneous increases in electron transport and improved capacity for RuBP regeneration in two different tobacco cultivars. These examples show that independent stimulation of electron transport (by expression of cytochrome *c*₆) and stimulation of RuBP regeneration (by expression of FBP/SBPase or overexpression of SBPase) increased photosynthesis and biomass in plants grown under controlled conditions. Furthermore, these examples demonstrated that the targeting of these two processes simultaneously (in the S_{B}C₆ and SC₆ plants) had an even greater effect in stimulating photosynthesis and growth. Additionally, in field studies, the plants with simultaneous stimulation of electron transport and of RuBP regeneration presented increased *iWUE* and biomass.

Under glasshouse conditions, increases in photosynthetic parameters were observed in all of the analyzed transgenic plants, and these were found to be consistently correlated with increases in biomass. The examples presented here provide the first report of increased photosynthesis and biomass by the simultaneous stimulation of electron transport and RuBP regeneration. Increases in A were observed under glasshouse conditions in the leaves of all analyzed transgenic tobacco plants in both tobacco cultivars tested here (*N. tabacum* cv. Petit Havana and *N. tabacum* cv. Samsun). Analysis of the *A*/*Cᵢ* response curves showed that the average values for the photosynthetic parameters *Vc*ₘₐₓ, *J*ₘₐₓ, and *A*ₘₐₓ increased by up to 17%, 14%, and 12%, respectively. These results indicated that not only was the maximal rate of electron transport and RuBP regeneration increased, but the rate of carboxylation by Rubisco was also increased. Although Rubisco activity was not directly targeted, this result is consistent with a study by Wullschleger, et al., J. Exp. Bot. (1993) 44:907-920 of over 100 plant species that showed a linear correlation between *J*ₘₐₓ and *V_{cmax}.* Furthermore, it has also been shown previously that overexpression of SBPase leads not only to a significant increase in *J*ₘₐₓ, but also increases in *Vc*ₘₐₓ and Rubisco activation state.

Notably, in the greenhouse study, the highest photosynthetic rates were obtained from the leaves of plants in which both electron transport and RuBP regeneration (S_{B}C₆ and SC₆) were increased, showing that the co-expression of these genes results in an additive effect on improving photosynthesis. In addition to the increases in *A,* the plants with simultaneous stimulation of electron transport and RuBP regeneration displayed a significant increase in *F*_{q}'/*F*ₘ', indicating a higher quantum yield of linear electron flux through PSII compared to the control plants. These results show that reduction of PSI is stimulated by using alternative, more efficient electron donors to PSI (Chida, et al., Plant Cell Physiol. (2007) 48:948-957; Finazzi, et al., Proc. Natl. Acad. Sci. U S A. (2005) 102:7031-7036), which is consistent with published data showing that introduction of cytochrome *c*₆ and overexpression of the Rieske FeS protein in Arabidopsis (Simkin, et al., Plant Physiol. (2017) 175:134-145; Chida, et al., Plant Cell Physiol. (2007) 48:948-957) causes increases in the quantum yield of PSII and a more oxidized plastoquinone pool. Furthermore, in the S_{B}C₆ and SC₆ plants, the increase in F_{q}'/Fₘ' was found to be largely driven by the increase in the PSII efficiency factor (F_{q}'/Fᵥ'). This suggests that the increase in efficiency in these plants is likely due to stimulation of processes downstream of PSII, such as CO₂ assimilation.

To provide further evidence of the applicability of targeting both electron transport and RuBP regeneration to improve crop yields, plants were tested in the field. The field results showed that the expression of FBP/SBPase alone led to an increase in growth and biomass in the 2016 field-grown plants of between 22% to 40% when harvested during early vegetative growth (prior to the onset of flowering). Interestingly, when plants with the same transgenic manipulations were harvested later in development, after the onset of flowering in the 2017 field trials, this advantage was no longer evident and the single FBP/SBPase expressing-lines were indistinguishable from the control plants.

The transgenic plants expressing cytochrome *c*₆ alone also showed enhanced growth and biomass when harvested early in development, but as with the FBP/SBPase plants, this improvement was no longer evident when plants were harvested after flowering. This phenotypic difference in biomass gain between early and late harvest was not observed in a parallel experiment where the overexpression of H-protein was shown to increase biomass under field conditions in plants harvested in early development and after the onset of flowering (López-Calcagno, et al., Plant Biotechnol. J. (2019) 17(1):141-151)). These results suggest that the expression of FBP/SBPase or cytochrome *c*₆ alone may provide an advantage under particular sets of conditions or at specific stages of plant development. This might be exploitable for some crops where an early harvest is desirable (*e.g.,* some types of lettuce, spinach, and tender greens) (Ichikawa, et al., GM Crops (2010) 1:322-326). In contrast with the results with the single manipulations described above, plants simultaneously expressing both cytochrome *c₆* and FBP/SBPase displayed a consistent increase in biomass after flowering under field conditions.

In the transgenic lines grown in the field, the correlations between increases in photosynthesis and biomass were less consistent than those observed under glasshouse conditions. The transgenic lines with individual manipulations, namely FBP/SBPase (S_{B} lines) and cytochrome *c*₆ (C₆ lines) had significant increases in photosynthetic capacity in the 2017 experiment 1, without an increase in biomass. In contrast, the C₆ lines in 2017 experiment 2 had increased biomass, but no significant differences in photosynthetic capacity. The transgenic lines with double gene manipulations, namely FBP/SBPase + cytochrome *c*₆ (S_{B}C₆) also had increased biomass without significant differences in photosynthetic capacity in 2017 experiment 2. Across all experiments, the average A values of the transgenic plants were consistently higher than those of the controls. Even if the differences were not consistently statistically different across all experiments, it is known that even small increases in assimilation throughout the lifetime of a plant will have a cumulative effect, which could translate into a significant biomass accumulation (Simkin, et al., J. Exp. Bot. (2015) 66:4075-4090).

At light intensities above 1000 µmol m² s⁻¹, it was observed that plants with simultaneous expression of FBP/SBPase + cytochrome *c*₆ (S_{B}C₆) had lower stomatal conductance (gs) and lower *C*ᵢ concentration when compared to control plants (**FIG. 12C****).** Normally, lower *C*ᵢ would be expected to lead to a reduction in photosynthesis, but interestingly, these plants were able to maintain CO₂ assimilation rates equal to or higher than control plants, resulting in an improvement in *iWUE.* A similar improvement in *iWUE* was seen in plants overexpressing the NPQ-related protein, PsbS (Glowacka, et al., Nat. Commun. (2018) 9). It was shown that light-induced stomatal opening was reduced in these plants, which had a more oxidized QA pool which has been proposed to act as a signal in stomatal movement (Busch, Photosynth. Res. (2014) 119:131-140).

The results in these examples provide support for the proposal that the increased photosynthetic capacity in S_{B}C₆ plants compensates for the reduction *C*ᵢ. The higher *iWUE* and the fact that a higher productivity compared to controls has been reported in field studies with CO₂ enrichment (Rosenthal, et al., BMC Plant Biol. (2011) 11:123; Ichikawa, et al., GM Crops (2010) 1:322-326) for transgenic lines with increased RuBP regeneration highlights the potential of manipulating electron transport and RuBP regeneration for the development of new plant varieties able to sustain photosynthesis and yields under climate change scenarios.

The results in these examples provide a clear demonstration that combining manipulations leading to simultaneous stimulation of electron transport and RuBP regeneration under the conditions tested leads to significant increases in biomass over the single manipulations and emphasizes the potential of this strategy for the development of high yielding crops.

## Claims

1. A genetically altered plant, plant part, or plant cell, wherein the plant, part thereof or cell comprises one or more RuBP regeneration enhancing genetic alterations that increase activity of a Calvin Benson cycle (CB) protein and one or more photosynthetic electron transport enhancing genetic alterations as compared to the unaltered plant, plant part, or plant cell grown under the same conditions,
wherein the one or more RuBP regeneration enhancing genetic alterations comprise overexpression of a CB protein, and wherein the CB protein comprises a sedoheptulose-1,7-bisphosphatase (SBPase), a fructose-1,6-bisphophate aldolase (FBPA), a chloroplastic fructose-1,6-bisphosphatase (FBPase), or a bifunctional fructose-1,6-bisphosphatases/sedoheptulose-1,7-bisphosphatase (FBP/SBPase);
wherein the one or more photosynthetic electron transport enhancing genetic alterations comprise overexpression of one or more photosynthetic electron transport proteins, and wherein the one or more photosynthetic electron transport proteins comprises a cytochrome c₆ protein, a Rieske FeS protein, or a cytochrome c₆ protein and a Rieske FeS protein; and
wherein the plant is not exclusively obtained by means of an essentially biological process.

2. The genetically altered plant, plant part, or plant cell of claim 1, wherein the cytochrome c₆ protein comprises an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 102; or
wherein the Rieske FeS protein comprises an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 101.

3. The genetically altered plant, plant part, or plant cell of claim 1 or 2, wherein the cytochrome c₆ protein is localized to a thylakoid lumen of at least one chloroplast within a cell of the genetically altered plant.

4. The genetically altered plant, plant part, or plant cell of claim 3, wherein the cytochrome c₆ protein comprises a transit peptide that localizes the cytochrome c₆ protein to the thylakoid lumen, and wherein the transit peptide comprises a chlorophyll a/b binding protein 6 transit peptide, a light-harvesting complex I chlorophyll a/b binding protein 1 transit peptide, or a plastocyanin signal peptide.

5. The genetically altered plant, plant part, or plant cell of claim 1 or 2, wherein the Rieske FeS protein is localized to a thylakoid membrane of at least one chloroplast within a cell of the genetically altered plant.

6. The genetically altered plant, plant part, or plant cell of claim 5, wherein the Rieske FeS protein comprises a transit peptide that localizes the Rieske FeS protein to the thylakoid membrane, and wherein the transit peptide comprises a cytochrome f transit peptide, a cytochrome b6 transit peptide, a PetD transit peptide, a PetG transit peptide, a PetL transit peptide, a PetN transit peptide, a PetM transit peptide, or a plastoquinone transit peptide.

7. The genetically altered plant, plant part, or plant cell of any one of claims 1-6, further comprising a plant promoter operably linked to a nucleic acid sequence encoding the cytochrome c₆ protein or the Rieske FeS protein, wherein the plant promoter comprises a constitutive promoter, an inducible promoter, a tissue or cell type specific promoter, or an inducible, tissue or cell type specific promoter.

8. The genetically altered plant, plant part, or plant cell of claim 1, wherein the SBPase comprises an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 96,
wherein the FBPA comprises an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 97,
wherein the FBPase comprises an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 98, or
wherein the FBP/SBPase comprises an amino acid sequence with at least 70% sequence identity to, at least 75% sequence identity to, at least 80% sequence identity to, at least 85% sequence identity to, at least 90% sequence identity to, at least 95% sequence identity to, or at least 99% sequence identity to SEQ ID NO: 99.

9. The genetically altered plant, plant part, or plant cell of claim 8, wherein the SBPase, the FBPA, the FBPase, or the FBP/SBPase is localized to a chloroplast stroma of at least one chloroplast within a cell of the genetically altered plant, and wherein the SBPase, the FBPA, the FBPase, or the FBP/SBPase comprises a transit peptide that localizes the SBPase, the FBPA, the FBPase, or the FBP/SBPase to the chloroplast stroma in the plant.

10. The genetically altered plant, plant part, or plant cell of any one of claims 8 or 9, further comprising a plant promoter operably linked to a nucleic acid sequence encoding the SBPase, the FBPA, the FBPase, or the FBP/SBPase, wherein the plant promoter comprises a constitutive promoter, an inducible promoter, a tissue or cell type specific promoter, or an inducible, tissue or cell type specific promoter.

11. The genetically altered plant of any one of claims 1-10, wherein the plant has increased biomass as compared to an unaltered wild type (WT) plant.

12. The genetically altered plant of any one of claims 1-11, wherein the plant has improved water use efficiency as compared to an unaltered WT plant when grown in conditions with light intensities above 1000 µmol m⁻² s⁻¹.

13. The genetically altered plant of any one of claims 1-12, wherein the plant is selected from the group of cowpea, soybean, cassava, rice, wheat, barley, tomato, potato, tobacco, canola, or other C3 crop plants.

14. A method of producing the genetically altered plant of any one of claims 1-13, comprising:
a) introducing the one or more RuBP regeneration enhancing genetic alterations that increase activity of a CB protein, the one or more photosynthetic electron transport enhancing genetic alterations, or both the one or more RuBP regeneration enhancing genetic alterations that increase activity of a CB protein and the one or more photosynthetic electron transport enhancing genetic alterations into a plant cell, tissue, or other explant;
b) regenerating the plant cell, tissue, or other explant into a genetically altered plantlet; and
c) growing the genetically altered plantlet into a genetically altered plant with the one or more RuBP regeneration enhancing genetic alterations that increase activity of a CB protein, the one or more photosynthetic electron transport enhancing genetic alterations, or both the one or more RuBP regeneration enhancing genetic alterations that increase activity of a CB protein and the one or more photosynthetic electron transport enhancing genetic alterations.

15. The genetically altered plant of any one of claims 1-13 or the method of claim 14, wherein the CB protein comprises a bifunctional fructose-1,6-bisphosphatases/sedoheptulose-1,7-bisphosphatase (FBP/SBPase) and the one or more photosynthetic electron transport proteins comprises a cytochrome c₆ protein.

## Patentansprüche

1. Genetisch veränderte Pflanze, Pflanzenteil oder Pflanzenzelle, wobei die Pflanze, der Pflanzenteil oder die Zelle Folgendes umfasst: eine oder mehrere genetische Veränderungen, die die RuBP-Regeneration fördern, die die Aktivität eines Calvin-Benson-Zyklus (CB)-Proteins erhöhen, und eine oder mehrere genetische Veränderungen, die den photosynthetischen Elektronentransport im Vergleich zur unveränderten Pflanze, dem Pflanzenteil oder der Pflanzenzelle, die unter denselben Bedingungen gezüchtet wurden, verbessern,
wobei die eine oder mehreren genetischen Veränderungen zur Steigerung der RuBP-Regeneration die Überexpression eines CB-Proteins umfassen, und wobei das CB-Protein Folgendes umfasst: eine Sedoheptulose-1,7-Bisphosphatase (SBPase), eine Fructose-1,6-bisphosphat-Aldolase (FBPA), eine chloroplastische Fructose-1,6-bisphosphatase (FBPase) oder eine bifunktionelle Fructose-1,6-bisphosphatase/Sedoheptulose-1,7-bisphosphatase (FBP/SBPase);
wobei die eine oder mehreren genetischen Veränderungen zur Steigerung des photosynthetischen Elektronentransports eine Überexpression eines oder mehrerer Proteine des photosynthetischen Elektronentransports umfassen, und wobei das eine oder die mehreren Proteine des photosynthetischen Elektronentransports ein Cytochrom-C₆-Protein, ein Rieske-FeS-Protein oder ein Cytochrom-C₆-Protein und ein Rieske-FeS-Protein umfassen; und
wobei die Pflanze nicht ausschließlich mittels eines im Wesentlichen biologischen Verfahrens gewonnen wird.

2. Genetisch veränderte Pflanze, Pflanzenteil oder Pflanzenzelle nach Anspruch 1, wobei das Cytochrom-C₆-Protein eine Aminosäuresequenz umfasst, die mindestens 70 % Sequenzidentität, mindestens 75 % Sequenzidentität, mindestens 80 % Sequenzidentität, mindestens 85 % Sequenzidentität, mindestens 90 % Sequenzidentität, mindestens 95 % Sequenzidentität oder mindestens 99 % Sequenzidentität mit SEQ ID NO: 102 aufweist; oder
wobei das Rieske-FeS-Protein eine Aminosäuresequenz umfasst, die mindestens 70 % Sequenzidentität, mindestens 75 % Sequenzidentität, mindestens 80 % Sequenzidentität, mindestens 85 % Sequenzidentität, mindestens 90 % Sequenzidentität, mindestens 95 % Sequenzidentität oder mindestens 99 % Sequenzidentität mit SEQ ID NO: 101 aufweist.

3. Genetisch veränderte Pflanze, Pflanzenteil oder Pflanzenzelle nach Anspruch 1 oder 2, wobei das Cytochrom-C₆-Protein im Thylakoid-Lumen mindestens eines Chloroplasten innerhalb einer Zelle der genetisch veränderten Pflanze lokalisiert ist.

4. Genetisch veränderte Pflanze, Pflanzenteil oder Pflanzenzelle nach Anspruch 3, wobei das Cytochrom-C₆-Protein ein Transitpeptid umfasst, das das Cytochrom-C₆-Protein in das Thylakoidlumen lokalisiert, und wobei das Transitpeptid ein Chlorophylla/b-bindendes Protein-6-Transitpeptid, ein Lichtrezeptorkomplex-I-Chlorophyll-a/b-bindendes Protein-1-Transitpeptid oder ein Plastocyanin-Signalpeptid umfasst.

5. Genetisch veränderte Pflanze, Pflanzenteil oder Pflanzenzelle nach Anspruch 1 oder 2, wobei das Rieske-FeS-Protein an einer Thylakoidmembran mindestens eines Chloroplasten innerhalb einer Zelle der genetisch veränderten Pflanze lokalisiert ist.

6. Genetisch veränderte Pflanze, Pflanzenteil oder Pflanzenzelle nach Anspruch 5, wobei das Rieske-FeS-Protein ein Transitpeptid umfasst, das das Rieske-FeS-Protein an der Thylakoidmembran lokalisiert, und wobei das Transitpeptid Folgendes umfasst: ein Cytochrom-f-Transitpeptid, ein Cytochrom-b6-Transitpeptid, ein PetD-Transitpeptid, ein PetG-Transitpeptid, ein PetL-Transitpeptid, ein PetN-Transitpeptid, ein PetM-Transitpeptid oder ein Plastoquinon-Transitpeptid.

7. Genetisch veränderte Pflanze, Pflanzenteil oder Pflanzenzelle nach einem der Ansprüche 1 bis 6, die ferner einen Pflanzenpromotor umfasst, der funktionsfähig mit einer Nukleinsäuresequenz verbunden ist, die für das Cytochrom-C₆-Protein oder das Rieske-FeS-Protein codiert, wobei der Pflanzenpromotor einen konstitutiven Promotor, einen induzierbaren Promotor, einen gewebe- oder zelltypspezifischen Promotor oder einen induzierbaren, gewebe- oder zelltypspezifischen Promotor umfasst.

8. Genetisch veränderte Pflanze, Pflanzenteil oder Pflanzenzelle nach Anspruch 1, wobei die SBPase eine Aminosäuresequenz umfasst, die mindestens 70 % Sequenzidentität, mindestens 75 % Sequenzidentität, mindestens 80 % Sequenzidentität, mindestens 85 % Sequenzidentität, mindestens 90 % Sequenzidentität, mindestens 95 % Sequenzidentität oder mindestens 99 % Sequenzidentität mit SEQ ID NO: 96 aufweist,
wobei die FBPA eine Aminosäuresequenz umfasst, die mindestens 70 % Sequenzidentität, mindestens 75 % Sequenzidentität, mindestens 80 % Sequenzidentität, mindestens 85 % Sequenzidentität, mindestens 90 % Sequenzidentität, mindestens 95 % Sequenzidentität oder mindestens 99 % Sequenzidentität mit SEQ ID NO: 97 aufweist,
wobei die FBPase eine Aminosäuresequenz umfasst, die mindestens 70 % Sequenzidentität, mindestens 75 % Sequenzidentität, mindestens 80 % Sequenzidentität, mindestens 85 % Sequenzidentität, mindestens 90 % Sequenzidentität, mindestens 95 % Sequenzidentität oder mindestens 99 % Sequenzidentität mit SEQ ID NO: 98 aufweist oder
wobei die FBP/SBPase eine Aminosäuresequenz umfasst, die mindestens 70 % Sequenzidentität, mindestens 75 % Sequenzidentität, mindestens 80 % Sequenzidentität, mindestens 85 % Sequenzidentität, mindestens 90 % Sequenzidentität, mindestens 95 % Sequenzidentität oder mindestens 99 % Sequenzidentität mit SEQ ID NO: 99 aufweist.

9. Genetisch veränderte Pflanze, Pflanzenteil oder Pflanzenzelle nach Anspruch 8, wobei die SBPase, die FBPA, die FBPase oder die FBP/SBPase im Stroma mindestens eines Chloroplasten innerhalb einer Zelle der genetisch veränderten Pflanze lokalisiert ist, und wobei die SBPase, die FBPA, die FBPase oder die FBP/SBPase ein Transitpeptid umfasst, das die SBPase, die FBPA, die FBPase oder die FBP/SBPase im Chloroplasten-Stroma der Pflanze lokalisiert.

10. Gentechnisch veränderte Pflanze, Pflanzenteil oder Pflanzenzelle nach einem der Ansprüche 8 oder 9, die ferner einen Pflanzenpromotor umfasst, der funktionsfähig mit einer Nukleinsäuresequenz verbunden ist, die für die SBPase, die FBPA, die FBPase oder die FBP/SBPase codiert, wobei der Pflanzenpromotor einen konstitutiven Promotor, einen induzierbaren Promotor, einen gewebe- oder zelltypspezifischen Promotor oder einen induzierbaren, gewebe- oder zelltypspezifischen Promotor umfasst.

11. Genetisch veränderte Pflanze nach einem der Ansprüche 1-10, wobei die Pflanze im Vergleich zu einer unveränderten Wildtyp-Pflanze (WT) eine erhöhte Biomasse aufweist.

12. Genetisch veränderte Pflanze nach einem der Ansprüche 1-11, wobei die Pflanze im Vergleich zu einer unveränderten WT-Pflanze eine verbesserte Wassernutzungseffizienz aufweist, wenn sie unter Bedingungen mit Lichtintensitäten über 1000 µmol m⁻² s⁻¹ angebaut wird.

13. Genetisch veränderte Pflanze nach einem der Ansprüche 1-12, wobei die Pflanze aus der Gruppe bestehend aus Augenbohne, Sojabohne, Maniok, Reis, Weizen, Gerste, Tomate, Kartoffel, Tabak, Raps oder anderen C3-Kulturpflanzen ausgewählt ist.

14. Verfahren zur Herstellung der genetisch veränderten Pflanze nach einem der Ansprüche 1 bis 13, das umfasst:
Einbringen der einen oder mehreren genetischen Veränderungen zur Verbesserung der RuBP-Regeneration, die die Aktivität eines CB-Proteins erhöhen, der einen oder mehreren genetischen Veränderungen zur Verbesserung des photosynthetischen Elektronentransports oder sowohl der einen oder mehreren genetischen Veränderungen zur Verbesserung der RuBP-Regeneration, die die Aktivität eines CB-Proteins erhöhen, als auch der einen oder mehreren genetischen Veränderungen zur Verbesserung des photosynthetischen Elektronentransports in eine Pflanzenzelle, ein Gewebe oder ein anderes Explantat;
Regenerierung der Pflanzenzelle, des Gewebes oder eines anderen Explantats zu einem gentechnisch veränderten Pflänzchen; und
Züchten des genetisch veränderten Pflänzchens zu einer genetisch veränderten Pflanze mit der einen oder den mehreren genetischen Veränderungen zur Steigerung der RuBP-Regeneration, die die Aktivität eines CB-Proteins erhöhen, der einen oder mehreren genetischen Veränderungen zur Verbesserung des photosynthetischen Elektronentransports oder sowohl der einen oder mehreren genetischen Veränderungen zur Verbesserung der RuBP-Regeneration, die die Aktivität eines CB-Proteins erhöhen, als auch der einen oder mehreren genetischen Veränderungen zur Verbesserung des photosynthetischen Elektronentransports.

15. Genetisch veränderte Pflanze nach einem der Ansprüche 1 bis 13 oder Verfahren nach Anspruch 14, wobei das CB-Protein eine bifunktionelle Fructose-1,6-Bisphosphatase/Sedoheptulose-1,7-Bisphosphatase (FBP/SBPase) umfasst und das eine oder die mehreren Proteine des photosynthetischen Elektronentransports ein Cytochrom-C₆-Protein umfassen.

## Revendications

1. Plante, partie de plante ou cellule de plante génétiquement modifiée, ladite plante, partie de plante ou cellule comprenant au moins une modification génétique améliorant la régénération du RuBP qui augmente l'activité d'une protéine du cycle de Calvin Benson (CB) et au moins une modification génétique améliorant le transport des électrons photosynthétiques en comparaison d'une plante, partie de plante ou cellule de plante non modifiée ayant poussé dans les mêmes conditions,
dans laquelle la ou les modifications génétiques améliorant la régénération du RuBP comprennent une surexpression d'une protéine CB, ladite protéine CB comprenant une sédoheptulose-1,7-bisphosphatase (SBPase), une fructose-1,6-bisphophate aldolase (FBPA), une fructose-1,6-bisphosphatase (FBPase) chloroplastique, ou une fructose-1,6-bisphosphatases/sédoheptulose-1,7-bisphosphatase (FBP/SBPase) bifonctionnelle ;
dans laquelle la ou les modifications génétiques améliorant le transport des électrons photosynthétiques comprennent une surexpression d'une ou plusieurs protéines de transport des électrons photosynthétiques, la ou lesdites protéines de transport des électrons photosynthétiques comprenant une protéine cytochrome C₆, une protéine FeS de Rieske, ou une protéine cytochrome C₆ et une protéine FeS de Rieske ;
ladite plante n'étant pas exclusivement obtenue au moyen d'un procédé essentiellement biologique.

2. Plante, partie de plante ou cellule de plante génétiquement modifiée selon la revendication 1, dans laquelle la protéine cytochrome C₆ comprend une séquence d'acides aminés présentant au moins 70 % d'identité de séquence, au moins 75 % d'identité de séquence, au moins 80 % d'identité de séquence, au moins 85 % d'identité de séquence, au moins 90 % d'identité de séquence, au moins 95 % d'identité de séquence ou au moins 99 % d'identité de séquence avec la SEQ ID NO : 102, ou
dans laquelle la protéine FeS de Rieske comprend une séquence d'acides aminés présentant au moins 70 % d'identité de séquence, au moins 75 % d'identité de séquence, au moins 80 % d'identité de séquence, au moins 85 % d'identité de séquence, au moins 90 % d'identité de séquence, au moins 95 % d'identité de séquence ou au moins 99 % d'identité de séquence avec la SEQ ID NO : 101.

3. Plante, partie de plante ou cellule de plante génétiquement modifiée selon la revendication 1 ou 2, dans laquelle la protéine cytochrome C₆ est localisée dans un lumen du thylakoïde d'au moins un chloroplaste au sein d'une cellule de la plante génétiquement modifiée.

4. Plante, partie de plante ou cellule de plante génétiquement modifiée selon la revendication 3, dans laquelle la protéine cytochrome C₆ comprend un peptide de transit qui localise la protéine cytochrome C₆ dans le lumen du thylakoïde, ledit peptide de transit comprenant un peptide de transit de la protéine 6 de liaison à la chlorophylle a/b, un peptide de transit de la protéine 1 de liaison à la chlorophylle a/b du complexe I de captation de la lumière, ou un peptide signal de la plastocyanine.

5. Plante, partie de plante ou cellule de plante génétiquement modifiée selon la revendication 1 ou 2, dans laquelle la protéine FeS de Rieske est localisée dans une membrane du thylakoïde d'au moins un chloroplaste au sein d'une cellule de la plante génétiquement modifiée.

6. Plante, partie de plante ou cellule de plante génétiquement modifiée selon la revendication 5, dans laquelle la protéine FeS de Rieske comprend un peptide de transit qui localise la protéine FeS de Rieske dans la membrane du thylakoïde, ledit peptide de transit comprenant un peptide de transit du cytochrome f, un peptide de transit du cyctochrome b6, un peptide de transit PetD, un peptide de transit PetG, un peptide de transit PetL, un peptide de transit PetN, un peptide de transit PetM ou un peptide de transit de la plastoquinone.

7. Plante, partie de plante ou cellule de plante génétiquement modifiée selon l'une des revendications 1 à 6, comprenant en outre un promoteur végétal lié de manière fonctionnelle à une séquence d'acide nucléique codant pour la protéine cytochrome C₆ et la protéine FeS de Rieske, ledit promoteur végétal comprenant un promoteur constitutif, un promoteur inductible, un promoteur spécifique à un type de tissu ou de cellule, ou un promoteur inductible spécifique à un type de tissu ou de cellule.

8. Plante, partie de plante ou cellule de plante génétiquement modifiée selon la revendication 1, dans laquelle la SBPase comprend une séquence d'acides aminés présentant au moins 70 % d'identité de séquence, au moins 75 % d'identité de séquence, au moins 80 % d'identité de séquence, au moins 85 % d'identité de séquence, au moins 90 % d'identité de séquence, au moins 95 % d'identité de séquence ou au moins 99 % d'identité de séquence avec la SEQ ID NO : 96,
dans laquelle la FBPA comprend une séquence d'acides aminés présentant au moins 70 % d'identité de séquence, au moins 75 % d'identité de séquence, au moins 80 % d'identité de séquence, au moins 85 % d'identité de séquence, au moins 90 % d'identité de séquence, au moins 95 % d'identité de séquence ou au moins 99 % d'identité de séquence avec la SEQ ID NO : 97,
dans laquelle la FBPase comprend une séquence d'acides aminés présentant au moins 70 % d'identité de séquence, au moins 75 % d'identité de séquence, au moins 80 % d'identité de séquence, au moins 85 % d'identité de séquence, au moins 90 % d'identité de séquence, au moins 95 % d'identité de séquence ou au moins 99 % d'identité de séquence avec la SEQ ID NO : 98, ou
dans laquelle la FBP/SBPase comprend une séquence d'acides aminés présentant au moins 70 % d'identité de séquence, au moins 75 % d'identité de séquence, au moins 80 % d'identité de séquence, au moins 85 % d'identité de séquence, au moins 90 % d'identité de séquence, au moins 95 % d'identité de séquence ou au moins 99 % d'identité de séquence avec la SEQ ID NO : 99.

9. Plante, partie de plante ou cellule de plante génétiquement modifiée selon la revendication 8, dans laquelle la SBPase, la FBPA, la FBPase ou la FBP/SBPase est localisée dans un stroma chloroplastique d'au moins un chloroplaste au sein d'une cellule de la plante génétiquement modifiée, ladite SBPase, FBPA, FBPase ou FBP/SBPase comprenant un peptide de transit qui localise la SBPase, la FBPA, la FBPase ou la FBP/SBPase dans le stroma chloroplastique de la plante.

10. Plante, partie de plante ou cellule de plante génétiquement modifiée selon l'une des revendications 8 et 9, comprenant en outre un promoteur végétal lié de manière fonctionnelle à une séquence d'acide nucléique codant pour la SBPase, la FBPA, la FBPase ou la FBP/SBPase, ledit promoteur végétal comprenant un promoteur constitutif, un promoteur inductible, un promoteur spécifique à un type de tissu ou de cellule, ou un promoteur inductible spécifique à un type de tissu ou de cellule.

11. Plante, partie de plante ou cellule de plante génétiquement modifiée selon l'une des revendications 1 à 10, ladite plante ayant une biomasse accrue en comparaison d'une plante de type sauvage (WT) non modifiée.

12. Plante, partie de plante ou cellule de plante génétiquement modifiée selon l'une des revendications 1 à 11, ladite plante ayant une efficacité d'utilisation de l'eau améliorée en comparaison d'une plante WT non modifiée ayant poussé dans des conditions d'intensité lumineuse supérieure à 1000 µmol m⁻² s⁻¹.

13. Plante, partie de plante ou cellule de plante génétiquement modifiée selon l'une des revendications 1 à 12, ladite plante étant sélectionnée dans le groupe constitué par : le niébé, le soja, le manioc, le riz, le blé, l'orge, la tomate, la pomme de terre, le tabac, le colza ou d'autres plantes cultivées en C3.

14. Procédé de production de la plante génétiquement modifiée selon l'une des revendications 1 à 13, comprenant :
a) l'introduction, dans une cellule, un tissu ou autre explant de la plante, de la ou des modifications génétiques améliorant la régénération du RuBP qui augmentent l'activité d'une protéine CB, de la ou des modifications génétiques améliorant le transport des électrons photosynthétiques, ou à la fois de la ou des modifications génétiques améliorant la régénération du RuBP qui augmentent l'activité d'une protéine CB et de la ou des modifications génétiques améliorant le transport des électrons photosynthétiques ;
b) la régénération de la cellule, du tissu ou autre explant de la plante en une plantule génétiquement modifiée ; et
c) la culture de la plantule génétiquement modifiée pour en faire une plante génétiquement modifiée présentant la ou les modifications génétiques améliorant la régénération du RuBP qui augmentent l'activité d'une protéine CB, la ou les modifications génétiques améliorant le transport des électrons photosynthétiques, ou à la fois la ou les modifications génétiques améliorant la régénération du RuBP qui augmentent l'activité d'une protéine CB et la ou les modifications génétiques améliorant le transport des électrons photosynthétiques.

15. Plante génétiquement modifiée selon l'une des revendications 1 à 13 ou procédé selon la revendication 14, dans lesquels la protéine CB comprend une fructose-1,6-bisphosphatases/sédoheptulose-1,7-bisphosphatase (FBP/SBPase) bifonctionnelle et la ou les protéines de transport des électrons photosynthétiques comprennent une protéine cytochrome C₆.
